# EUROPEAN PATENT APPLICATION

(11) **EP 1 582 520 A1**
(43) Date of publication of application: **05.10.2005**
(21) Application number: 03811117.5
(22) Date of filing: 11.11.2003
(51) Int. Cl.: C07D 471/06, C07D 487/06, A61K 31/519, A61K 31/551, A61P 9/00, A61P 19/02, A61P 29/00, A61P 43/00

(54) **NOVEL PARP INHIBITORS**

(30) Priority: 12.11.2002 JP 2002328935
(71) Applicant: MOCHIDA PHARMACEUTICAL CO., LTD., Tokyo 160-8515 (JP)
(72) Inventor: SHIROMIZU, Ikuya, c/o Mochida Pharmaceut. Co., Ltd, Tokyo 160-8515 (JP); KATO, Kazuo, c/o Mochida Pharmaceutical Co., Ltd, Tokyo 160-8515 (JP); YAMAMOTO, Ichiro, c/o Mochida Pharmaceut. Co., Ltd, Tokyo 160-8515 (JP); HAMAMOTO, Hajime, c/o Mochida Pharmaceut. Co., Ltd, Tokyo 160-8515 (JP)
(74) Representative: HOFFMANN EITLE
(86) International application number: PCT/JP2003/014319
(87) International publication number: WO 2004/043959

(57) **Abstract**

A compound represented by: 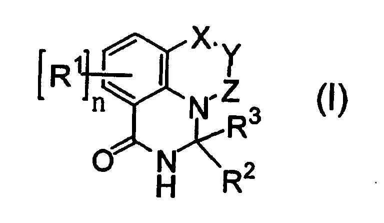 (wherein R¹ represents H, halogen, OH, amino, or C₁₋₄ alkyl or C₁₋₄ alkoxy unsubstituted or substituted by 1 to 5 halogens; n represents 0 to 2; R² and R³ each represent H, C₁₋₆ alkyl, C₂₋₄ alkenyl, or -L-Ar (L represents a bond or C₁₋₆ alkylene, and Ar represents a saturated or unsaturated 5- or 6-membered ring or saturated or unsaturated 8- to 12-membered condensed-bicyclic hydrocarbon group which does not contain or contain one to three N, S, or O atoms); any group in R¹ to R³ may have 1 to 3 substituents selected from group A [halogen, NO₂, CN, OH, oxo, and substituted or unsubstituted amino]; X represents - (CHR⁴) -, - (C=NOR⁵) - or -C(O)-; R⁴ represents -NR⁶R⁷ (R⁶ and R⁷ each represent H, C₁₋₆ alkyl, C₁₋₆ alkylcarbonyl, or the like); R⁵ represents H or C₁₋₆ alkyl; Y represents -NH- or methylene; Z represents methylene, ethylene or vinylene; and the hydrogen of Y and Z are unsubstituted or substituted by halogen, NO₂, alkyl or the like) or a salt thereof, and a pharmaceutical composition containing the compound or the salt.

The compound of the present invention has a poly(ADP-ribose)polymerase inhibitory effect, and is safe and advantageous for drug formulation.

## Description

### Technical Field

The present invention relates to a compound represented by Formula (I), a pharmaceutical composition containing the compound as an active ingredient, and a complex of human poly(ADP-ribose)polymerase with the compound or crystals of the human poly(ADP-ribose)polymerase alone.

### Background Art

Poly(ADP-ribose)polymerase (hereinafter referred to as PARP)(EC2. 4. 2. 30, also known as poly(ADP-ribose)synthase (PARS), poly(ADP-ribose)transferase (pADPRT)) is a specific intranuclear enzyme activated depending on DNA damage induced by, for example, active oxygen species, such as peroxynitrite (ONOO-), or radiation. If a DNA is damaged, PARP recognizes the single-strand cleavage site of the DNA, and the.Zn finger domain at the N terminus of the PARP is bonded to the DNA so that the PARP is activated to catalyze a transfer reaction using nicotinamide adenine dinucleotide (hereinafter referred to as NAD) as the substrate, in which the ADP-ribose site of the NAD is transferred to an adjacent protein, such as histone, DNA-polymerase, or DNA-topoisomerase. It is therefore considered that PARP not only signals the occurrence of DNA single-strand cleavage, but also contributes to DNA repair.

It is also considered that excessive activation of PARP causes the depletion of NAD and thus reduces ATP, and that consequently, the energy generation system is collapsed to result in cell death. A PARP inhibitor is therefore expected to act as an effective preventive and therapeutic drug against ischemic disease and ischemia reperfusion injury.

PARP is subject to limited hydrolysis as a substrate of caspase-3 (one of the interleukin-1β-converting enzyme-like cysteine protease family) which is a protease contributing to occurrence of apoptosis. It is therefore considered that PARP is involved in apoptosis.

Some studies using a generally known PARP inhibitor, such as 3-aminobenzamide or nicotinamide, and experiments using PARP knockout mice show that PARP inhibitors suppress cell death and remedy various states of diseases, such as ischemic diseases of, for example, brain, heart, gustrointestinal tract, skeletal muscle and retina; inflammatory diseases, such as arthritis, inflammatory bowel disease, and multiple sclerosis (see, for example, Non-Patent Document 5); diabetes; shock; extrapyramidal disorders; hyperalgesia (see, for example, Non-Patent Document 8); and ataxia telangiectasia. In addition, some reports have taught that PARP inhibitor are effective as antiretroviral agents against, for example, HIV (see, for example, Non-Patent Document 10), sensitizers for cancer radiotherapy or chemotherapy, and agents for alleviating side effects of anticancer drug treatment, such as cisplatin toxicity.

Already reported PARP inhibitors include monocyclic compounds, such as the above-mentioned 3-aminobenzamide, and compounds having a polycyclic nucleus containing a benzamide skeleton (for example, Patent Document 1: PCT Publication No. WO 01/42219).

Unfortunately, these compounds have problems to be solved in solubility, tissue transfer, safety and potency. Accordingly, further study for effective compounds is desired.

In order to solve the problems, some reports have been made about interactions between PARP catalytic fragments and substrate NAD or PARP inhibitors, including an analysis using crystals of an chicken PARP catalytic fragment and a complex of the chicken PARP catalytic fragment with an inhibitor (see, for example, Non-Patent Document 1: Ruf, A, et al., Biochemistry, 1998, Vol. 37, pp. 3893-3900). On the other hand, crystallization of human-derived PARP has not yet been achieved, and information for drug design to develop pharmaceuticals has not been sufficiently obtained.

### Disclosure of Invention

The object of the present invention is to provide a safe compound exhibiting effective pharmacological activity and having a PARP inhibitory activity, and a pharmaceutical composition containing the compound as an active ingredient.

The inventors of the present invention have conducted intensive research in order to overcome the above-described problems. As a result, the inventors found that a compound represented by Formula (I) has a superior PARP inhibitory activity and has high safety and advantageous to drug formulation, and the inventors thus accomplished the present invention. Also, the inventors successfully achieved the crystallization of a complex of the compound of the present invention with a human PARP catalytic fragment, and thus accomplished the invention.

The present invention relates to: (1) a compound represented by Formula (I) or its pharmaceutically acceptable salt; (2) a medical drug containing the compound or the salt as an active ingredient; and (3) a crystalline complex of the compound represented by Formula (I) or its salt with a human PARP catalytic fragment.

The compound of the present invention will now be described.
[1] Embodiment 1 of the present invention provides a compound represented by Formula (I) or its pharmaceutically acceptable salt: 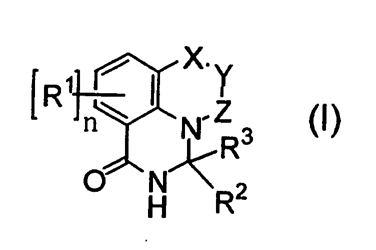 (wherein R¹ represents a hydrogen atom, a halogen atom, a hydroxyl group, an amino group, a straight or branched chain C1 to 4 alkyl group unsubstituted or substituted by 1 to 5 halogen atoms, or a C1 to 4 alkoxy group unsubstituted or substituted by 1 to 5 halogen atoms;
   n represents an integer of 0 to 2; and
   R² and R³ each independently represent a hydrogen atom, a straight or branched chain C1 to 6 alkyl group, a straight or branched chain C2 to 4 alkenyl group, or a group represented by -L-Ar (L represents a bond or a C1 to 6 alkylene, and Ar represents a saturated or unsaturated 5- to 6-membered ring or saturated or unsaturated 8- to 12-membered condensed-bicyclic hydrocarbon group which may contain 1 to 3 heteroatoms arbitrarily selected from the group consisting of nitrogen atom, sulfur atom and oxygen atom), and any of these groups may have 1 to 3 substituents arbitrarily selected from group A:
   [group A: halogen atoms, a nitro group, a cyano group, a hydroxy group, oxo groups, amino groups unsubstituted or substituted by one or two C1 to 4 alkyl groups, straight or branched chain C1 to 6 alkyl groups unsubstituted or substituted by 1 to 5 substituents arbitrarily selected from group Aa, straight or branched chain C1 to 5 alkoxy groups unsubstituted or substituted by 1 to 5 substituents arbitrarily selected from group Aa, straight or branched chain C1 to 5 alkylthio groups unsubstituted or substituted by 1 to 5 substituents arbitrarily selected from group Aa, straight or branched C2 to 4 alkenyl groups unsubstituted or substituted by 1 to 5 substituents arbitrarily selected from group Aa, and saturated or unsaturated 5- or 6-membered rings containing nitrogen unsubstituted or substituted by 1 to 5 substituents arbitrarily selected from group Aa (group Aa: a halogen atom, a nitro group, a hydroxy group, C1 to 3 alkoxy groups, cyano groups, amino groups unsubstituted or substituted by one or two C1 to 4 alkyl groups, carboxyl groups, and C1 to 3 alkoxycarbonyl groups)],
   X represents -(CHR⁴)-, -(C=NOR⁵)- or -(C=O)-,
   R⁴ represents -NR⁶R⁷ (R⁶ and R⁷ each independently represent a hydrogen atom, a C1 to 6 alkyl group, or a C1 to 6 alkylcarbonyl group; the hydrogen atom of the alkyl group and the alkylcarbonyl group are unsubstituted or substituted by a phenyl group, a piperidinyl group or an amino group unsubstituted or substituted by one or two C1 to 4 alkyl groups; and the hydrogen atom of the phenyl group and piperidinyl group are unsubstituted or substituted by an amino group unsubstituted or substituted by one or two C1 to 4 alkyl groups), a hydroxy group, a C1 to 6 alkoxy group, or a hydrogen atom; and
   R⁵ represents a hydrogen atom or a C1 to 6 alkyl group;
   Y represents an imino group (-NH-) or a methylene group;
   Z represents a methylene group, an ethylene group, or a vinylene group (-CH=CH-); the hydrogen atoms of Y and Z are unsubstituted or substituted by a halogen atom, a nitro group, a straight or branched chain C1 to 4 alkyl group, a straight or branched chain C1 to 4 alkoxy group, a cyano group, a phenyl group unsubstituted or substituted by one or two straight or branched C1 to 4 alkyl or alkoxy groups, or an amino group unsubstituted or substituted by one or two straight or branched C1 to 4 alkyl groups).
   The expression "C□ to □" herein represents the number of carbon atoms, and specifically, "C1 to 4" represents a carbon atom number of 1 to 4". For example, a "C1 to 4 alkyl group" represents an alkyl group having a carbon atom number of 1 to 4, such as methyl, ethyl, n-propyl, isopropyl, n-butyl, isobutyl, sec-butyl, and tert-butyl.
   The groups in formula (I) will now be described in detail.
   [1-1] Examples of the halogen atom in the definition R¹ in the formula include fluorine atom, chlorine atom, bromine atom and iodine atom; preferably, fluorine atom and chlorine atom. Examples of the "straight or branched chain C1 to 4 alkyl group" include methyl, ethyl, n-propyl, isopropyl, n-butyl, isobutyl, sec-butyl and tert-butyl; preferably, methyl. Examples of the "C1 to 6 alkoxy group" include methoxy, ethoxy, n-propoxy, isopropoxy, allyloxy, n-butoxy, isobutoxy, sec-butoxy and tert-butoxy; preferably, methoxy.
      Examples of the "straight or branched C1 to 6 alkyl group" in the definitions of R² and R³ include methyl, ethyl, n-propyl, isopropyl, n-butyl, isobutyl, sec-butyl, tert-butyl, pentyl, isopentyl, neopentyl, tert-pentyl, 1-methylbutyl, 2-methylbutyl and 1,2-dimethylpropyl; preferably, methyl. Examples of the "straight or branched chain C2 to 4 alkenyl group" include vinyl, allyl, isopropenyl, 2-methylallyl and butenyl.
      Examples of the "saturated or unsaturated 5- to 6-membered ring which may contain 0 to 3 heteroatoms" include phenyl, pyridyl, pyrazinyl, pyrimidinyl, pyridazinyl, triazinyl, pyranyl, pyrrolyl, imidazolyl, pyrazolyl, thienyl, furyl, thiazolyl, isothiazolyl, oxazolyl, isoxazolyl, thiazolyl, thiazinyl, thiadiazinyl, thiadiazolyl, triazolyl, cyclopentyl, cyclohexyl, 1-cyclopentene-1-yl, 2-cyclopentene-1-yl, 3-cyclopentene-1-yl, 1-cyclohexene-1-yl, pyrrolidinyl, imidazolinyl, pyrazolidinyl, pyrazolinyl, piperidyl, piperazinyl and morpholinyl. Examples of the "saturated or unsaturated 8- to 12-membered condensed-bicyclic hydrocarbon group" include naphthyl, indene-2-yl, indane-1-yl, indane-2-yl, indane-5-yl, 5,6,7,8-tetrahydro-2-naphthyl, 5,6,7,8-tetrahydro-5-naphthyl, and 5,6,7,8-tetrahydro-6-naphthyl. Among these preferred are 8- to 12-membered condensed-bicyclic aromatic hydrocarbon groups. More specifically, for example, naphthyl and indene-2-yl are preferable.
      Examples of the "C1 to 6 alkylene unsubstituted or substituted" in the detinition of L include methylene, ethylene, n-propylene, n-butylene, sec-butylene, n-pentylene and n-hexylene; preferably, C1 to 4 alkylene groups. The substituent for the hydrogen of the alkylene is selected from group A, and is preferably hydroxy, oxo, or amino.
      The "C1 to 6 alkoxy groups" in the definition of the "substituents" belonging to group A include methoxy, ethoxy, n-propoxy, isopropoxy, allyloxy, n-butoxy, isobutoxy, sec-butoxy and tert-butoxy; preferably, methoxy.
      The "alkylthio groups" include, for example, methylthio, ethylthio, n-propylthio, isopropylthio, n-butylthio, isobutylthio, sec-butylthio and tert-butylthio, preferably, methylthio.
      The "halogen atoms" include, for example, fluorine atom, chlorine atom, bromine atom and iodine atom; preferably, bromine atom. The "C2 to 4 alkenyl groups" include, for example, vinyl, 2-butenyl, 2-propenyl and 3-butenyl.
      The "saturated or unsaturated 5- or 6-membered rings containing nitrogen" include, for example, pyridyl, imidazolyl, pyrazinyl, pyrimidinyl, pyridazinyl, pyrrolidinyl, imidazolinyl, pyrazolidinyl, pyrazolinyl, piperidyl, piperazinyl and morpholinyl. The "alkoxycarbonyl groups" include, for example, methoxycarbonyl, ethoxycarbonyl, n-propoxycarbonyl and isopropoxycarbonyl.
      [1-1-a] Preferably, R² represents a hydrogen atom and R³ represents a group represented by -L-Ar (L represents a bond or a C1 to 6 alkylene, and Ar represents a saturated or unsaturated 5- to 6-membered ring or saturated or unsaturated 8- to 12-membered condensed-bicyclic hydrocarbon group which may contain 1 to 3 heteroatoms arbitrarily selected from the group consisting of nitrogen atom, sulfur atom and oxygen atom) .
      [1-1-b] More preferably, Ar in the -L-Ar group is a saturated or unsaturated 5- to 6-membered ring which may contain 1 to 3 heteroatoms arbitrarily selected from the group consisting of nitrogen atom, sulfur atom and oxygen atom.
      [1-1-C] Still more preferably, Ar is a 5- or 6-membered aromatic ring. Examples of such rings include phenyl, pyridyl, pyrazinyl, pyrimidinyl, pyridazinyl, triazinyl, pyranyl, pyrrolyl, imidazolyl, pyrazolyl, thienyl, furyl, thiazolyl, isothiazolyl, oxazole, isoxazole, thiazoline, thiazine, thiadiazinyl, thiadiazolyl and triazolyl; preferably, phenyl and pyridyl and more preferably, phenyl. [1-1-d] Still more preferably, L represents a bond.
   [1-2] In the Formula (I), Z represents:
      [1-2-a] a methylene group, an ethylene group or a vinylene (-CH=CH-) group. Hydrogen atoms in these groups are unsubstituted or substituted by a halogen atom, a nitro group, a C1 to 4 alkoxy group, a cyano group, a phenyl group or amino group. Hydrogen atom of the "phenyl group" is unsubstituted or substituted by one or two of straight or branched chain C1 to 4 alkyl groups or one or two of straight or branched C1 to 4 alkoxy groups. Hydrogen atoms of the "amino group" is unsubstituted or substituted by one or two of straight or branched chain C1 to 4 alkyl groups
      [1-2-b] Preferably, Z is a methylene group.
   [1-3] In Formula (I), Y represents:
      [1-3-a] an imino (-NH-) group or a methylene (-CH₂-) group.
      [1-3-b] Preferably, Y is a methylene group.
   [1-4] In Formula (I), X represents:
      [1-4-a] - (CHR⁴) -, - (C=NOR⁵) - or -(C=O)-.
         R⁴ represents -NR⁶R⁷ (R⁶ and R⁷ each independently represent a hydrogen atom, a C1 to 6 alkyl group or a C1 to 6 alkylcarbonyl group, the alkyl group and the alkylcarbonyl group may have a phenyl group, and the phenyl group is unsubstituted or substituted by an amino group unsubstituted or substituted by one or two C1 to 4 alkyl groups), a hydroxy group, a C1 to 6 alkoxy group, or a hydrogen atom.
         R⁵ represents a hydrogen atom or a C1 to 6 alkyl group.
      [1-4-b] Preferably, X represents -(CHR⁴)-.
      [1-4-c] More preferably, R⁴ is an amino group.
[2] Embodiment 2 of the present invention provides a pharmaceutical composition characterized by containing at least one active ingredient selected from the group consisting of the compounds represented by Formula (I) and their pharmaceutically acceptable salts.
[3] Embodiment 3 of the present invention provides a PARP inhibitor characterized by containing the compound represented by Formula (I) and its pharmaceutically acceptable salt.
   Preferably, the compound in the inhibitor has an enzyme inhibitor activity exhibiting an IC₅₀ of less than 1 µM, more preferably of less than 0.1 µM. IC₅₀ shows the 50% inhibitory concentration.
[4] Embodiment 4 of the present invention provides a preventive and therapeutic agent characterized by containing the compound represented by Formula (I) and its pharmaceutically acceptable salt, used against diseases in which the PARP activity seems to be increasing.
   In diseases in which the PARP activity seems to be increasing, the PARP is probably activated by DNA damage resulting from the production of reactive oxygen species such as peroxynitrite and a hydroxy radical. Examples of such diseases include various types ischemic diseases, such as cerebral ischemic disorder, ischemic heart disease and organ damage resulting from ischemia-reperfusion, inflammatory disease, multiple sclerosis, arthritis, chronic rheumatism, shock, septicemia, neurodegenerative disease, immunologic disease, allergic disease, lifestyle-related disease, Parkinson's disease, Alzheimer's disease, AIDS, Huntington's chorea, diabetes, cancer, renal insufficiency, osteoporosis, and hyperalgesia. It has been well known that nitrogen oxide (NO) or a hydroxy radical is involved in the state of the diseases, and that a hydroxy radical generated by ischemia converts NO into peroxynitrite being a cytotoxic active oxygen species.
[5] Embodiment 5 of the present invention provides a preventive and curative treatment using a composition characterized by containing the compound represented by Formula (I) and its pharmaceutically acceptable salt, which is applied to disease in which the PARP activity seems to be increasing.
[6] Embodiment 6 of the present invention provides a method for crystallizing a natural or recombinant human PARP catalytic fragment. The human PARP catalytic fragment is a fragment of a human PARP peptide consisted of 1014 amino acids, forming a catalytic domain consisted of the binding site and active center of the substrate NAD. More specifically, the fragment is consisted of 361 amino acids from the C terminus Lys 654 to Trp 1014 (sequence ID No. 1). The human PARP catalytic fragment of the present invention can form a co-crystal with a compound binding to the catalytic site by a method described later. In addition, the analysis of the sterostructural information or the like of the co-crystal can lead to a pharmacophore needed for a compound controlling the catalytic activity of the human PARP, and contribute to drug design. In general, protein is crystallized by, for example, batch methods, free interface diffusion, dialysis or vapor diffusion (A. Ducruix and R. Giege: Crystallization of nucleic Acids and Proteins, A Practical Approach, IRL PRESS at Oxford University Press (1991) pp. 121-146). In these processes, the solubility of protein in a protein solution is gradually reduced by use of a precipitant to produce single crystals of the protein. The single crystal mentioned herein refers to a crystal grown from a crystal nucleus precipitated by decrease in solubility of the protein occurring through crystallization by the above processes. The single crystal is required for three-dimensional structural analysis of protein, but such analysis cannot be achieved by use of polycrystals grown from a plurality of nucleuses.
   Any one of the above processes may be applied to crystallization in the method of the present invention, but the method is not limited to the processes. Preferably, crystallization is performed by vapor diffusion.
   Techniques for vapor diffusion include hanging-drop vapor diffusion, sitting-drop vapor diffusion, and sandwich-drop vapor diffusion, which are different in how to place a protein-containing drop in an excess amount of a precipitant-containing reservoir solution. In these techniques, the concentration of the precipitant in the drop is increased by vapor diffusion between the reservoir solution and the drop in an airtight container, so that the solubility of the protein is reduced and, thus, single crystals are precipitated. In order to produce superior single crystals, hanging-drop vapor diffusion is preferably applied.
   This method uses an excess amount of reservoir solution containing a precipitant in an airtight container and a drop containing a solution of a protein to be crystallized and a precipitant. The drop is placed in such a manner as not to come into contact with the reservoir solution. The drop is placed on a support, for example, a siliconized cover glass. For hanging-drop, the cover glass is turned upside down so that the drop keeps hanging in the direction of gravitational force. As equilibrium between the reservoir solution and the drop proceeds, the concentration of the precipitant in the drop increases to precipitate single crystals of the protein in the drop.
   The drop contains human PARP, a low-concentration precipitant, an inorganic salt, and a buffer solution. The reservoir solution is adjusted by a high-concentration precipitant and a buffer solution. The precipitant may be an inorganic salt or a polyol. A preferred polyol is polyethylene glycol. More preferably, the polyethylene glycol has a mean molecular weight of 600 to 8000. In order to increase the ion strength of the reservoir solution together with the precipitant and to buffer undesirable interaction among proteins, it is preferable that the reservoir solution contain an appropriate inorganic salt. Preferred inorganic salts include ammonium acetate, lithium chloride, magnesium chloride, calcium chloride, potassium chloride, ammonium chloride, calcium acetate, potassium acetate, magnesium acetate, zinc acetate, and sodium acetate. It is preferable that the reservoir solution has a pH between weak acid and neutral from the viewpoint of precipitating crystals. More preferably, the pH is 4.4 to 6.0, and particularly about 5.2.
   Preferably, the drop contains human PARP concentrated to 1 to 10 mg/mL. The human PARP is more preferably concentrated to 1 to 7 mg/mL, and still more preferably to 3 to 5 mg/mL. Furthermore, for example, the precipitator, inorganic salt or the like contained in the reservoir solution is preferably added at a low concentration to the drop. More preferably, such an additive is added at a concentration of half the concentration of the reservoir solution. Crystallization is performed at a constant temperature. The crystallization temperature is preferably 4 to 30°C, more preferably 10 to 25°C, and still more preferably 20°C.
   By performing crystallization under the above-described conditions, a crystal of human PARP catalytic fragment (having a width of 0.1 mm or more) can be produced normally within 1 week to 2 months.
   The process for producing the crystals of the present invention is not limited to the production of crystals composed of only human PARP catalytic fragment, and it includes a production of complexes of the human PARP catalytic fragment crystal with a substance having an affinity to the human PARP catalytic fragment. The process for producing the crystalline complex includes the step of binding the human PARP catalytic fragment to this substance. The substance having an affinity to the human PARP catalytic fragment is, for example, a chemical compound inhibiting human PARP activity.
   The binding step is performed between pretreatment step and crystallization step. The crystallization of a complex by this process is generally referred to as cocrystallization.
[7] Embodiment 7 of the present invention provides an orthorhombic crystal of human PARP catalytic fragment having a space group of P2₁2₁2. The crystal of human PARP catalytic fragment is produced by the method described in Embodiment 6 of the present invention.
[8] In Embodiment 8 of the present invention, the crystal of human PARP catalytic fragment of Embodiment 7 has lattice constants of a = 67Å±5Å, b = 92Å±5Å, and c = 64Å±5Å, preferably a = 67Å±2Å, b = 92Å±2Å, and c = 64Å±2Å, and more preferably a = 67Å±1Å, b = 92Å±1Å, and C = 64Å±1 Å.
[9] Embodiment 9 of the present invention provides a co-crystal of a purified human PARP catalytic fragment with a compound having an affinity to the human PARP catalytic fragment. Preferably, the compound having an affinity to the human PARP catalytic fragment inhibits human PARP activity.
[10] Embodiment 10 of the present invention provides a co-crystal of a purified human PARP catalytic fragment with at least one selected from the group consisting of at least one of the compounds described in Embodiments 1 to 3 or one of their pharmaceutically acceptable salts.
[11] Embodiment 11 of the present invention provides a co-crystal of a purified human PARP catalytic fragment with a specific novel compound (+)-(3R,7R)-7-amino-3-(4-methoxyphenyl)-6,7-dihydro-3H,5H-pyrido[3,2,1-ij]quinazoline-1(2H)-one hydrochloride (Example 9) which inhibits human PARP activity. One of the processes for producing the co-crystal is described in "Example of X-ray crystal structure analysis".
[12] Embodiment 12 of the present invention provides a co-crystal of a purified human PARP with a specific novel compound 7-amino-3-(4-methoxy-3-nitrophenyl)-6,7-dihydro-3H,5H-pyrido[3,2,1-ij]quinazoline-1(2H)one (Example 11) which inhibits human PARP activity.
[13] Embodiment 13 of the present invention provides a co-crystal of a purified human PARP and a specific novel compound 7-(4-N,N-dimethylaminophenyl)methylamino-3-(4-methoxyphenyl)-6,7-dihydro-3H,5H-pyrido[3,2,1-ij]quinazoline-1(2H)-one (Example 13) which inhibits human PARP activity.

The tertiarystructural coordinates of the complexes can be obtained by a crystallographic analysis of the above co-crystal, described in the embodiment below. By analyzing the coordinates obtained by the crystallographic analysis, an amino acid residue for interaction between the human PARP and the compound selectively inhibiting the human PARP can be specified. In addition, extracting the amino acid residue associated with the interaction by displaying the coordinates of the complex molecule with existing molecular design software (SYBYL of Tripos, Insight II of Accelrys, etc.), properties and other information particularly important for applications to industry can be extracted. For example, if an amino acid residue of the human PARP catalytic fragment, present within several angstroms from the compound is extracted from the crystal structure of the complex, the directly interacting amino acid residue on the human PARP catalytic fragment can be extracted or specified. By use of conformational coordinates obtained as above, the pharmacophore of a compound selectively inhibiting human PARP can be extracted.

The word "compound" used in any of the above embodiments includes the meaning of the "pharmaceutically acceptable salt of the compound".

The "pharmaceutically acceptable salt" mentioned herein is referred to as a salt acceptable in terms of biological and other aspects while maintaining the biological efficacy and properties of its mother compound. For example, such salts include: salts of the compound of the present invention with inorganic acids generally used in the field of the invention, such as hydrochloric acid, hydrobromic acid, sulfuric acid and phosphoric acid; salts of the compound with organic acids (including mono-salts, di-salts, and tri-salts), such as acetic acid, lactic acid, tartaric acid, malic acid, succinic acid, fumaric acid, maleic acid, citric acid, benzoic acid, trifluoroacetic acid, p-toluenesulfonic acid and methanesulfonic acid; inorganic salts, such as sodium salts, potassium salts, magnesium salts, calcium salts, and ammonium salts; organic salts, such as monoethanolamine, diethanolamine, and triethanolamine; and hydrates of the compound of the present invention and its salts; various solvates of the compound of the present invention and its pharmaceutically acceptable salts; and crystalline polymorphisms of the compound of the present invention, acceptable as a medical drug.

The compound represented by Formula (I) of the present invention also includes its isomers. For example, if the compound has an asymmetric carbon, the compound may exist in a mixture containing different stereoisomers or steroisomers including racemic modification. In the present invention, the compound may include such various forms, and these forms can be used as active ingredient compounds as well. Those steroisomers can be isolated or purified by a process commonly used by those skilled in the art through preferential crystallization, optical resolution with column chromatography, or asymmetric synthesis.

The present invention also includes intermediates that are novel substances obtained in the synthesis of the compound of the present invention. The ¹H-NMR data of some of the intermediates are shown in Table 6, but the intermediates of the present invention are not limited to these.

### Brief Description of the Drawing

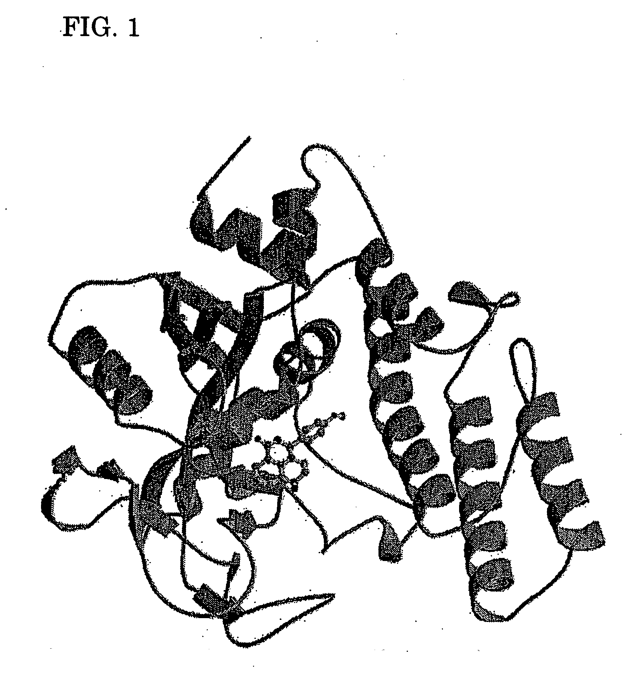

Fig. 1 shows the crystal structure of a complex of Example Compound 9 with a human PARP catalytic fragment, obtained by crystal structure analysis.

### Best Mode for Carrying Out the Invention

### [Process for producing the compound of the present invention]

The compound of the present invention can be produced by the following process or a process described in examples described later.

A general production process will be shown by Reaction Scheme 1.

A tricyclic pyridoquinazolinone compound [I] of the present invention is produced from an aniline derivative [III] (described later) through tetrahydroquinoline derivatives [V] and [VI], and, if required, subsequently a pyridoquinazolinone derivative [VII]. 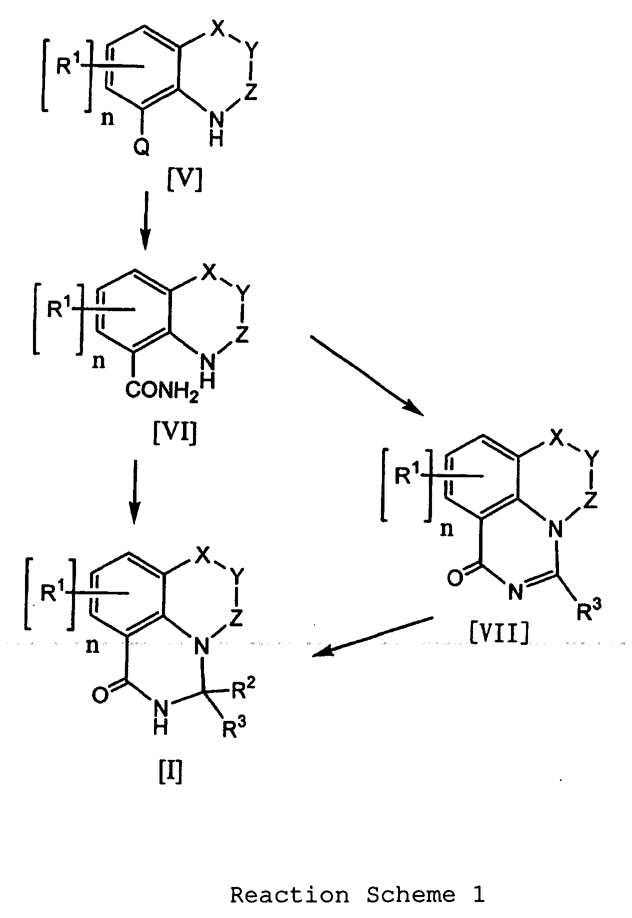 (In Reaction Scheme 1, R¹, R², R³ and n have the same meanings as defined above, and X' represents -(CHR⁴)- or - (C=NOR⁵) - (R⁴ and R⁵ are the same as above).)

More specifically, a form [I'] of the compound [I] of the invention other than oxo forms [Ia] is produced by converting the group X into various types of group, as shown in Reaction Scheme 2. For the production of [I'], the step of converting the group X into various types of group may be performed on the stage of a tetrahydroquinolinone derivative [V]. 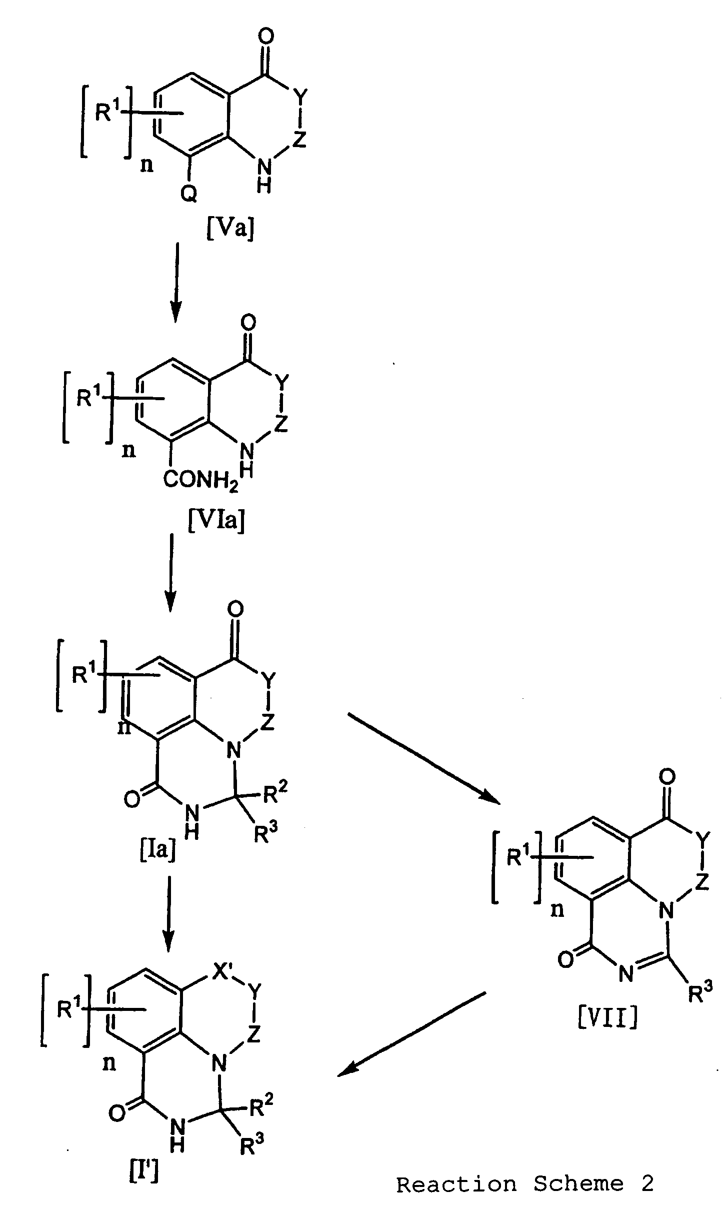 (In Reaction Scheme 2, R¹, R², R³, n, and X' have the same meanings as defined above.)

Each reaction step will now be described in detail.

### Step 1

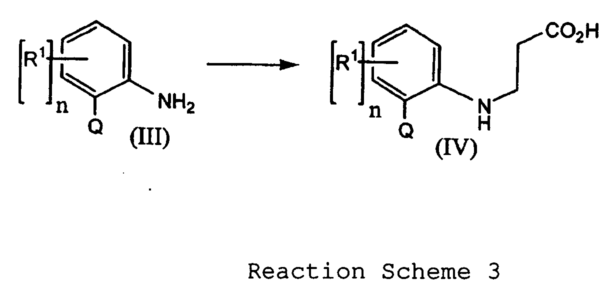

(In Reaction Scheme of Step 1, R¹ and n are the same as above, and Q represents a chlorine atom, a bromine atom, an iodine atom, an alkoxycarbonyl group, a carbamoyl group, or a cyano group.)

Acrylic acid is reacted with an aniline derivative (III) or the aniline derivative is subjected to an addition reaction to form an acrylic ester, and then hydrolysis is performed to yield a substituted phenylaminopropionic acid derivative (IV). This reaction is carried out by use of an equivalent or excessive acrylic acid or acrylic ester at a temperature between room temperature and about 120°C in absence of solvent or, if necessary, in water, alcohol, acetonitrile, or other solvents.

### Step 2

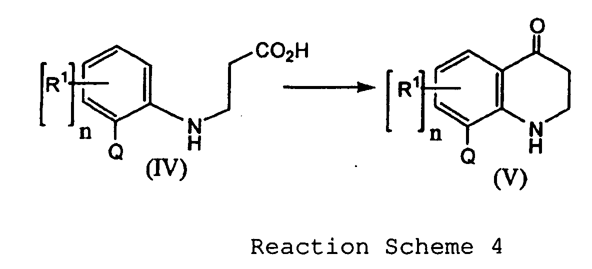

(In Reaction Scheme of Step 2, R¹, n, and Q have the same meanings as defined above.)

Tetrahydroquinolinone derivative (V) is produced from the substituted phenylaminopropionic acid derivative represented by Formula (IV) by use of a dehydration condensing agent, such as polyphosphoric acid, phosphorus pentoxide or polyphosphate ester, at a temperature between room temperature and about 120°C in absence of solvent or, if necessary, in an appropriate solvent.

### Step 3

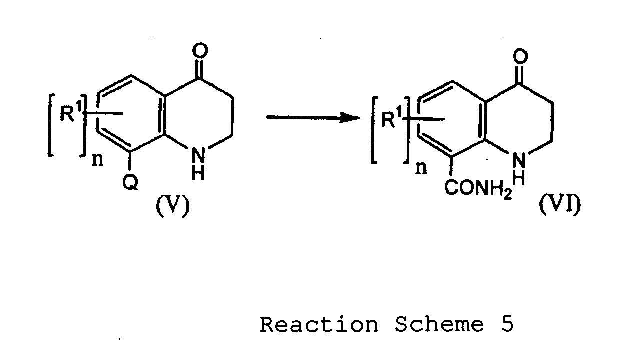

(In Reaction Scheme of Step 3, R¹, n and Q have the same meanings as defined above.)

A carboxylic amide derivative (VI) is produced from the substituted tetrahydroquinolinone derivative represented by Formula (V). If Q represents a chlorine atom, a bromine atom or an iodine atom, a cyano derivative is produced in the presence of a palladium catalyst, and the cyano derivative is hydrolyzed to yield a carboxylic acid amide derivative (VI). If Q represents an alkoxycarbonyl group, a carboxylic amide derivative (VI) is produced by use of ammonia. Alternatively, after hydrolysis, condensation with ammonia is performed with a dehydration condensing agent such as a BOP reagent (benzotriazole-1-yl-oxy-tris(dimethylamino)phosphonium hexafluorophosphate) to yield a carboxylic amide derivative (VI).

### Step 4

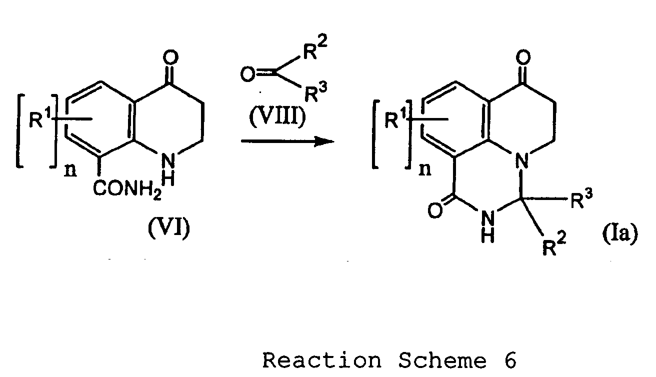

(In Reaction Scheme of Step 4, R¹, R², R³ and n have the same meanings as defined above.)

Tetrahydroquinolinone carboxylic acid amide (VI) is reacted with a ketone or an aldehyde represented by Formula (VIII) in the presence of an acid catalyst to yield a pyridoquinazolinone derivative represented by Formula (Ia). This reaction is carried out in a protic solvent such as ethanol, methanol, or acetic acid. Preferred acid catalysts include mineral acids such as concentrated hydrochloric acid and nitric acid, and acids such as trifluoromethanesulfonic acid, p-toluenesulfonic acid, and sulfuric acid. The reaction temperature is set between room temperature and a boiling point of the solvent used. Preferably, the reaction is performed under reflux conditions.

### Step 5

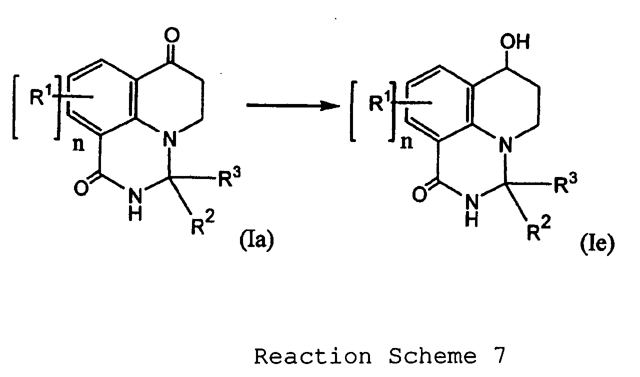

(In Reaction Scheme of Step 5, R¹, R², R³ and n are the same meanings as defined above.)

Pyridoquinazolinone derivative represented by Formula (Ia) is reacted with a reducing agent such as sodium borohydride or lithium aluminum hydride, to yield a pyridoquinazoline derivative represented by Formula (Ie). This reaction is performed between ice-cooled temperature and room temperature. Preferably, it is performed at ice-cooled temperature. Suitable solvents are alcohol solvents such as methanol for boron-based reducing agents, and ether solvents such as tetrahydrofuran for aluminum-based reducing agents. Step 6 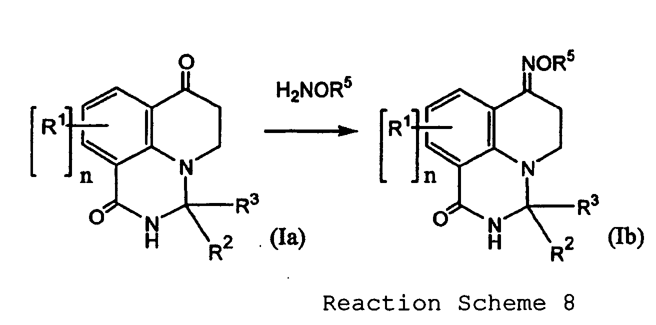 (In Reaction Scheme of Step 6, R¹, R², R³, R⁵ and n have the same meanings as defined above.)

Pyridoquinazolinone derivative represented by Formula (Ia) is reacted with a hydroxylamine derivative to yield a pyridoquinazolinone oxime derivative represented by Formula (Ib). In this reaction, the hydroxylamine derivative is allowed to act in an alcohol solvent, such as ethanol or isopropanol. The reaction temperature is set between room temperature and a boiling point of the solvent used. Preferably, the reaction is performed under reflux conditions. Step 7 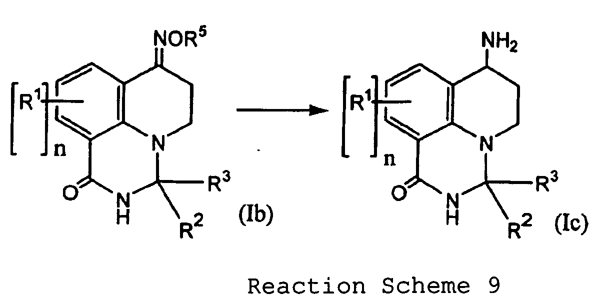 (In Reaction Scheme of Step 7, R¹, R², R³, R⁵ and n have the same meanings as defined above.)

The pyridoquinazolinone oxime derivative represented by Formula (Ib) is hydrogenated in the presence of a catalyst, or is reduced by use of zinc, iron under acidic conditions, or by use of sodium cyanoborohydride to prepare a pyridoquinazolinone derivative represented by general formula (Ic). Exemplary catalysts include palladium, platinum oxide and nickel; exemplary solvents include alcohol solvents such as methanol and ethanol; organic acids such as formic acid and acetic acid; amide solvents such as dimethylformamide and N-methylpyrrolidine; and ether solvents such as tetrahydrofuran and dioxane. The reaction proceeds at a temperature between room temperature and a boiling point of the solvent used. If the reduction is performed by use of zinc, iron or tin, preferable solvents are, for example, diluted hydrochloric acid, diluted sulfuric acid or formic acid.

### Step 8

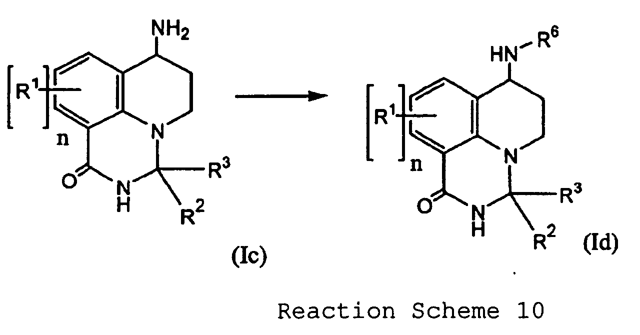

(In Reaction Scheme of Step 8, R¹, R², R³, R⁶ and n have the same meanings as defined above.)

The pyridoquinazolinone derivative represented by Formula (Ic) is reductively aminated or acylated to yield a compound represented by Formula (Id). The reductive amination proceeds between ice-cooled temperature and room temperature, and preferable solvents are chlorine solvents, such as methylene chloride and chloroform. The acylation proceeds between ice-cooled cooling temperature and room temperature, and preferable solvents include halogen solvents such as methylene chloride and chloroform; ether solvents, such as tetrahydrofuran; ester solvents such as ethyl acetate; and amide solvents such as dimethylformamide.

### Step 9

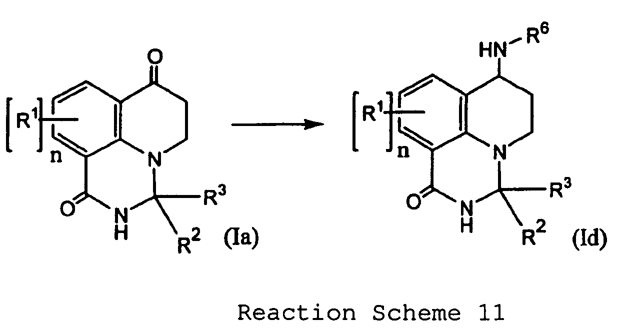

(In Reaction Scheme of Step 9, R¹, R², R³, R⁶ and n have the same meanings as defined above.)

The pyridoquinazolinone derivative represented by Formula (Ia) is reacted with an amine derivative R⁶NH₂ in coexistence of a reducing agent such as sodium cyanoborohydride to yield a compound represented by Formula (Id). The reductive amination proceeds between ice-cooled temperature and room temperature, and preferable solvents halogen solvents such as methylene chloride and chloroform.

### Step 10

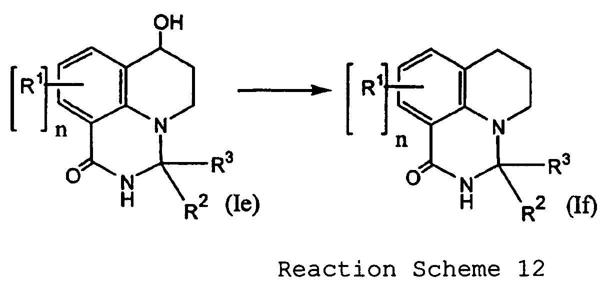

(In Reaction Scheme of Step 10, R¹, R², R³ and n have the same meanings as defined above.)

Pyridoquinazolinone derivative represented by Formula (Ie) is reacted with methanesulfonyl chloride, p-toluenesulfonyl in the presence of a base, and subsequently with a reducing agent such as lithium aluminum hydride to yield a pyridoquinazolinone derivative represented by Formula (If). This reaction proceeds between water cooling temperature and room temperature. Preferable solvents are ether solvents such as tetrahydrofuran. Step 7 may produce a by-product, the derivative (If).

### Step 11

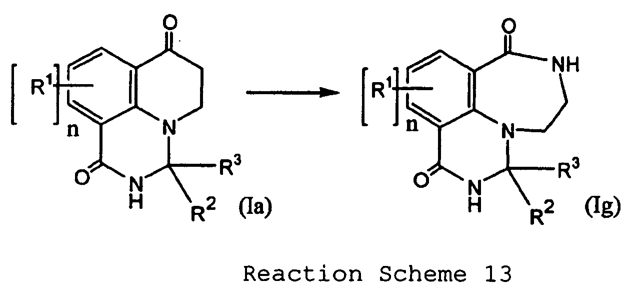

(In the formula of Step 11, R¹, R², R³ and n have the same meanings as defined above.)

Pyridoquinazolinone derivative represented by Formula (Ia) is reacted with sodium azide to yield a pyrimidobenzodiazepin derivative having an enlarged ring and represented by Formula (Ig). This reaction can be performed between ice-cooled temperature and room temperature, and it is preferably performed at ice-cooled temperature. Preferable solvents are methanesulfonic acid sulfuric acid, hydrochloric acid or the like. The product may be obtained by subjecting the compound produced in Step 6 of Formula (Ib) (R⁵ represents a hydrogen atom) to a rearrangement reaction under acidic conditions.

### Step 12

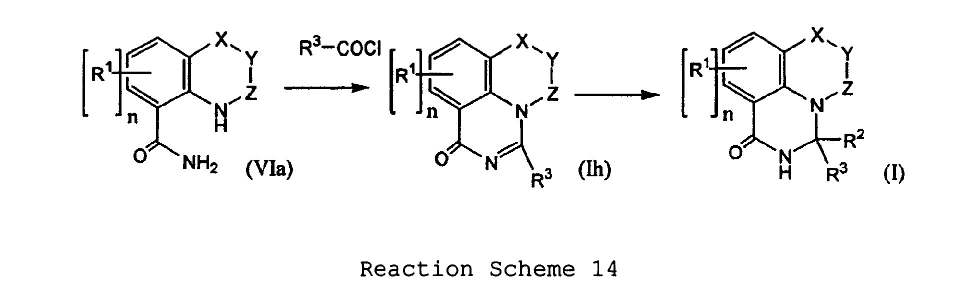

(In Reaction Scheme of Step 12, R¹, R², R³, n, X, Y and Z have the same meanings as defined above.)

The tetrahydroquinolinecarboxylic amide represented by Formula (VIa) is reacted with an acid chloride R³COCl to yield pyridoquinazolinone derivative (Ih). Then, a reducing agent, such as lithium aluminum hydride or sodium borohydride, is allowed to act on this product to yield a pyridoquinazolinone derivative represented by Formula (I). The solvent used for the reaction with the acid chloride is preferably an amide solvent such as N,N-dimethylformamide or N-methylpyrrolidone, and the reaction proceeds between ice-cooled temperature and about 100°C. In the subsequent reduction reaction, ether solvents such as tetrahydrofuran are suitable for use of lithium aluminum hydride, and alcohol solvents such as methanol and ethanol, are suitable for use of sodium borohydride. The reaction temperature is set between ice-cooled temperature and a boiling point of the solvent used.

The efficacy of the compound of the present invention and a pharmaceutical composition of.the present invention will now be described.

The pharmaceutical composition containing as one or more of active ingredients compounds of the present invention and their pharmaceutically acceptable salts is used for preparing capsules, pills, tablets, granules, subtle granules, powder, liquid medicines such as suspended drug, emulsion, limonade, elixir and syrup, injectable solution, transnasal absorbents, suppositories, ointment, and plasters, singly or in combination with a commonly used carrier or excipient or other additives for pharmaceuticals. The resulting product is administered orally or parenterally to human and other animals.

Normally used carriers for pharmaceuticals include, but not limited to, sterilized water, isotonic sodium chloride, vegetable oil, mineral oil, higher alcohols, higher fatty acids and harmless organic solvents, and further include, if necessary, excipients, colorants, emulsifiers, suspensions, surfactants, solubilizing agent, adsorption inhibitor, stabilizing agents, preservatives, moisturizing agents, anti-oxidants, buffering agents, tonicities and soothing agents.

Solid compositions for oral administration which the present invention can provide include capsules, pills, tablets, powder, and granules. These solid compositions are produced in combination of at least one active substance with at least one inactive carrier. More specifically, the solid compositions contain excipients (for example, lactose, sucrose, mannitol, glucose, hydroxypropyl cellulose, microcrystalline cellulose or metasilicic acid), a binder (for example, crystalline cellulose, saccharides, dextrin, hydroxypropyl cellulose, hydroxypropylmethyl cellulose, polyvinylpyrrolidone or macrogol), lubricants (for example, magnesium stearate, calcium stearate, or talc), disintegrants (for example, cornstarch, carboxymethyl cellulose, or calcium cellulose glycolate), stabilizing agents (for example, sugar, sugar alcohol such as lactose or its alcohol), solubilizers (for example, cholesterol, triethanolamine, glutamic acid or aspartic acid), colorants, flavoring agents, antiseptics, tonicities, dispersants, anti-oxidants (for example, ascorbic acid or butylhydroxyanisole), buffering agents or preservatives (for example, paraben or benzyl alcohol). The pills, tablets, granules and the like may be coated with a gastric coating or enteric coating made of, for example, sucrose, gelatin, or hydroxypropylmethyl cellulose phthalate, if necessary.

The injectable solution for parenteral administration contains an aseptic aqueous or nonaqueous dissolving agents, suspensions or emulsifiers. Carriers of the aqueous dissolving agents and suspensions include, for example, distilled injection water and isotonic sodium chloride solution. Carriers of the nonaqueous dissolving agents and suspensions include propylene glycol, polyethylene glycol, vegetable oils such as olive oil, alcohols such as ethyl alcohol, and Polysorbate 80 (TM). This type of composition also serves as the above-listed additives such as tonicities, antiseptics, wetting agents, emulsifiers, dispersants, stabilizers and solubilizers. This type of composition is asepticized by, for example, filtration through a membrane filter, addition of bactericide, or UV irradiation. This type of composition can be produced as an aceptic solid composition that is dissolved, emulsified, or suspended to be used as an injectable solution before use. If the compound of the present invention has a low solubility, it may be solubilized. For solubilization, known methods applicable to medicinal preparation can be applied. For example, a surfactant (polyoxyethylene hardened castor oil, polyoxyethylene sorbitan higher fatty acid ester, sucrose fatty acid ester) may be added; solid dispersion of the drug may be formed with a solubilizer, for example, polymer (water-soluble polymer such as polyethylene glycol (PEG), hydroxypropylmethyl cellulose (HPMC) or polyvinylpyrrolidone (PVP); or enteric polymer such as hydroxypropylmethyl cellulose phthalate (HPMCP), methyl methacrylate-methacrylic acid copolymer (Eudragit L or S (TM), produced by Rohm & Haas Company)). Alternatively, if necessary, an inclusion compound may be formed with α-, β-, or γ-cyclodextrin, hydroxypropylcyclodextrin or the like. These methods of solubilization may be appropriately modified according to the desired drug, with reference to, for example, "Yakugaku monographs No. 1, Seibutsu Kagaku Riyou Nou", Nagai et al., Soft Science, Inc., pp. 78-82, 1988; or "Saikin no Seizai Gijutsu to Sono Ouyou", Utsumi et al., Iyaku Journal, pp. 157-159, 1983. Preferably, the method of forming solid dispersion containing the drug and a dissolving agent is applied to improve the solubility (Japanese Patent Application Laid-open No. Sho 56-49314 and FR 2460667).

An appropriate amount of the compound of the present invention is clinically administered to a person, depending on the symptom, weight, age, sex, and other factors of the patient to whom the compound is to be given. In general, the administration amount for an adult is orally 0.1 mg to 1000 mg per day, preferably 1 mg to 300 mg per day, and parenterally 0.01 mg to 300 mg per day, preferably 0.1 mg to 100 mg per day. Such an amount of the compound is administered at a time, or several. Since the administration amount depends on various factors, a smaller amount than the above ranges may suffice.

The compound represented by Formula (I) and its salt of the present invention do not exhibit toxicity in administration in an amount producing pharmacological effects. The compound of the present invention can be administered as a PARP inhibitor and a preventive or therapeutic agent against the above-listed diseases, singly or in combination with another pharmacologically active ingredient. Such pharmacologically active ingredients include, for example, other known therapeutic drugs against ischemic heart diseases, such as calcium antagonists, nitrite drugs, beta blockers and antiplatelet drugs.

For administration in combination, a mixture containing both the compound of the present invention and the pharmacologically active ingredient may be administrated, or separate drugs respectively containing the compound of the present invention and the pharmacologically active ingredient may be administrated at one time or with a time shift. How these drugs are administered is no objection, as long as the drugs are simultaneously present in the blood of the patient.

Diseases against which the medical drug of the present invention is effective will now be described.

The medical drug of the present invention is effective against diseases in which the PARP activity seems to be increasing, that is, diseases in which PARP is activated due to DNA damage resulting from the production of reactive oxygen species, such as peroxynitrite and hydroxy radicals, or radiation, and retrovirus infection (PARP may be involved in the reverse transcription of virus RNA to DNA). Examples of such diseases include myocardial infarction, angina pectoris, arrhythmia, cardiac failure, myocardial ischemia reperfusion injury, other ischemic heart diseases, heart transplantion, pulmonary hypertension, cerebral ischemic injury, cerebral infarction, apoplexy, sequelae of apoplexy, brain edema, damage of organs (gastrointestinal tract, skeletal muscle, kidney, retinas, cochlea, etc.) resulting from ischemia or ischemia-reperfusion, renal failure, multiple organ failure, septicemia, shock, inflammatory bowel diseases(ulcerative colitis and Crohn's disease), ataxia telangiectasia, multiple sclerosis, neurodegenerative disease, allergic diseases (asthma, atopic dermatitis, etc.), immunologic diseases (systemic lupus erythematosus (SLE), etc.), brain contusion, head injury, brain or spinal cord injury, Parkinson's disease, Alzheimer's disease, pneumonitis, hepatitis (acute hepatitis, fulminant hepatitis, etc.), uveitis, Huntington's chorea, muscular dystrophy, arthritis, chronic rheumatoid arthritis, psoriasis, AIDS, adult T-cell leukemia, hairy cell leukemia, Sezary syndrome, cutaneous T-cell lymphoma, hepatitis B, cytomegalovirus infectious disease, herpesvirus infectious disease, ARDS (acute respiratory distress syndrome), chronic obstructive pulmonary disease (COPD), arteriosclerosis, diabetes, diabetic nephropathy, diabetic retinopathy, cancer, osteoporosis, gout, hyperalgesia, and side effects by anticancer drug treatment, such as cisplatin toxicity. The medical drug of the present invention can be thought of as an effective preventive or therapeutic agent against these diseases, and also as effective sensitizer for irradiation and chemical therapies against cancer.

### Examples

The present invention will be further described in detail with reference to examples and comparative examples, but it is not limited to these examples.

### <Pharmacological Examples>

Experimental examples of specific pharmacological effects show effects of representative compounds, but these effects do not limit the present invention.

### 1) Measurement of PARP inhibitory activity

PARP activity is determined by measuring the amount of ADP-ribosylated histone protein applied onto an assay plate according to the manual of a measurement kit "PARP Inhibition Assay" (produced by Travigen, catalog number: 4669-96-K). Specifically, 25 µL/well of "2 × PARP cocktail" (containing 800 µM of NAD, 50 µM of biotinylated NAD, and denatured DNA) was added to each well of assay plates (96-well multiplate) previously coated with histone protein. Further added was 12.5 µL/well of test compound diluted with "PARP Buffer" (50 mM Tris-hydrochloric acid buffer (pH 8.0) containing 25 mM of MgCl₂) and 12.5 µL/well (final concentration: 0.3 unit/50 µL/well) of "HSA PARP Enzyme" diluted with "PARP Buffer" in that order. The mixture was allowed to react at room temperature for 30 minutes. Enzyme reaction of PARP was suspended by discarding the reaction solution and rinsing with a phosphate buffer. Then, 50 µL/well of "Strep-HRP" (streptavidin-labeled horseradish peroxidase) was added and allowed to react at 37°C for 20 to 30 minutes to bind to biotin-labeled ADP-ribosylated histone. After the reaction solution was discarded, resultings were rinsed with a phosphate buffer, and 50 µL/well of colorimetric substrate TACS-Sapphire™ was added to the mixture to react at room temperature for 5 to 10 minutes in the dark. The reaction was terminated by adding 50 µL/well of 0.2 N hydrochloric acid solution, and the absorbance at 450 nm was measured with a microplate reader. The compound of the present invention inhibited PARP activity with IC₅₀ value of about 1 nM to 1 µM. Concrete examples are shown in Table 1.

**Table 1**

| Example compound number | IC₅₀ (µM) |
|---|---|
| 4 | 0.058 |
| 17 | 0.24 |
| 93 | 0.006 |
| 99 | 0.042 |
| 100 | 0.17 |
| 122 | 0.12 |
| 123 | 0.11 |
| 129 | 0.049 |

### 2) Measurement of inhibitory effect against active oxygen-induced cell injury with U937 cells

U937 cells were suspended in RPMI 1640 without serum, and were seeded at a concentration of 1 × 10⁴ cells/50 µL/well in a solid assay plate (produced by Corning, black, tissue culture treated, Type: 3916). After adding 1 µL/well of test compound solution in DMSO, 50 µL/well of 20 mM hydrogen peroxide solution was added, followed by incubation at 37°C for 3 hours in an atmosphere of 5% CO₂. Then, 2 µL/well of 1.5 mM propidium iodide solution was added and incubation was further carried out at 37°C for 1 hour. The fluorescence was measured with a fluorescence plate reader ARVO (excitation wavelength: 530 nm, fluorescence wavelength: 620 nm) to investigate hydrogen peroxide solution-induced necrosis inhibition. As shown in Table 2, the present example showed that the compound of the present invention exhibited an inhibitory effect against active oxygen-induced cell injury.

**Table 2**

| Example compound number | IC₅₀ (µM) |
|---|---|
| 4 | 0.61 |
| 17 | 1.2 |
| 66 | 1.7 |
| 123 | 0.30 |

### <Pharmaceutical Preparation Example>

The following will describe examples of the pharmaceutical composition of the present invention. Compound M described herein is the compound represented by Formula (I) or its salt acceptable as a medical drug (pharmaceutically acceptable salt), and more specifically, it is any one of the compounds in the examples.

### (a) Tablets (active ingredient content: 1 mg)

Weighed out were 1.0 g of Compound M, 90.0 g of lactose, 5.0 g of carboxymethylcellulose sodium, 1.0 g of cornstarch paste (5% W/V paste) and 1.0 g of magnesium stearate. The mixture of these materials was formed into tablets of 100 mg each by a common process.

### (b) Tablets (active ingredient content: 10 mg)

Weighed out were 10 g of Compound M, 150 g of lactose, 6.0 g of sodium croscarmellose, 28.5 g of cornstarch paste (5% W/V paste), 2.5 g of polyvinylpyrrolidone, and 3 g of magnesium stearate. The mixture of these materials was formed into tablets of 200 mg each, and the tables were coated with cellulose acetate phthalate to yield enteric-coated tablets.

### (c) Capsules (active ingredient content: 50 mg)

Weighed out were 100 g of Compound M, 395.5 g of lactose and 4.5 g of magnesium stearate, followed by homogenous mixing. The mixture in an amount of 250 mg was encapsulated in each of Japanese Pharmacopoeia No. 1 hard capsules.

### (d) Injectable solution (active ingredient content: 0.1 mg/mL)

Mixed were 0.1% W/V of Compound M, 2.3% W/V of sodium phosphate buffer, 0.4% W/V of citric acid, 3.5% W/V of macrogol 400, and distilled injection water to prepare a solution. The solution in an amount of 1 mL was encapsulated in each injection ampule to yield an injectable solution.

### (e) Injectable solution (active ingredient content: 1 mg/mL)

Mixed were 0.1% W/V of Compound M, 2.3% W/V of sodium phosphate buffer, 0.4% W/V of citric acid, 3.5% W/V of Macrogol 400 and distilled injection water to prepare a solution. The solution in an amount of 1 mL was encapsulated in each injection ampule to yield an injectable solution.

### <<Synthesis Examples>>

The present invention will be further described in detail with reference to synthesis examples and comparative examples, but it is not limited to these examples.

### EXAMPLE 1

Synthesis of 3-(4-methoxyphenyl)-6,7-dihydro-3H,5H-pyrido[3,2,1-ij]quinazoline-1,7(2H)-dione

### <Step 1> Synthesis of 3-(2-bromophenylamino)propionic acid

To 440 mL of water were added 275 g of 2-bromoaniline and 220 mL of acrylic acid. The mixture was heated at 100°C for 4 hours and subsequently cooled to room temperature. The resulting precipitated crystals were collected by filtration and rinsed with water. Thus, 323 g of pale yellow crystal was obtained.

### <Step 2> Synthesis of 8-bromotetrahydroquinolinone

Polyphosphoric acid in an amount of 3.5 kg was prepared and its external temperature was set at 60°C. To this material was gradually added 352 g of the product of the first step. The internal temperature was set at 100°C and the mixture was stirred with heating for 10 hours. The resulting red viscous solution was added to ice water. This solution was subjected to extraction with ethyl acetate.

The collected ethyl acetate solution was washed with saturated sodium bicarbonate solution, water, and saturated brine, followed by drying with anhydrous sodium sulfate. The solvent was removed under reduced pressure. The resulting reddish brown residue was purified by silica gel column chromatography to obtain 231 g of light reddish yellow titled compound.

### <Step 3> Synthesis of 8-cyanotetrahydroquinolinone

In 700 mL of N,N-dimethylacetamide were suspended 3 g of tris(dibenzylideneacetone)dipalladium (O), 8 g of diphenylphosphinoferrocene, 85.2 g of zinc cyanide and 1 g of metallic zinc, followed by stirring at room temperature for 1 hour. To this suspension was added a solution of 220 g of the compound obtained in the Step 2 dissolved in 800 mL of N,N-dimethylacetamide. The reaction system was purged with nitrogen and stirred at 90°C for 2 hours.

After cooling to room temperature, ethyl acetate was added to the reaction liquid. The precipitated crystals were filtered through Cerite. To the black filtrate was added 10% aqueous ammonia. The resulting solution was extracted with ethyl acetate. The combined ethyl acetate layer was washed with water and saturated brine, and dried over anhydrous sodium sulfate. The solvent was removed under reduced pressure. The resulting residue was dissolved in tetrahydrofuran by heating, and recrystallized to obtain 123 g of yellowish brown crystal.

### <Step 4> Synthesis of tetrahydroquinolinone-8-carboxylic acid amide

To 330 g of 90% sulfuric acid was added 126.8 g of the cyano compound obtained in the Step 3 while the solution was vigorously stirred at room temperature. The mixture was stirred at an internal temperature of 60°C for 20 hours. After cooling to room temperature, ice water was little by little added to the resulting tangerine solution to precipitate crystals. The tangerine crystals were separated by filtration. The resulting crystals were sufficiently washed with water, and then dried under reduced pressure to yield 111 g of orange crystals.

### <Step 5> Synthesis of 3-(4-methoxyphenyl)-6,7-dihydro-3H,5H-pyrido[3,2,1-ij]quinazoline-1,7(2H)-dione

To a mixed solution containing 50 g of the amide compound obtained in the Step 4, 52.7 g of para-anisaldehyde and 150 mL of dehydrated ethanol, 0.5 mL of concentrated sulfuric acid was added and refluxed at 90°C for 4.5 hours. After allowing to cool, the mixed solution was concentrated. The resulting residue was washed with saturated sodium hydrogencarbonate solution and extracted with ethyl acetate. The combined ethyl acetate solution was washed with water and saturated brine successively. After drying with anhydrous sodium sulfate, the solvent was removed under reduced pressure to obtain 85 g of brown crude liquid. This residue was purified by silica gel column chromatography to obtain 38.7 g of light reddish yellow liquid, the titled compound.

### EXAMPLE 2

### Synthesis of 7-hydroxy-3-(4-methoxyphenyl)-6,7-dihydro-3H,5H-pyrido[3,2,1-ij]quinazoline-1(2H)-one

The compound obtained in Example 1 in an amount of 0.2 g was dissolved in 30 mL of methanol at room temperature. To this solution was added 0.03 g of sodium borohydride under cooling with ice, followed by stirring at room temperature for 1 hour. The reaction mixture was concentrated under reduced pressure, and the resulting residue was dissolved in ethyl acetate, washed with water and saturated brine, and then dried over anhydrous sodium sulfate. The product was purified by silica gel column chromatography to yield 0.1 g of the titled compound.

### EXAMPLE 3

### Synthesis of 7-hydroxyimino-3-(4-methoxyphenyl)-6,7-dihydro-3H, 5H-pyrido[3,2,1-ij]quinazoline-1(2H)

In 32 mL of pyridine and 300 mL of ethanol were suspended 32 g of the compound obtained in Step 5 of Example 1 and 14.4 g of hydroxylamine hydrochloride. The resulting solution was stirred at 80°C for 3 hours and cooled to room temperature. The precipitated crystals were collected by filtration. The crystals was sufficiently washed with water and dried under reduced pressure to obtain 34.1 g of the titled compound as pale yellow crystals.

### EXAMPLE 4

### Synthesis of 7-amino-3-(4-methoxyphenyl)-6,7-dihydro-3H,5H-pyrido[3,2,1-ij]quinazolinone-1(2H)-one

To 340 mL of dimethylformamide was added 1.7 g of 10% palladium-carbon, followed by stirring at room temperature for 1 hour in a hydrogen atmosphere. To the resulting mixture was added 34 g of the compound obtained in Example 3 and 60 mL of 2.5 M hydrogen chloride-methanol solution, followed by stirring at 50°C for 12 hours in a hydrogen atmosphere. The catalyst was separated by filtration, and the pH of the filtrate was adjusted with 1 N sodium hydroxide to pH 8, followed by sufficient stirring. The precipitated crystals were separated by filtration, and the filtrate was concentrated under reduced pressure. The resulting residue was extracted with ethyl acetate. The combined ethyl acetate layer was washed with water and saturated brine successively, and dried over anhydrous potassium carbonate. The solvent was removed under reduced pressure, and the resulting residue (14 g of reddish brown solution) and 39 g of crystals collected by filtration were purified by silica gel column chromatography. The resulting crystals were dissolved in a small amount of methanol, and chloroform was slowly added to the solution to recrystallize. Thus, 14.7 g of light brown crystals were obtained. The mother liquid from which the crystals had been collected by filtration and the fraction that had not been separated with a column were concentrated, followed by repurifying with a column. Similarly, the product was recrystallized to yield 2.0 g of pale yellow crystals. The obtained crystals were mixed, and thus 16.7 g of titled compound was obtained. The reduction in the example is syn-selective, and the resulting product is a mixture of (3S,7S) isomer and (3R,7R) isomer.

### EXAMPLE 5

### Synthesis of 7-amino-3-(4-methoxyphenyl)-6,7-dihydro-3H,5H-pyrido[3,2,1-ij]quinazolinone-1(2H)-one hydrochloride

The compound obtained in Example 4 in an amount of 0.3 g was dissolved in 2 mL of methanol. To this solution was added 0.96 mL of 1 N hydrochloric acid. The solution was crystallized with methyl t-butyl ether to yield 0.2 g of pale yellow crystalline mono-hydrochloride.

### EXAMPLE 6 and EXAMPLE 7

### Synthesis of (-)-(3S,7S)-7-amino-3-(4-methoxyphenyl)-6,7-dihydro-3H, 5H-pyrido[3,2,1-ij]quinazoline-1(2H)-one (Example 6) and (+)-(3R,7R)-7-amino-3-(4-methoxyphenyl)-6,7-dihydro-3H,5H-pyrido-[3,2,1-ij]quinazoline-1(2H)-one (Example 7)

The amino compound obtained in Example 4 in an amount of 16.7 g was separated through Chiralcel AD2 (10 x 50 cm), with n-hexane: ethanol: diethylamine = 85:15:0.1 (140 mL/minute). Thus, 6.6 g of short-time retained product (99.3%ee, Example 6), 6.7 g of long-time retained product (99.1%ee, Example 7) and 1.1 g of mixed product were obtained.

The compound obtained in Example 7 was subjected to crystal diffraction to determine the structure. Specifically, 0.1 g of the compound was recrystallized with methylene chloride and tetrahydrofuran to obtain columnar single crystals. The single crystals were diffracted by using Rigaku R-AXIS IV with a radiation source of CuKα (1.54178 Å, 40 kV, 50 mA). As a result, it was confirmed that the obtained compound was (3R,7R)-amino-3-(4-methoxyphenyl)-6,7-dihydro-3H,5H-pyrido[3,2,1-ij]quinazoline-1(2H)-one. Since Example Compound 6 and Example Compound 7 are obtained by optical separation of Example Compound 4, which is a mixture of the (3S,7S) isomer and the (3R,7R) isomer, the compound obtained in Example 6 is the (3S,7S) isomer.
The data of the crystal structure are shown below.

| | x | y | z |
|---|---|---|---|
| O (1) | 0.5845 | -0.1782 | 0.0879 |
| O (2) | 0.2778 | -0.2770 | 0.4621 |
| N (1) | 0.5268 | -0.0391 | 0.7839 |
| N (2) | 0.2709 | 0.0481 | 0.7719 |
| N (3) | -0.1432 | 0.0085 | 0.7380 |
| C (1) | 0.2796 | -0.4104 | 0.4626 |
| C (2) | 0.3167 | -0.2127 | 0.5283 |
| C (3) | 0.2973 | -0.0828 | 0.5266 |
| C (4) | 0.3322 | -0.0105 | 0.5909 |
| C (5) | 0.3862 | -0.0663 | 0.6587 |
| C (6) | 0.4100 | -0.1961 | 0.6583 |
| C (7) | 0.3769 | -0.2705 | 0.5938 |
| C (8) | 0.4159 | 0.0167 | 0.7295 |
| C (9) | 0.4855 | -0.1204 | 0.8400 |
| C (10) | 0.3134 | -0.1339 | 0.8536 |
| C (11) | 0.2564 | -0.2249 | 0.9049 |
| C (12) | 0.0986 | -0.2292 | 0.9235 |
| C (13) | -0.0047 | -0.1391 | 0.8911 |
| C (14) | 0.0485 | -0.0470 | 0.8403 |
| C (15) | 0.2099 | -0.0441 | 0.8200 |
| C (16) | -0.0595 | 0.0534 | 0.8088 |
| C (17) | 0.0344 | 0.1741 | 0.7931 |
| C (18) | 0.1701 | 0.1455 | 0.7387 |

### EXAMPLE 8

The compound obtained in Example 6 in an amount of 6.6 g was dissolved in 5 mL of methanol, and 1.8 mL of concentrated hydrochloric acid was added to the solution under ice cooling. The solution was recrystallized with 50 mL of isopropanol to obtain 7.3 g of hydrochloride.
Optical rotation [α] ^{D}₂₀°C (20°C, methanol): -230°

### EXAMPLE 9

The polar product obtained in Example 7 in an amount of 6.7 g was dissolved in 25 mL of methanol, and 1.8 mL of concentrated hydrochloric acid was added to the solution under ice cooling. The solution was recrystallized with 50 mL of isopropanol to yield 6.8 g of hydrochloride.
Optical rotation [α] ^{D}₂₀°C (20°C, methanol): 220°

### EXAMPLE 10

### Synthesis of 3-(4-methoxy-3-nitrophenyl)-6,7-dihydro-1H,5H-pyrido[3,2,1-ij]quinazoline-1,7-(2H)-dione

To 20 mL of dehydrated ethanol were added 3 g of the amino compound obtained in the Step 4 of Example 1 and 2.9 g of 4-methoxy-3-nitrobenzaldehyde. To the resulting mixture was added 0.1 mL of concentrated hydrochloric acid, followed by heating under reflux for 3 hours. After cooling to room temperature, the precipitated crystals were separated by filtration. The crystals were washed with water and diethyl ether to obtain 4.2 g of pale yellow crystals.

### EXAMPLES 11 and 12

### Synthesis of 7-amino-3-(4-methoxy-3-nitrophenyl)-6,7-dihydro-3H,5H-pyrido[3,2,1-ij]quinazoline-1 (2H) one

To 2.5 g of the ketone obtained in Example 10 were added 5.5 g of ammonium acetate, 0.4 g of sodium cyanoborohydride, 25 mL of anhydrous chloroform and 25 mL of anhydrous methanol. The resulting mixture was heated and stirred at 50°C for 18 hours. After cooling to room temperature, the mixture was adjusted to be alkaline with 0.5 N sodium hydroxide, and water was added to partition the mixture. The water layer was extracted with chloroform. The organic layer was combined and washed with water and saturated brine, and dried over anhydrous potassium carbonate. After the drying agents were removed by filtration, the liquid was concentrated under reduced pressure to obtain 3 g of yellow crystals. This product was purified with silica gel to obtain: 0.55 g of less polar product ((3S,7S)-7-amino-3-(4-methoxy-3-nitrophenyl)-6,7-dihydro-3H,5H-pyrido[3,2,1-ij)quinazoline-1 (2H) one and (3R,7R)-7-amino-3-(4-methoxy-3-nitrophenyl)-6,7-dihydro-3H,5H-pyrido[3,2,1-ij]quinazoline-1(2H)one, Example 11); 0.32 g of highly polar product ((3S, 7R)-7-amino-3-(4-methoxy-3-nitrophenyl)-6,7-dihydro-3H,5H-pyrido[3,2,1-ij]quinazoline-1(2H)one and (3R,7S)-7-amino-3-(4-methoxy-3-nitrophenyl)-6,7-dihydro-3H,5H-pyrido[3,2,1-ij]quinazoline-1 (2H) one, Example 12); and 0.35 g of mixed product. This reaction exhibited low syn-anti selectivity and produced two types of mixture different in polarity: mixture of (3S,7S) isomer and (3R,7R) isomer (less polar); and mixture of (3R,7S)isomer and (3S,7R) isomer (highly polar).

### EXAMPLE 13

### Synthesis of 7-(4-N,N-dimethylaminophenyl)methylamino-3-(4-methoxyphenyl)-6,7-dihydro-3H,5H-pyrido[3,2,1-ij]quinazoline-1(2H)-one

In 3 mL of chloroform and 1 mL of methanol were dissolved 0.11 g of 4,4-dimethylaminobenzaldehyde and 0.15 g of the compound obtained in Example 4. To the resulting solution was added 1 mL of 2.5 M hydrogen chloride solution in methanol, followed by stirring at room temperature for 1 hour. Then, 0.03 g of sodium cyanoborohydride was added. The resulting solution was heated under reflux at 70°C for 3 days. After cooling, the pH of the solution was adjusted to 8 with 1 N sodium hydroxide solution. The water layer was extracted with chloroform. The organic layer was washed with water and saturated brine, and then dried over anhydrous sodium sulfate. The product was purified by silica gel column chromatography to obtain 0.075 g the titled compound as colorless crystals.

### EXAMPLE 14

### Synthesis of 3-(4-methoxyphenyl)-5,6-dihydro-3H-pyrimido[5,6,1-jk][1,4]benzdiazepin-1,8(2H,7H)-dione

The compound obtained in the Step 5 of Example 1 in an amount of 0.15 g was dissolved in 3.5 mL of methanesulfonic acid, and 0.05 g of sodium azide was gradually added to the solution under ice cooling. The solution sparkled and turned green. After stirring at room temperature for 1 hour, the solution was neutralized with saturated sodium bicarbonate solution, extracted with ethyl acetate. The extract was washed with saturated brine and dried over anhydrous sodium sulfate. The product was purified by silica gel thin-layer chromatography to obtain 0.01 g of the titled compound.

Carboxylic amide derivatives (Table 4) which are intermediates of the syntheses were synthesized according to the processes of the Step 1, Step 2, Step 3 and Step 4 of Example 1.

The compounds of Examples 15 to 49 were synthesized according to the process of the fifth step of Example 1.

The compounds of Examples 50 to 79 were synthesized according to the process of Example 3.

The compounds of Examples 80 to 135 were synthesized according to the processes of Examples 4, 10, 11, and 12.

### EXAMPLE 15

### 3-(phenyl)-6,7-dihydro-3H,5H-pyrido[3,2,1-ij]quinazoline-1,7(2H)-dione

### EXAMPLE 16

### 3-(4-cyanophenyl)-6,7-dihydro-3H,5H-pyrido[3,2,1-ij]quinazoline-1,7(2H)-dione

### EXAMPLE 17

### 3-(4-nitrophenyl)-6,7-dihydro-3H,5H-pyrido[3,2,1-ij]quinazoline-1,7(2H)-dione

### EXAMPLE 18

### 3-(4-methylphenyl)-6,7-dihydro-3H,5H-pyrido[3,2,1-ij]quinazoline-1,7(2H)-dione

### EXAMPLE 19

### 3-(4-fluorophenyl)-6,7-dihydro-3H,5H-pyrido[3,2,1-ij]quinazoline-1,7(2H)-dione

### EXAMPLE 20

### 3-(3,4-difluorophenyl)-6,7-dihydro-3H,5H-pyrido[3,2,1-ij]quinazoline-1,7(2H)-dione

### EXAMPLE 21

### 3-(4-bromophenyl)-6,7-dihydro-3H, 5H-pyrido[3,2,1-ij] quinazoline-1,7 (2H) -dione

### EXAMPLE 22

### 3-(4-carboxyphenyl)-6,7-dihydro-3H,5H-pyrido[3,2,1-ij]quinazoline-1,7(2H)-dione

### EXAMPLE 23

### 3-(4-methoxyphenyl)-9-trifluoromethoxy-6,7-dihydro-3H,5H-pyrido[3,2,1-ij]quinazoline-1,7(2H)-dione

### EXAMPLE 24

### 9-fluoro-3-(4-methoxyphenyl)-6,7-dihydro-3H,5H-pyrido[3,2,1-ij]quinazoline-1,7(2H)-dione

### EXAMPLE 25

### 8-chloro-3-(4-methoxyphenyl)-6,7-dihydro-3H,5H-pyrido[3,2,1-ij]quinazoline-1,7(2H)-dione

### EXAMPLE 26

### 3-(4-(3-N,N-dimethylaminopropoxy)phenyl)-6,7-dihydro-3H, 5H-pyrido[3,2,1-ij]quinazoline-1,7(2H)-dione

### EXAMPLE 27

### 3-(3-bromo-4-methoxyphenyl)-6,7-dihydro-3H,5H-pyrido[3,2,1-ij]quinazoline-1,7(2H)-dione

### EXAMPLE 28

### 3-(3-hydroxy-4-methoxyphenyl)-6,7-dihydro-3H,5H-pyrido[3,2,1-ij]quinazoline-1,7(2H)-dione

### EXAMPLE 29

### 3-(3-bromo-4,5-dimethoxyphenyl)-6,7-dihydro-3H,5H-pyrido[3,2,1-ij]quinazoline-1,7(2H)-dione

### EXAMPLE 30

### 3-(4-methoxy -3-methylphenyl)-6,7-dihydro-3H,5H-pyrido[3,2,1-ij]quinazoline-1,7(2H)-dione

### EXAMPLE 31

### 3-[4-(imidazole-1-yl)phenyl]-6,7-dihydro-3H,5H-pyrido[3,2,1-ij]quinazoline-1,7(2H)-dione

### EXAMPLE 32

### 3-(4-ethoxycarbonylmethyloxyphenyl)-6,7-dihydro-3H,5H-pyrido[3,2,1-ij]quinazoline-1,7(2H)-dione

### Example 33

### 3-(4-hydroxyethyloxyphenyl)-6,7-dihydro-3H,5H-pyrido[3,2,1-ij]quinazoline-1,7(2H)-dione

### EXAMPLE 34

### 3-(4-hydroxy-3-methoxyphenyl)-6,7-dihydro-3H,5H-pyrido[3,2,1-ij]quinazoline-1,7(2H)-dione

### EXAMPLE 35

### 3-(4-trifluoromethylphenyl)-6,7-dihydro-3H, 5H-pyrido[3,2,1-ij]quinazoline-1,7(2H)-dione

### EXAMPLE 36

### 3-(4-fluoro-3-methoxyphenyl)-6,7-dihydro-3H,5H-pyrido[3,2,1-ij]quinazoline-1,7(2H)-dione

### EXAMPLE 37

### 3-(4-isopropoxyphenyl)-6,7-dihydro-3H,5H-pyrido[3,2,1-ij]quinazoline-1,7(2H)-dione

### EXAMPLE 38

### 3-(3,4-dimethoxyphenyl)-6,7-dihydro-3H,5H-pyrido[3,2,1-ij]quinazoline-1,7(2H)-dione

### EXAMPLE 39

### 3-(3-fluoro-4-methoxyphenyl)-6,7-dihydro-3H,5H-pyrido[3,2,1-ij]quinazoline-1,7(2H)-dione

### EXAMPLE 40

### 3-(3-pyridyl)-6,7-dihydro-3H,5H-pyrido[3,2,1-ij]quinazoline-1,7(2H)-dione

### EXAMPLE 41

### 3-(4-methoxyphenyl)methyl-6,7-dihydro-5H-pyrido [3,2,1-ij] quinazoline-1,7-dione

### EXAMPLE 42

### 3-(3-chloro-4-methoxyphenyl)-6,7-dihydro-3H,5H-pyrido[3,2,1-ij]quinazoline-1,7(2H)-dione

### EXAMPLE 43

### 3-(2-methoxyphenyl)-6,7-dihydro-3H,5H-pyrido[3,2,1-ij]quinazoline-1,7(2H)-dione

### EXAMPLE 44

### 3-(3-methoxyphenyl)-6,7-dihydro-3H,5H-pyrido[3,2,1-ij]quinazoline-1,7(2H)-dione

### EXAMPLE 45

### 3-(4-trifluoromethylthiophenyl)-6,7-dihydro-3H,5H-pyrido[3,2,1-ij]quinazoline-1,7(2H)-dione

### EXAMPLE 46

### 3-(3-thienyl)-6,7-dihydro-3H,5H-pyrido[3,2.1-ij]quinazoline-1,7(2H)-dione

### EXAMPLE 47

### 3-(6-methoxynaphthalene-2-yl)-6,7-dihydro-3H,5H-pyrido[3,2,1-ij]quinazoline-1,7(2H)-dione

### EXAMPLE 48

### 3-(4-N,N-dimethylaminophenyl)-6,7-dihydro-3H,5H-pyrido[3,2,1-ij]quinazoline-1,7(2H)-dione

### EXAMPLE 49

### 3-(2-methyl-4-methoxyphenyl)-6,7-dihydro-3H,5H-pyrido[3,2,1-ij]quinazoline-1,7(2H)-dione

### EXAMPLE 50

### 7-hydroxyimino-3-(4-methoxyphenyl)-9-trifluoromethoxy-6,7-dihydro-3H,5H-pyrido[3,2,1-ij]quinazoline-1(2H)-one

### EXAMPLE 51

### 9-fluoro-7-methoxyimino-3-(4-methoxyphenyl)-6,7-dihydro-3H,5H-pyrido[3,2,1-ij]quinazoline-1(2H)-one

### EXAMPLE 52

### 8-chloro-7-hydroxyimino-3-(4-methoxyphenyl)-6,7-dihydro-3H,5H-pyrido[3,2,1-ij]quinazoline-1(2H)-one

### EXAMPLE 53

### 7-methoxyimino-3-[4-(3-N,N'-dimethylaminopropoxy)phenyl]-6,7-dihydro-3H, 5H-pyrido[3,2,1-ij]quinazoline-1 (2H)-one

### EXAMPLE 54

### (3-bromo-4-methoxyphenyl)-7-methoxyimino-6,7-dihydro-3H,5H-pyrido[3,2,1-1j]quinazoline-1(2H)-one

### EXAMPLE 55

### 3-(3-hydroxy-4-methoxyphenyl)-7-methoxyimino-6,7-dihydro-3H,5H-pyrido[3,2,1-ij]quinazoline-1(2H)-one

### EXAMPLE 56

### 3-(3-bromo-4,5-dimethoxyphenyl)-7-hydroxyimino-6,7-dihydro-3H,5H-pyrido[3,2,1-ij]quinazoline-1(2H)-one

### EXAMPLE 57

### 7-hydroxyimino-3-(3-methyl-4-methoxyphenyl)-6,7-dihydro-3H,5H-pyrido[3,2,1-ij]quinazoline-1(2H)-one

### EXAMPLE 58

### 3-[4-(imidazole-1-yl)phenyl]-7-methoxyimino-6,7-dihydro-3H,5H-pyrido[3,2,1-ij]quinazoline-1(2H)-one

### EXAMPLE 59

### 3-(4-ethoxycarbonylmethoxyphenyl)-7-hydroxyimino-6,7-dihydro-3H,5H-pyrido[3,2,1-ij]quinazoline-1(2H)-one

### EXAMPLE 60

### 7-hydroxyimino-3-(4-hydroxyethoxyphenyl)-6,7-dihydro-3H,5H-pyrido[3,2,1-ij]quinazoline-1(2H)-one

### EXAMPLE 61

### 7-hydroxyimino-3-(4-hydroxy-3-methoxyphenyl)-6,7-dihydro-3H,5H-pyrido[3,2,1-ij]quinazoline-1(2H)one

### EXAMPLE 62

### 7-methoxyimino-3-(4-trifluoromethylphenyl)-6,7-dihydro-3H,5H-pyrido[3,2,1-ij]quinazoline-1(2H)-one

### EXAMPLE 63

### 3-(4-fluoro-3-methoxyphenyl)-7-hydroxyimino-6,7-dihydro-3H, 5H-pyrido[3,2,1-ij]quinazoline-1(2H)-one

### EXAMPLE 64

### 3-(4-isopropoxyphenyl)-7-methoxyimino-6,7-dihydro-3H,5H-pyrido[3,2,1-ij]quinazoline-1(2H)-one

### EXAMPLE 65

### 3-(3,4-dimethoxyphenyl)-7-methoxyimino-6,7-dihydro-3H, 5H-pyrido[3,2,1-ij]quinazoline-1(2H)-one

### EXAMPLE 66

### 3-(3-fluoro-4-methoxyphenyl)-7-hydroxyimino-6,7-dihydro-3H,5H-pyrido[3,2,1-ij]quinazoline-1(2H)-one

### EXAMPLE 67

### 7-methoxyimino-3-(3-pyridyl)-6,7-dihydro-3H, 5H-pyrido[3,2,1-ij]quinazoline-1(2H)-one

### EXAMPLE 68

### 7-methoxyimino-3-(4-methoxyphenyl)methyl-6,7-dihydro-5H-pyrido[3,2,1-ij]quinazoline-1(2H)-one

### EXAMPLE 69

### 7-methoxyimino-3-(4-methoxyphenyl)methyl-6,7-dihydro-3H,5H-pyrido[3,2,1-ij]quinazoline-1(2H)-one

### EXAMPLE 70

### 3-(3-chloro-4-methoxyphenyl)-7-hydroxyimino-3-6,7-dihydro-3H,5H-pyrido[3,2,1-ij]quinazoline-1(2H)-one

### EXAMPLE 71

### 7-methoxyimino-3-(4-nitrophenyl)-6,7-dihydro-3H,5H-pyrido[3,2,1-ij]quinazoline-1(2H)-one

### EXAMPLE 72

### 7-hydroxyimino-3-phenyl-6,7-dihydro-3H,5H-pyrido[3,2,1-ij]quinazoline-1(2H)-one

### EXAMPLE 73

### 7-hydroxyimino-3-(4-nitrophenyl)-6,7-dihydro-3H,5H-pyrido[3,2,1-ij]quinazoline-1(2H)-one

### EXAMPLE 74

### 3-(4-cyanophenyl)-7-hydroxyimino-6,7-dihydro-3H,5H-pyrido[3,2,1-ij]quinazoline-1(2H)-one

### EXAMPLE 75

### 3-(3,4-difluorophenyl)-7-hydroxyimino-6,7-dihydro-3H,5H-pyrido[3,2,1-ij]quinazoline-1(2H)-one

### EXAMPLE 76

### 3-(4-fluorophenyl)-7-hydroxyimino-6,7-dihydro-3H,5H-pyrido[3,2,1-ij]quinazoline-1(2H)-one

### EXAMPLE 77

### 7-hydroxyimino-3-(4-methylphenyl)-6,7-dihydro-3H,5H-pyrido[3,2,1-ij]quinazoline-1(2H)-one

### EXAMPLE 78

### 3-(4-chlorophenyl)-7-hydroxyimino)-6,7-dihydro-3H,5H-pyrido[3,2,1-ij]quinazoline-1(2H)-one

### EXAMPLE 79

### 3-(4-bromophenyl)-7-hydroxyimino-6,7-dihydro-3H,5H-pyrido[3,2,1-ij]quinazoline-1(2H)-one

### EXAMPLE 80

### Mixture of (3R,7R)-7-amino-3-(4-methoxyphenyl)-9-trifluoromethoxy-6,7-dihydro-3H,5H-pyrido[3,2,1-ij]quinazoline-1(2H)-one and (3S,7S)-7-amino-3-(4-methoxyphenyl)-9-trifluoromethoxy-6,7-dihydro-3H,5H-pyrido[3,2,1-ij]quinazoline-1(2H)-one

### EXAMPLE 81

### Mixture of (3R,7R)-7-amino-9-fluoro-3-(4-methoxyphenyl)-6,7-dihydro-3H,5H-pyrido[3,2,1-ij]quinazoline-1(2H)-one and (3S,7S)-7-amino-9-fluoro-3-(4-methoxyphenyl)-6,7-dihydro-3H,5H-pyrido[3,2,1-ij]quinazoline-1(2H)-one

### EXAMPLE 82

### Mixture of (3S,7S)-7-amino-8-chloro-3-(4-methoxyphenyl)-6,7-dihydro-3H,5H-pyrido[3,2,1-ij]quinazoline-1(2H)-one and (3R,7R)-7-amino-8-chloro-3-(4-methoxyphenyl)-6,7-dihydro-3H,5H-pyrido[3,2,1-ij]quinazoline-1(2H)-one

### EXAMPLE 83

### Mixture of (3S,7R)-7-amino-8-chloro-3-(4-methoxyphenyl)-6,7-dihydro-3H,5H-pyrido[3,2,1-ij]quinazoline-1(2H)-one and (3R,7S)-7-amino-8-chloro-3-(4-methoxyphenyl)-6,7-dihydro-3H,5H-pyrido[3,2,1-ij]quinazoline-1(2H)-one

### EXAMPLE 84

### Mixture of (3S,7S)-7-amino-3-[4-(3-N,N-dimethylaminopropoxy)phenyl]-6,7-dihydro-3H, 5H-pyrido[3,2,1-ij]quinazoline-1(2H)-one and (3R,7R)-7-amino-3-[4-(3-N,N-dimethylaminopropoxy)phenyl]-6,7-dihydro-3H,5H-pyrido[3,2,1-ij]quinazoline-1(2H)-one

### EXAMPLE 85

### Mixture of (3S,7S)-7-amino-3-(3-bromo-4-methoxyphenyl)-6,7-dihydro-3H,5H-pyrido[3,2, 1-ij]quinazoline-1 (2H)-one and (3R,7R)-7-amino-3-(3-bromo-4-methoxyphenyl)-6,7-dihydro-3H,5H-pyrido[3,2,1-ij]quinazoline-1(2H)-one

### EXAMPLE 86

### Mixture of (3R,7S)-7-amino-3-(3-bromo-4-methoxyphenyl)-6,7-dihydro-3H,5H-pyrido[3,2,1-ij]quinazoline-1(2H)-one and (3S,7R)-7-amino-3-(3-bromo-4-methoxyphenyl)-6,7-dihydro-3H, 5H-pyrido[3,2,1-ij]quinazoline-1(2H)-one

### EXAMPLE 87

### Mixture of (3S,7S)-7-amino-3-(3-hydroxy-4-methoxyphenyl)-6,7-dihydro-3H,5H-pyrido[3,2,1-ij]quinazoline-1(2H)-one and (3R,7R)-7-amino-3-(3-hydroxy-4-methoxyphenyl)-6,7-dihydro-3H,5H-pyrido[3,2,1-ij]quinazoline-1(2H)-one

### EXAMPLE 88

### Mixture of (3S,7S)-7-amino-3-(3-bromo-4,5-dimethoxyphenyl)-6,7-dihydro-3H,5H-pyrido[3,2,1-ij]quinazoline-1(2H)-one and (3R,7R)-7-amino-3-(3-bromo-4,5-dimethoxyphenyl)-6,7-dihydro-3H, 5H-pyrido[3,2,1-ij]quinazoline-1(2H)-one

### EXAMPLE 89

### Mixture of (3R,7S)-7-amino-3-(3-bromo-4,5-dimethoxyphenyl)-6,7-dihydro-3H,5H-pyrido[3,2,1-ij]quinazoline-1(2H)-one and (3S,7R)-7-amino-3-(3-bromo-4,5-dimethoxyphenyl)-6,7-dihydro-3H,5H-pyrido[3,2,1-ij]quinazoline-1(2H)-one

### EXAMPLE 90

### Mixture of (3S,7S)-7-amino-3-(3-methyl-4-methoxyphenyl)-6,7-dihydro-3H,5H-pyrido[3,2,1-ij]quinazoline-1(2H)-one and (3R,7R)-7-amino-3-(3-methyl-4-methoxyphenyl)-6,7-dihydro-3H,5H-pyrido[3,2,1-ij]quinazoline-1(2H)-one

### EXAMPLE 91

### Mixture of (3S,7S)-7-amino-3-[4-(imidazole-1-yl)phenyl]-6,7-dihydro-3H,5H-pyrido[3,2,1-ij]quinazoline-1(2H)-one and (3R,7R)-7-amino-3-[4-(imidazole-1-yl)phenyl]-6,7-dihydro-3H,5H-pyrido[3,2,1-ij]quinazoline-1(2H)-one

### EXAMPLE 92

### Mixture of (3S,7S)-7'amino-3-(4-ethoxycarbonylmethoxyphenyl)-6,7-dihydro-3H,5H-pyrido[3,2,1-ij]quinazoline-1(2H)-one and (3R,7R)-7-amino-3-(4-ethoxycarbonylmethoxyphenyl)-6,7-dihydro-3H,5H-pyrido[3,2,1-ij]quinazoline-1(2H)-one

### EXAMPLE 93

### Mixture of (3S,7S)-7-amino-3-(4-hydroxyethoxyphenyl)-6,7-dihydro-3H,5H-pyrido[3,2,1-ij]quinazoline-1(2H)-one and (3R,7R)-7-amino-3-(4-hydroxyethoxyphenyl)-6,7-dihydro-3H,5H-pyrido[3,2,1-ij]quinazoline-1(2H)-one

### EXAMPLE 94

### Mixture of (3S,7S)-7-amino-3-(4-hydroxy -3-methoxyphenyl)-6,7-dihydro-3H,5H-pyrido[3,2,1-ij]quinazoline-1(2H)-one and (3R,7R)-7-amino-3-(4-hydroxy-3-methoxyphenyl)-6,7-dihydro-3H,5H-pyrido[3,2,1-ij]quinazoline-1(2H)-one

### EXAMPLE 95

### Mixture of (3S,7S)-7-amino-3-(4-trifluoromethylphenyl)-6,7-dihydro-3H,5H-pyrido[3,2,1-ij]quinazoline-1(2H)-one and (3R,7R)-7-amino-3-(4-trifluoromethylphenyl)-6,7-dihydro-3H,5H-pyrido[3,2,1-ij]quinazoline-1(2H)-one

### EXAMPLE 96

### Mixture of (3S,7S)-7-amino-3-(4-fluoro-3-methoxyphenyl)-6,7-dihydro-3H, 5H-pyrido[3,2,1-ij]quinazoline-1(2H)-one and (3R,7R)-7-amino-3-(4-fluoro-3-methoxyphenyl)-6,7-dihydro-3H,5H-pyrido[3,2,1-ij]quinazoline-1(2H)-one

### EXAMPLE 97

### Mixture of (3S,7S)-7-amino-3-(4-isopropoxyphenyl)-6,7-dihydro-3H,5H-pyrido[3,2,1-ij]quinazoline-1(2H)-one and (3R,7R)-7-amino-3-(4-isopropoxyphenyl)-6,7-dihydro-3H,5H-pyrido[3,2,1-ij]quinazoline-1(2H)-one

### EXAMPLE 98

### Mixture of (3S,7S)-7-amino-3-(3,4-dimethoxyphenyl)-6,7-dihydro-3H, 5H-pyrido [3,2,1-ij]quinazoline-1(2H)-one and (3R,7R)-7-amino-3-(4,5-dimethoxyphenyl)-6,7-dihydro-3H, 5H-pyrido[3,2,1-ij]quinazoline-1(2H)-one

### EXAMPLE 99

### Mixture of (3S,7S)-7-amino-3-(3-fluoro-4-methoxyphenyl)-6,7-dihydro-3H,5H-pyrido[3,2,1-ij]quinazoline-1(2H)-one and (3R,7R)-7-amino-3-(3-fluoro-4-methoxyphenyl)-6,7-dihydro-3H, 5H-pyrido[3,2,1-ij]quinazoline-1(2H)-one

### EXAMPLE 100

### Mixture of (3S,7S)-7-amino-3-(3-pyridyl)-6,7-dihydro-3H,5H-pyrido[3,2,1-ij]quinazoline-1(2H)-one and (3R,7R)-7-amino-3-(3-pyridyl)-6,7-dihydro-3H,5H-pyrido[3,2,1-ij]quinazoline-1(2H)-one

### EXAMPLE 101

### Mixture of (3S,7S)-7-amino-3-(4-methoxyphenyl)methyl-6,7-dihydro-3H,5H-pyrido[3,2,1-ij]quinazoline-1(2H)-one and (3R,7R)-7-amino-3-(4-methoxyphenyl)methyl-6,7-dihydro-3H,5H-pyrido[3,2,1-ij]quinazoline-1(2H)-one

### EXAMPLE 102

### Mixture of (3R,7S)-7-amino-3-(4-methoxyphenyl)-6,7-dihydro-3H,5H-pyrido[3,2,1-ij]quinazoline-1(2H)-one and (3S,7R)-7-amino-3-(4-methoxyphenyl)-6,7-dihydro-3H,5H-pyrido[3,2,1-ij]quinazoline-1(2H)one

### EXAMPLE 103

### 3-(4-methoxyphenyl)-6,7-dihydro-3H,5H-pyrido[3,2,1-ij]quinazoline-1(2H)-one

### EXAMPLE 104

### Mixture of (3S,7S)-7-amino-3-(2-methoxyphenyl)-6,7-dihydro-3H,5H-pyrido[3,2,1-ij]quinazoline-1(2H)-one and (3R,7R)-7-amino-3-(2-methoxyphenyl)-6,7-dihydro-3H,5H-pyrido[3,2,1-ij]quinazoline-1(2H)-one

### EXAMPLE 105

### Mixture of (3R,7S)-7-amino-3-(2-methoxyphenyl)-6,7-dihydro-3H,5H-pyrido[3,2,1-ij]quinazoline-1(2H)-one and (3S, 7R)-7-amino-3-(2-methoxyphenyl)-6,7-dihydro-3H, 5H-pyrido[3,2,1-ij]quinazoline-1(2H)-one

### EXAMPLE 106

### Mixture of (3S,7S)-7-amino-3-(3-methoxyphenyl)-6,7-dihydro-3H,5H-pyrido[3,2,1-ij]quinazoline-1(2H)-one and (3R,7R)-7-amino-3-(3-methoxyphenyl)-6,7-dihydro-3H,5H-pyrido[3,2,1-ij]quinazoline-1(2H)-one

### EXAMPLE 107

### (3R,7S)-7-amino-3-(3-methoxyphenyl)-6,7-dihydro-3H,5H-pyrido[3,2,1-ij]quinazoline-1(2H)-one and (3S,7R)-7-amino-3-(3-methoxyphenyl)-6,7-dihydro-3H,5H-pyrido[3,2,1-ij-1]quinazoline-1(2H)-one

### EXAMPLE 108

### Mixture of (3S,7S)-7-amino-3-(4-trifluoromethylthiophenyl) -6,7-dihydro-3H, 5H-pyrido[3,2,1-ij]quinazoline-1(2H)-one and (3R,7R)-7-amino-3-(4-trifluoromethylthiophenyl)-6,7-dihydro-3H,5H-pyrido[3,2,1-ij]quinazoline-1(2H)-one

### EXAMPLE 109

### Mixture of (3R,7S)-7-amino-3-(4-trifluoromethylthiophenyl)-6,7-dihydro-3H,5H-pyrido[3,2,1-ij]quinazoline-1(2H)-one and (3S,7R)-7-amino-3-(4-trifluoromethylthiophenyl)-6,7-dihydro-3H,5H-pyrido[3,2,1-ij]quinazoline-1(2H)-one

### EXAMPLE 110

### Mixture of (3S;7S)-7-amino-3-(3-thienyl)-6,7-dihydro-3H,5H-pyrido[3,2,1-ij]quinazoline-1(2H)-one and (3R,7R)-7-amino-3-(3-thienyl)-6,7-dihydro-3H,5H-pyrido[3,2,1-ij]quinazoline-1(2H)-one

### EXAMPLE 111

### Mixture of (3R,7S)-7-amino-3-(3-thienyl)-6,7-dihydro-3H,5H-pyrido[3,2,1-ij]quinazoline-1(2H)-one and (3S,7R)-7-amino-3-(3-thienyl)-6,7-dihydro-3H,5H-pyrido[3,2,1-ij]quinazoline-1(2H)-one

### EXAMPLE 112

### Mixture of (3S,7S)-7-amino-3-(6-methoxynaphthalene-2-yl)-6,7-dihydro-3H,5H-pyrido[3,2,1-ij]quinazoline-1(2H)-one and (3R,7R)-7-amino-3-(6-methoxynaphthalene-2-yl)-6,7-dihydro-3H,5H-pyrido[3,2,1-ij]quinazoline-1(2H)-one

### EXAMPLE 113

### Mixture of (3R,7S)-7-amino-3-(6-methoxynaphthalene-2-yl)-6,7-dihydro-3H,5H-pyrido[3,2,1-ij]quinazoline-1(2H)-one and (3S,7R)-7-amino-3-(6-methoxynaphthalene-2-yl)-6,7-dihydro-3H,5H-pyrido[3,2,1-ij]quinazoline-1(2H)-one

### EXAMPLE 114

### Mixture of (3S,7S)-7-amino-3-phenyl-6,7-dihydro-3H,5H-pyrido[3,2,1-ij]quinazoline-1(2H)-one and (3R,7R)-7-amino-3-phenyl-6,7-dihydro-3H,5H-pyrido[3,2,1-ij]quinazoline-1(2H)-one

### EXAMPLE 115

### Mixture of (3S,7S)-7-amino-3-(4-aminophenyl)-6,7-dihydro-3H,5H-pyrido[3,2,1-ij]quinazoline-1(2H)-one and (3R,7R)-7-amino-3-(4-aminophenyl)-6,7-dihydro-3H,5H-pyrido[3,2,1-ij]quinazoline-1(2H)-one

### EXAMPLE 116

### Mixture of (3S,7S)-7-amino-3-(4-nitrophenyl)-6,7-dihydro-3H,5H-pyrido[3,2,1-ij]quinazoline-1(2H)-one and (3R,7R)-7-amino-3-(4-nitrophenyl)-6,7-dihydro-3H,5H-pyrido[3,2,1-ij]quinazoline-1(2H)-one

### EXAMPLE 117

### Mixture of (3R,7S)-7-amino-3-(4-nitrophenyl)-6,7-dihydro-3H,5H-pyrido[3,2,1-ij]quinazoline-1(2H)-one and (3S,7R)-7-amino-3-(4-nitrophenyl)-6,7-dihydro-3H,5H-pyrido[3,2,1-ij]quinazoline-1(2H)-one

### EXAMPLE 118

### Mixture of (3S,7S)-7-amino-3-(4-methylphenyl)-6,7-dihydro-3H,5H-pyrido[3,2,1-ij]quinazoline-1(2H)-one and (3R,7R)-7-amino-3-(4-methylphenyl)-6,7-dihydro-3H,5H-pyrido[3,2,1-ij]quinazoline-1(2H)-one

### EXAMPLE 119

### Mixture of (3S,7S)-7-amino-3-(4-fluorophenyl)-6,7-dihydro-3H,5H-pyrido[3,2,1-ij]quinazoline-1(2H)-one and (3R,7R)-7-amino-3-(4-fluorophenyl)-6,7-dihydro-3H,5H-pyrido[3,2,1-ij]quinazoline-1(2H)-one

### EXAMPLE 120

### Mixture of (3S,7S)-7-amino-3-(4-cyanophenyl)-6,7-dihydro-3H,5H-pyrido[3,2,1-ij]quinazoline-1(2H)-one and (3R,7R)-7-amino-3-(4-cyanophenyl)-6,7-dihydro-3H,5H-pyrido[3,2,1-ij]quinazoline-1(2H)-one

### EXAMPLE 121

### Mixture of (3R,7S)-7-amino-3-(4-cyanophenyl)-6,7-dihydro-3H,5H-pyrido[3,2,1-ij]quinazoline-1(2H)-one and (3S,7R)-7-amino-3-(4-cyanophenyl)-6,7-dihydro-3H,5H-pyrido[3,2,1-ij]quinazoline-1(2H)-one

### EXAMPLE 122

### Mixture of (3S,7S)-7-amino-3-(3,4-difluorophenyl)-6,7-dihydro-3H, 5H-pyrido[3,2,1-ij]quinazoline-1(2H)-one and (3R, 7R)-7-amino-3-(3,4-difluorophenyl)-6,7-dihydro-3H,5H-pyrido[3,2,1-ij]quinazoline-1(2H)-one

### EXAMPLE 123

### Mixture of (3S,7S)-7-amino-3-(4-bromophenyl)-6,7-dihydro-3H, 5H-pyrido[3,2,1-ij]quinazoline-1(2H)-one and (3R,7R)-7-amino-3-(4-bromophenyl)-6,7-dihydro-3H,5H-pyrido[3,2,1-ij]quinazoline-1(2H)-one

### EXAMPLE 124

### Mixture of (3R,7S)-7-amino-3-(4-bromophenyl)-6,7-dihydro-3H,5H-pyrido[3,2,1-ij]quinazoline-1(2H)-one and (3S,7R)-7-amino-3-(9-bromophenyl)-6,7-dihydro-3H,5H-pyrido[3,2,1-ij]quinazoline-1(2H)-one

### EXAMPLE 125

### Mixture of (3S,7S)-7-amino-3-(4-chlorophenyl)-6,7-dihydro-3H,5H-pyrido[3,2,1-ij]quinazoline-1(2H)-one and (3R,7R)-7-amino-3-(4-chlorophenyl)-6,7-dihydro-3H,5H-pyrido[3,2,1-ij]quinazoline-1(2H)-one

### EXAMPLE 126

### Mixture of (3R,7S)-7-amino-3-(4-chlorophenyl)-6,7-dihydro-3H,5H-pyrido[3,2,1-ij]quinazoline-1(2H)-one and (3S,7R)-7-amino-3-(4-chlorophenyl)-6,7-dihydro-3H,5H-pyrido[3,2,1-ij]quinazoline-1(2H)-one

### EXAMPLE 127

### 7-amino-3-(4-carboxyphenyl)-6,7-dihydro-3H, 5H-pyrido[3,2,1-ij]quinazolinone-1-(2H)-one

### EXAMPLE 128

### Mixture of (3S,7S)-7-amino-3-(4-N,N-dimethylaminophenyl)-6,7-dihydro-3H,5H-pyrido[3,2,1-ij]quinazoline-1(2H)-one and (3R,7R)-7-amino-3-(4-N,N-dimethylaminophenyl)-6,7-dihydro-3H,5H-pyrido[3,2,1-ij]quinazoline-1(2H)-one

### EXAMPLE 129

### Mixture of (3R,7S)-7-amino-3-(4-N,N-dimethylaminophenyl)-6,7-dihydro-3H,5H-pyrido[3,2,1-ij]quinazoline-1(2H)-one and (3S,7R)-7-amino-3-(4-N,N-dimethylaminophenyl)-6,7-dihydro-3H,5H-pyrido[3,2,1-ij]quinazoline-1(2H)-one

### EXAMPLE 130

### Mixture of (3S,7S)-7-amino-3-(2-methyl-4-methoxyphenyl)-6,7-dihydro-3H,5H-pyrido[3,2,1-ij]quinazoline-1(2H)-one and (3R,7R)-7-amino-3-(2-methyl-4-methoxyphenyl)-6,7-dihydro-3H,5H-pyrido[3,2,1-ij]quinazoline-1(2H)-one

### EXAMPLE 131

### Mixture of (3R,7S)-7-amino-3-(2-methyl-4-methoxyphenyl)-6,7-dihydro-3H,5H-pyrido[3,2,1-ij]quinazoline-1(2H)-one and (3S,7R)-7-amino-3-(2-methyl-4-methoxyphenyl)-6,7-dihydro-3H,5H-pyrido[3,2,1-ij]quinazoline-1(2H)-one

### EXAMPLE 132

### 7-(4-N,N-dimethylamïno)benzoylamino-3-(4-methoxyphenyl)-6,7-dihydro-3H,5H-pyrido[3,2,1-ij]quinazoline-1(2H)-one

### EXAMPLE 133

### 7-[3-(piperidine-1-yl)propanoyl]amino-3-(4-methoxyphenyl)-6,7-dihydro-3H, 5H-pyrido[3,2,1-ij]quinazoline-1(2H)-one

### EXAMPLE 134

### 7-(1-methylpiperidine-4-yl)carbonylamino-3-(4-methoxyphenyl)-6,7-dihydro-3H, 5H-pyrido[3,2,1-ij]quinazoline-1(2H)-one

### EXAMPLE 135

### 7-dimethylamino-3-(4-methoxyphenyl)-6,7-dihydro-3H,5H-pyrido[3,2,1-ij]quinazoline-1(2H)-one

### EXAMPLE 136

### 7-(N,N-dimethylaminoacetyl)amino-3-(4-methoxyphenyl)-6,7-dihydro-3H,5H-pyrido[3,2,1-ij]quinazoline-1(2H)-one

Table 3 shows the structures of the Example Compounds; Table 4 shows the structures of synthetic intermediates; Table 5 shows physical property data of the Example Compounds; and Table 6 shows the physical property data of the synthetic intermediates.

### <Example of X-Ray Crystal Structure Analysis>

Examples of a method for obtaining co-crystal of exemplary compounds with the human PARP catalytic fragment and of crystallographic analysis will now be described, but the present invention is not limited to these examples.

### [Construction of Escherichia coli plasmid expressing human PARP catalytic fragment]

Cloning of the human PARP catalytic fragment is performed by the PCR method. PCR is performed 30 cycles at 98°C for 10 seconds, at 55°C for 30 seconds, and at 72°C for 1 minute, with Pyrobest DNA Polymerase (Takara Bio) using a sense primer
(5'-AAAAAGCTTAAAAAAGGGTATAAAATAAAATGAAGCTCACAGTAAATCCTGGCACC-3') and an anti-sense primer
(5'-AAAGTCGACTTACCACAGGGAGGTCTTAAAATTG-3'), and a human colon-derived cDNA library (Clontech) as a template. The resulting PCR product is digested with restriction enzymes Hind III and SalI, and a band of about 1100 bp is collected by agarose gel electrophoresis. Plasmid pM710 (see Japanese Patent Application Laid-open No. Hei 06-25289, Example 10) is digested with Hind III and SalI and collected by agarose gel electrophoresis to be used as a vector fragment. This vector fragment and the PCR-amplified DNA fragment are ligated by a usual method to transform JM109 Competent Cells. The resulting colony is analyzed by PCR and a transformant having a desired plasmid is obtained.

### [Expression by Escherichia Coli and Purification]

The above recombinant is cultured in 5 mL of L-broth containing 50 µg/mL ampicillin at 37°C overnight long. Then, the resulting culture broth is inoculated into 50 µg/mL ampicillin-containing L-broth in the amount of 50 times and cultured at 37°C for about 1 hour. To the culture media is added 3β-indoleacrylic acid (Wako Pure Chemical Industries) at a final concentration of 10 µg/mL to induce expression, followed by culturing at 37°C for another 5 hours. The resulting culture mixture is centrifuged to collect Escherichia coli. The inclusion bodies containing expressed protein are collected from the collected Escherichia coli and washed according to a method of Lin, et al. (Methods in Enzymology 214, 195-224, 1994). The inclusion bodies are dissolved in 8 M urea, 10 mM dithiothreitol, 1 mM reduced glutathione, and 0.1 mM oxidized glutathione, and 20 mM Tris is added to the solution to adjust the pH to 9.0 immediately, according to a method of Lin, et al. (ProNAS, 97, 1456-1460, 2000). The pH of the solution is reduced by 0.2 every 24 hours, to 8.0 by gradually adding 1 M hydrochloric acid, and thus refolding is performed. Only the correctly folded protein is separated from the human PARP catalytic fragment solubilized by refolding, with Sephacryl S-300 (Amersham Bioscience, Tokyo). The human PARP catalytic fragment thus prepared is dialyzed against 20 mM Tris-hydrochloric acid buffer (pH 8.0) containing 50 mM sodium chloride and 14 mM mercaptoethanol to perform buffer substitution.

### [Physical Analysis of Sample to Be Crystallized]

The human PARP catalytic fragment prepared by the above-described process was analyzed to determine whether it is suitable for a sample to be crystallized, as follows.

First, the sample was subjected to SDS-PAGE. As a result, the sample showed a single band by coomassie brilliant blue staining. Subsequently, reverse-phase HPLC analysis was performed for demineralization of the sample and purification test with YMC-PACK Protein-RPS5 (250 mm × 4.6 mm ID, YMC) for a column and an apparatus HP1100 (Yokogawa Analytical Systems) for HPLC. Mobile phase A was 0.08% TFA/pure water, and mobile phase B was 0.05% TFA/acetonitrile. To a column equilibrated with 95% mobile phase A and 5% mobile phase B, 110 µL of about 1 mg/mL sample was applied, and gradient elution was performed until the point of 10% mobile phase A and 90% mobile phase B over a period of 50 minutes. The human PARP catalytic fragment was eluted as a symmetry peak at a retension time of about 33 minutes. This desalted sample in an amount of 30 µL was subjected to 10 cycles of N-terminus analysis with a high sensitive N-terminus amino-acid sequence analyzer Procise 494cLc (Applied Biosystems) a single amino-acid sequence was identified. Thus, it was found that the sample had an intended N-terminus. Subsequently, mass spectroscopy was performed by MALDI-TOFMS. Sinapic acid was dissolved in 50% acetonitrile/0.1% TFA to prepare 10 mg/mL matrix solution. 0.5 µL of the reverse-phase HPLC peak and 0.5 µL of the matrix solution were mixed on a pale to form a spot. After the spot was dried, another 0.5 µL of the reverse-phase HPLC peak was added. The analysis was performed with Voyager DERP (PerSeptive Biosystems) and a spectrum substantially close to that of the theoretical molecular weight was obtained.

The crystallization ability of the sample was measured with a dynamic light scattering molecular size detector DynaPro-MS/X (Protein Solution). The sample after dialysis was centrifuged at 10000 rpm, at 4°C for 10 minutes to remove dust. The supernatant was placed in a cuvette and subjected to measurement continuously 20 times. The sample exhibited monodisperse and the %Polyd value was on the level of 20%. This shows a high probability of crystallization.

Thus, it was confirmed that the human PARP catalytic fragment has purity and physical properties satisfying the requirement for a sample to be crystallized.

### [Crystallization]

The human PARP catalytic fragment was attempted to crystallize in accordance with a method for crystallizing a chicken PARP catalytic fragment, that is, a method of Jung, S, et al. (J Mol Biol, 1994, Vol. 244, pp. 114-116), but no crystal was obtained. Even though possible conditions were varied repeatedly, including the type and concentration of PEG and the concentration and type of salt, no crystal was obtained. However, the inventors surprisingly found that crystallization can be achieved under acidic conditions, in which a reservoir solution of pH 5.2 was used in place that the crystallization should have been performed under basic conditions using pH 8.5 Tris-hydrochloric acid buffer. Details are described below.

The human PARP catalytic fragment sample to be crystallized was concentrated with Centricon 10 (Millipore) to obtain a preparation having a composition of 16 mg/mL human PARP catalytic fragment/20 mM Tris-hydrochloric acid buffer (pH 8.0, containing 50 mM NaCl and 14 mM 2-mercaptoethanol). To 50 µL of the preparation were added 10 µL of 20 mM test compound (dissolved in pure water or 20 mM hydrochloric acid) and 40 µL of 20 mM Tris-hydrochloric acid buffer (pH 8.0, containing 50 mM NaCl and 14 mM 2-mercaptoethanol) to prepare a 8 mg/mL of complex of the human PARP catalytic fragment and a test compound.

Crystallization was performed by hanging-drop vapor diffusion. As a crystallization plate was used a 24-well Linbro Plate (Dainippon Pharmaceutical). Into each of the wells was placed 800 µL of reservoir solution, 0.1 M acetic acid buffer (pH 5.2) containing 15% PEG 4000 and 0.5 M ammonium acetate. A drop was formed on each silicon-treated circular cover glass of 22 mm in diameter. The wells of the plate containing the reservoir solution were tightly covered with the cover glasses turned upside down. The drops were mixed with the reservoir solution to a volume of 2 µL each in a ratio of sample: reservoir solution = 1:1. After setting up, the plate was allowed to stand at 20°C to precipitate crystals.

### [X-ray Diffraction Experiment and Analysis]

The co-crystal prepared above was subjected to X-ray diffraction experiments with Pharmaceutical Industry Beamline (BL32B2) in a large synchrotron radiation facility SPring-8. A CCD detector Jupiter 210 (Rigaku) was used for data collection and the crystals were measured with a 180° coverage by an oscillation photographic method at an oscillation angle of 1°. As a result; a data set with a resolution of 1.7 to 1.8Å was obtained. Integration of the data and calculation of intensity were performed with a program MOSFLM (Collaborative Computational Project, Number 4, 1994, "The CCP4 Suite: Programs for Protein Crystallography", Acta Cryst., D50, 760-763), and scaling was performed with a program SCALA (Collaborative Computational Project, Number 4, 1994, "The CCP4 Suite: Programs for Protein Crystallography", Acta Cryst., D50, 760-763). Also, structure factor calculation was performed with a program TRUNCATE (Collaborative Computational Project, Number 4, 1994, "The CCP4 Suite: Programs for Protein Crystallography", Acta Cryst., D50, 760-763). Phase estimation was performed by molecular replacement using a program AMoRe (Collaborative Computational Project, Number 4, 1994, "The CCP4 Suite: Programs for Protein Crystallography", Acta Cryst., D50, 760-763). The protein part of 2PAW in the Protein Data Bank was used as the search model. For the construction of a model, a program Quanta 2000 (Accelrys) was used. The structure was accurized by simulated annealing with a program CNX (Accelrys). The structure of the crystalline complex was drawn by programs MOLSCRIPT and Raster 3D.

The resulting crystals were the orthorhombic as in the crystals of the known chicken PARP catalytic fragment, but they had a space group of P2₁2₁2 different from the space group of the chicken PARP catalytic fragment, P2₁2₁2₁.

The following Table 7 shows the parameters (crystal system, space groups, and lattice constants) of the obtained crystalline human PARP catalytic fragments. The crystal structure of the co-crystal of Example Compound 9 with the human PARP catalytic fragment, obtained by crystal structure analysis is shown in Fig. 1. The human PARP catalytic fragment is shown by a ribbon model and Example Compound 9 is shown by a ball-and-stick model.

**Table 7**

| Example Compound | Crystal system | Space group | Lattice constant | | |
|---|---|---|---|---|---|
| 9 | Orthorhombic | P2₁2₁2 | a=67.02, | b=92.64, | c=64.46 |
| 11 | Orthorhombic | P2₁2₁2 | a=66.91, | b=92.07, | c=64.34 |
| 13 | Orthorhombic | P2₁2₁2 | a=66.82, | b=92.19, | c=64.50 |

The structures of the example compounds are shown in Table 3; the structures of the synthetic intermediates are shown in Table 4; the physical properties of the example compounds are shown in Table 5; and the physical properties of the synthetic intermediates are shown in Table 6. In Tables 4 and 6, for example, Example Number 1-1 refers to Step 1 in Example 1. In Tables 5 and 6, IR (infrared absorption) was measured by use of potassium bromide tablets and represented in cm⁻¹; and NMR (nuclear magnetic resonance) was measured at 270 and 300 MHz at room temperature with use of TMS (tetramethylsilane) as the internal standard and represented in ppm.

The used solvents are represented as follows: CDCl₃ is heavy chloroform; DMSO-d₆ is heavy dimethyl sulfoxide; CD₃OD is heavy methanol; and CF₃CO₂D is heavy trifluoroacetic acid. The multiplicity of each absorption line is represented as follows: S represents a singlet; d represents a doublet, t represents a triplet; q represents a quartet; dd represents a double doublet; ddd represents a triple doublet; m represents a multiplet; and brs represents a wide singlet. The coupling constant (J) is represented in Hz.

### Industrial Applicability

The compound of the present invention inhibits poly(ADP-ribose)polymerase, and is used as an effective preventive and therapeutic agent against various diseases, such as ischemic diseases (brain, heart, gastrointestinal tract, skeletal muscle, retina, etc.), inflammatory diseases (inflammatory bowel disease, multiple sclerosis, arthritis, chronic rheumatism, etc.), neurodegenerative diseases (extrapyramidal system impairment, Alzheimer's disease, muscular dystrophy, etc.), immunologic diseases (systematic lupus erythematosus (SLE), etc.), allergic diseases (asthma, atopic dermatitis, etc.), lifestyle-related diseases (diabetes, arteriosclerosis, chronic obstructive pulmonary diseases (COPD), etc), shock, head injury, renal failure, and hyperalgesia, and also as an effective antiretroviral agent (against HIV, etc.) and an effective sensitizer for anticancer therapy.

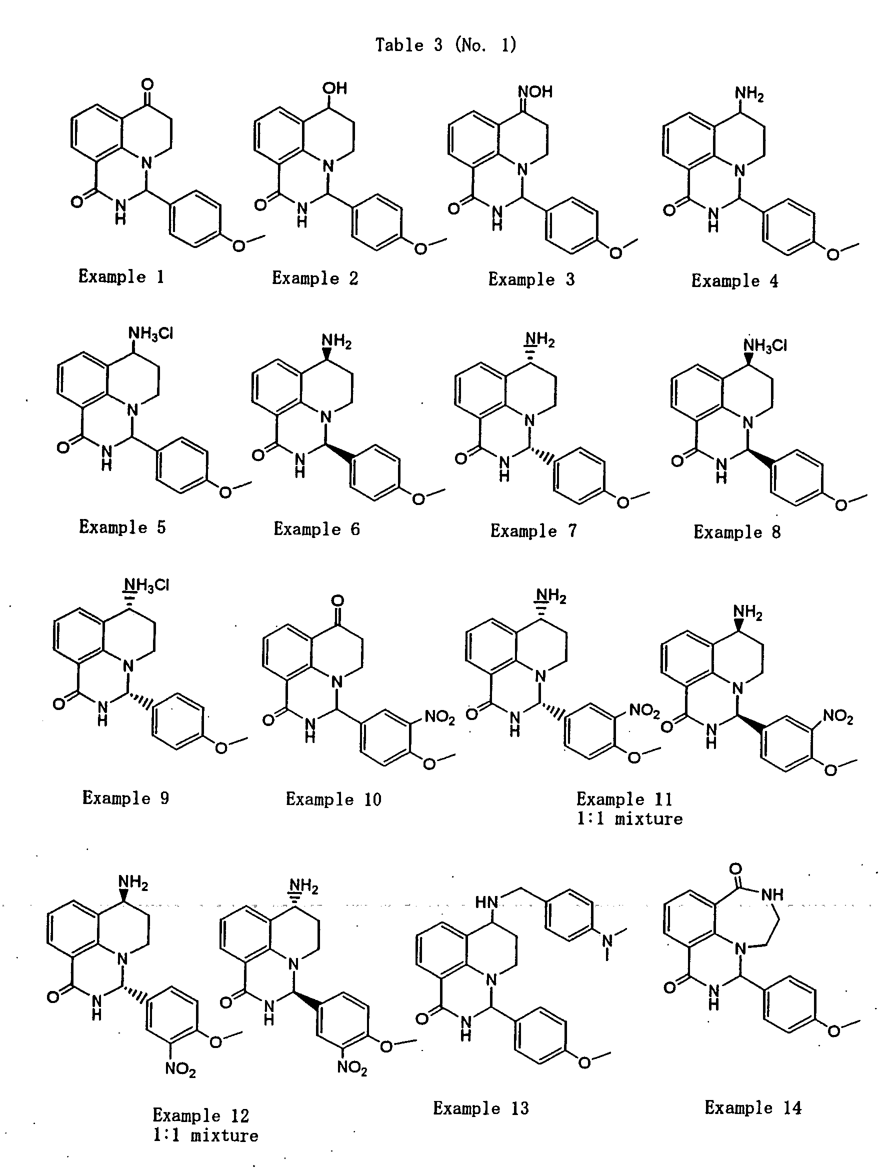

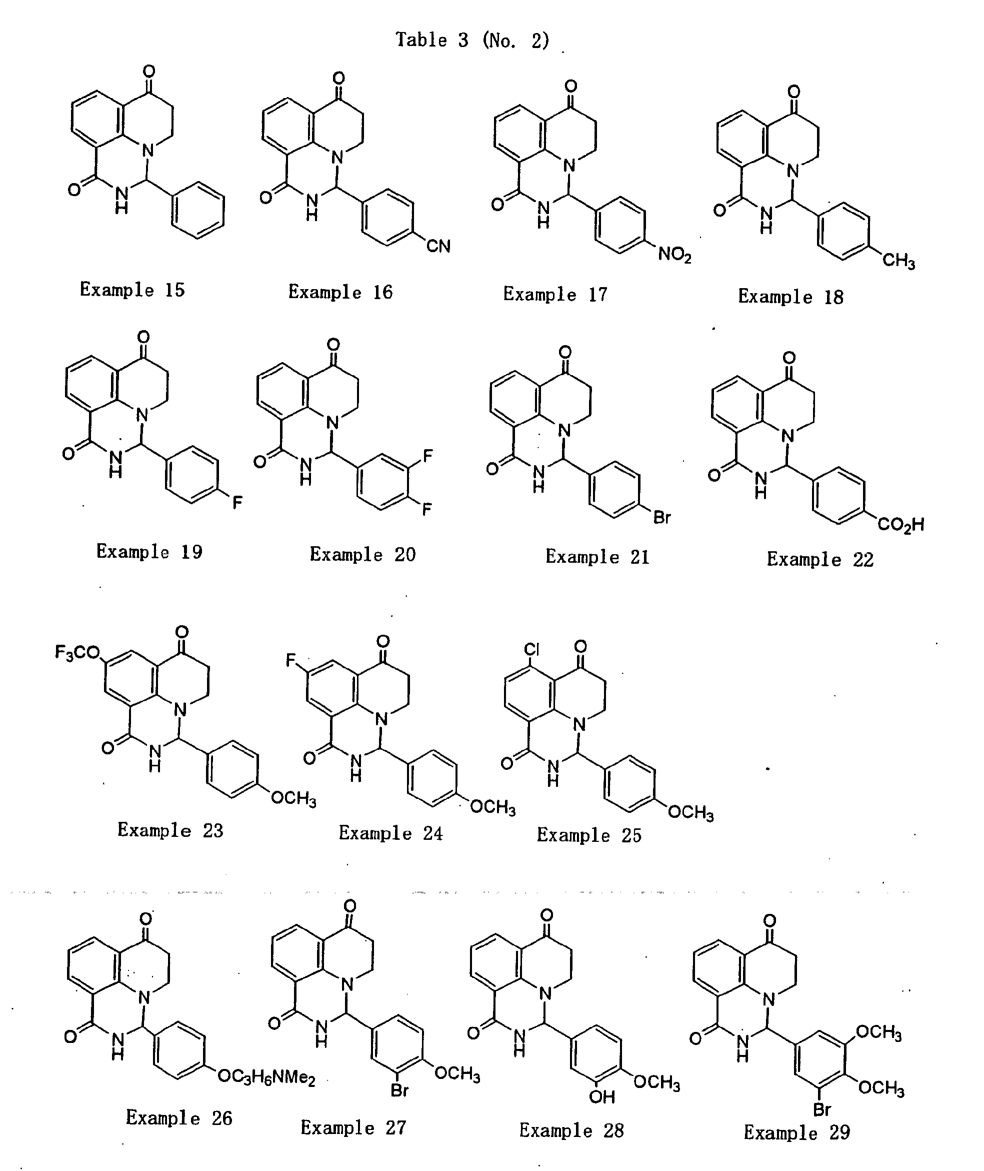

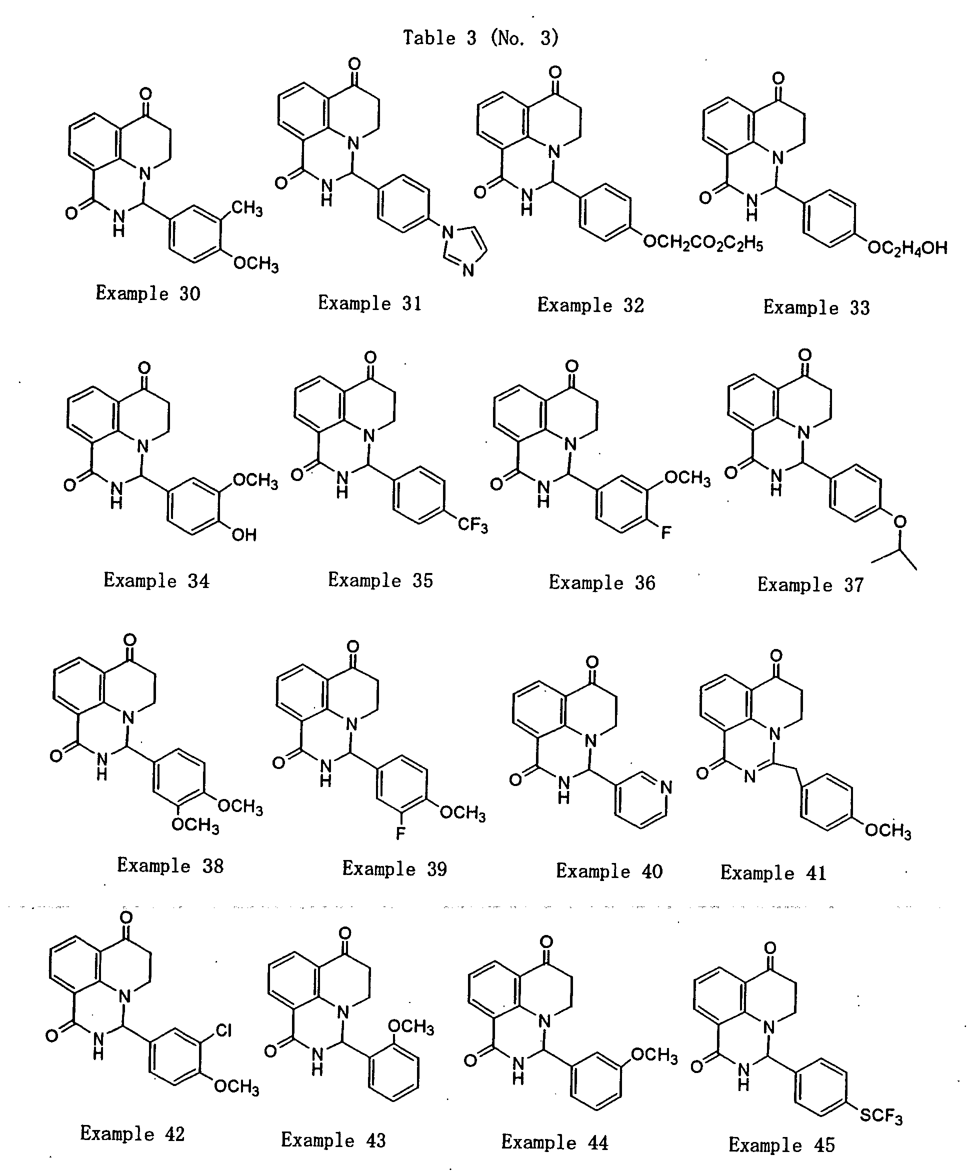

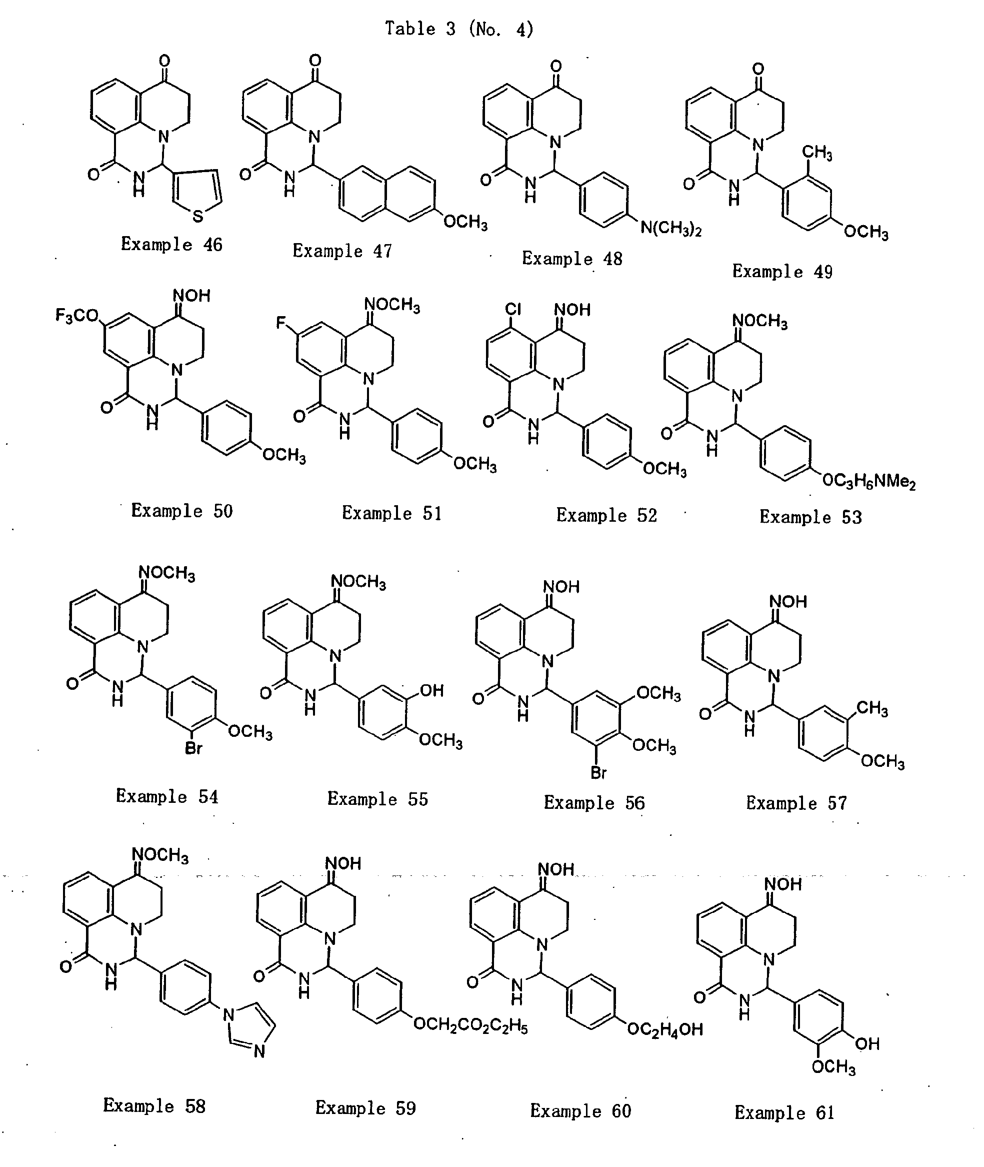

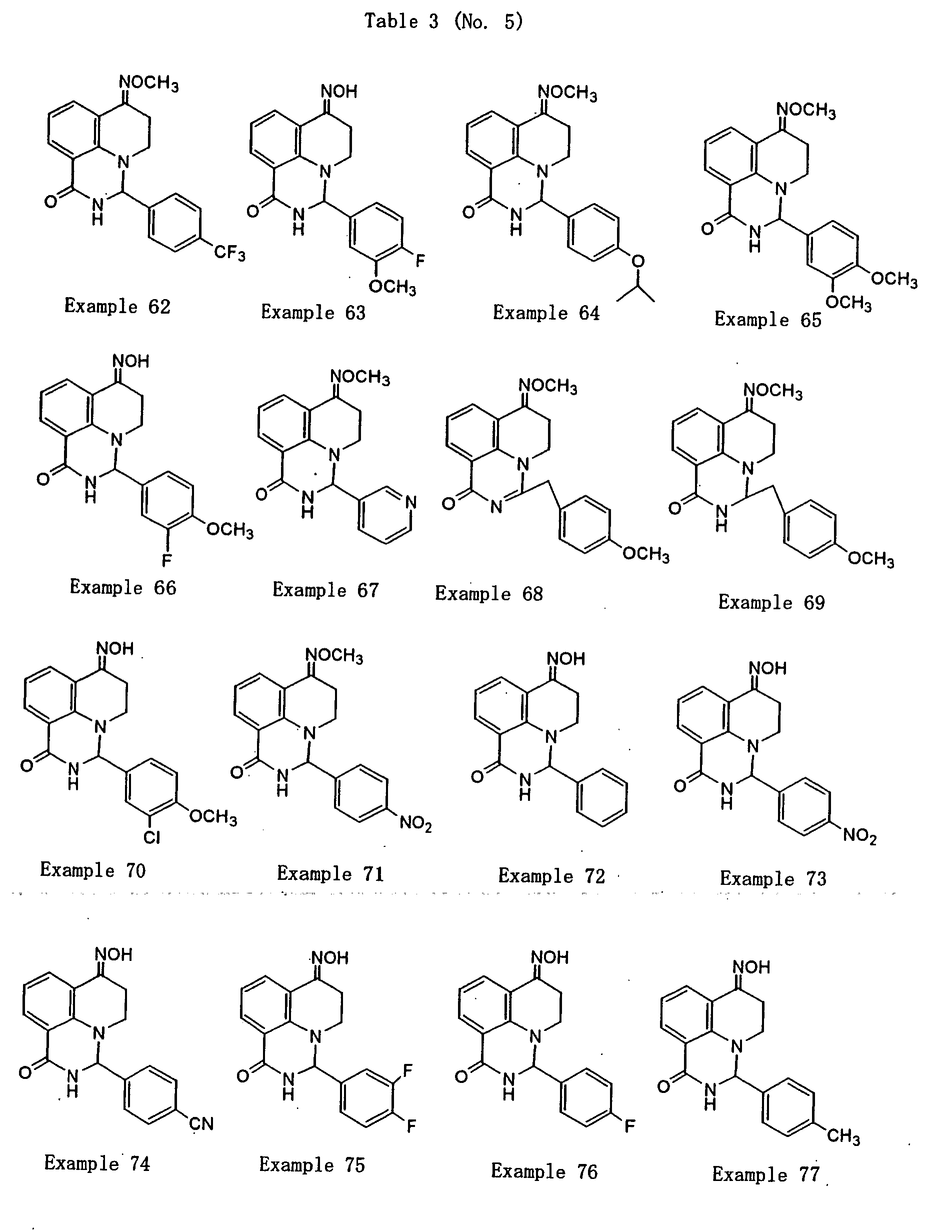

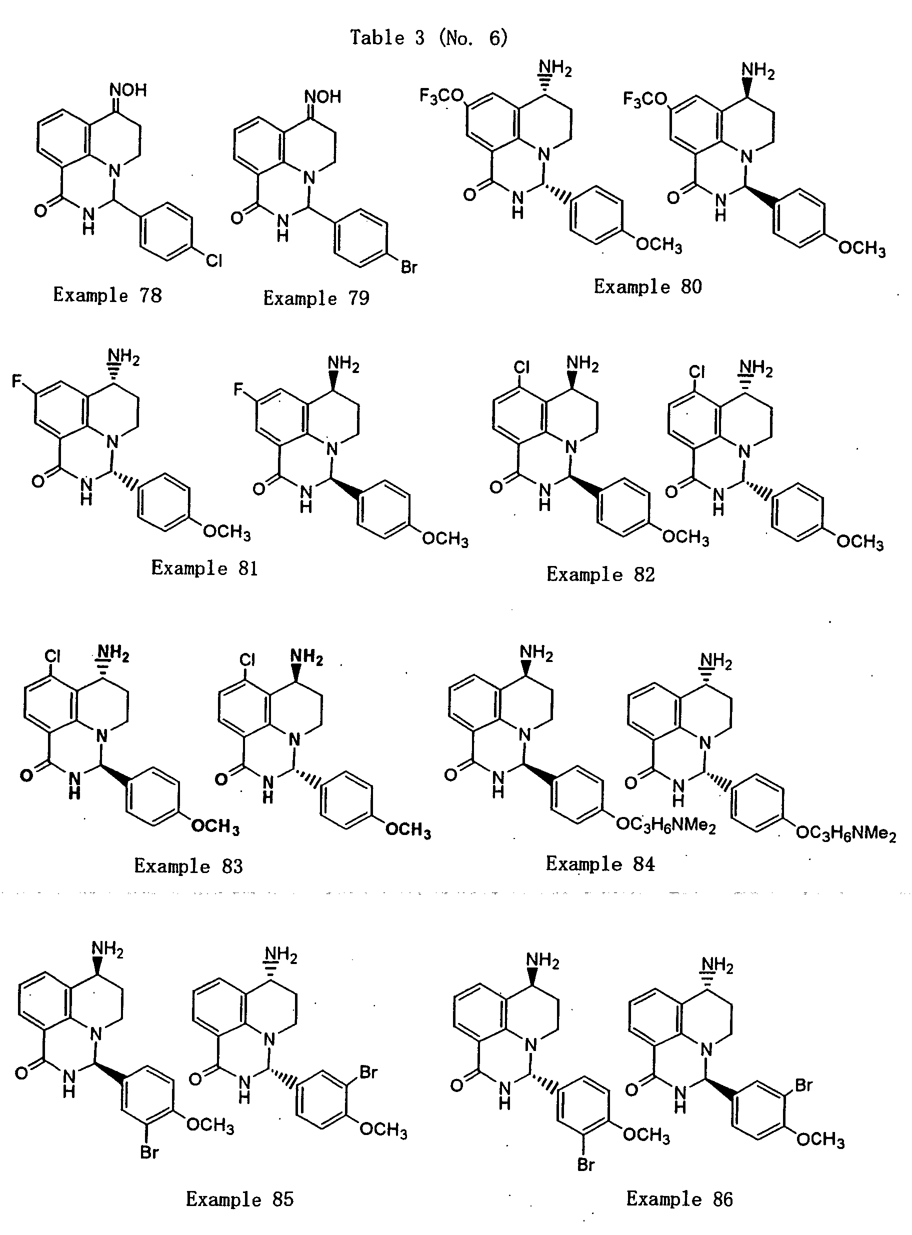

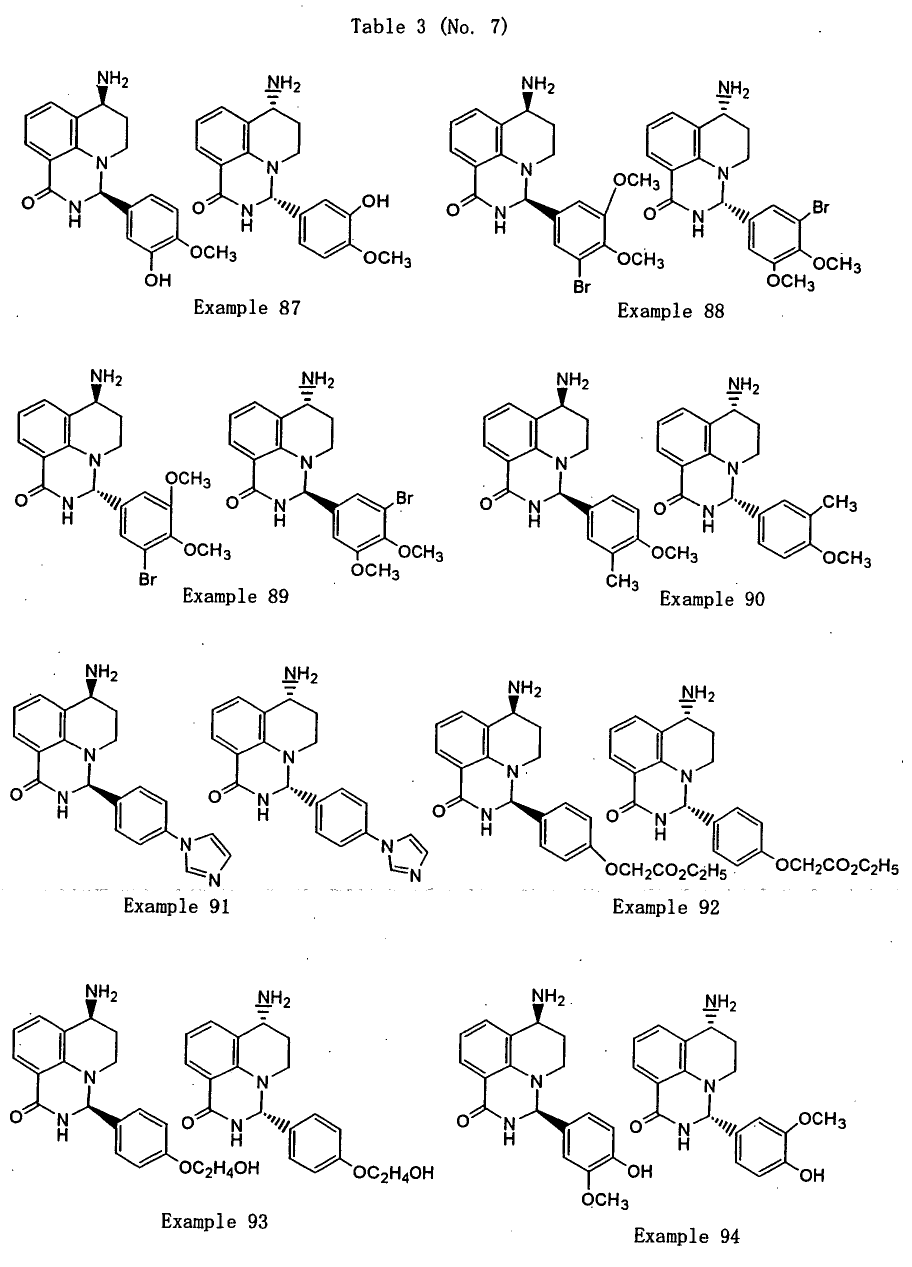

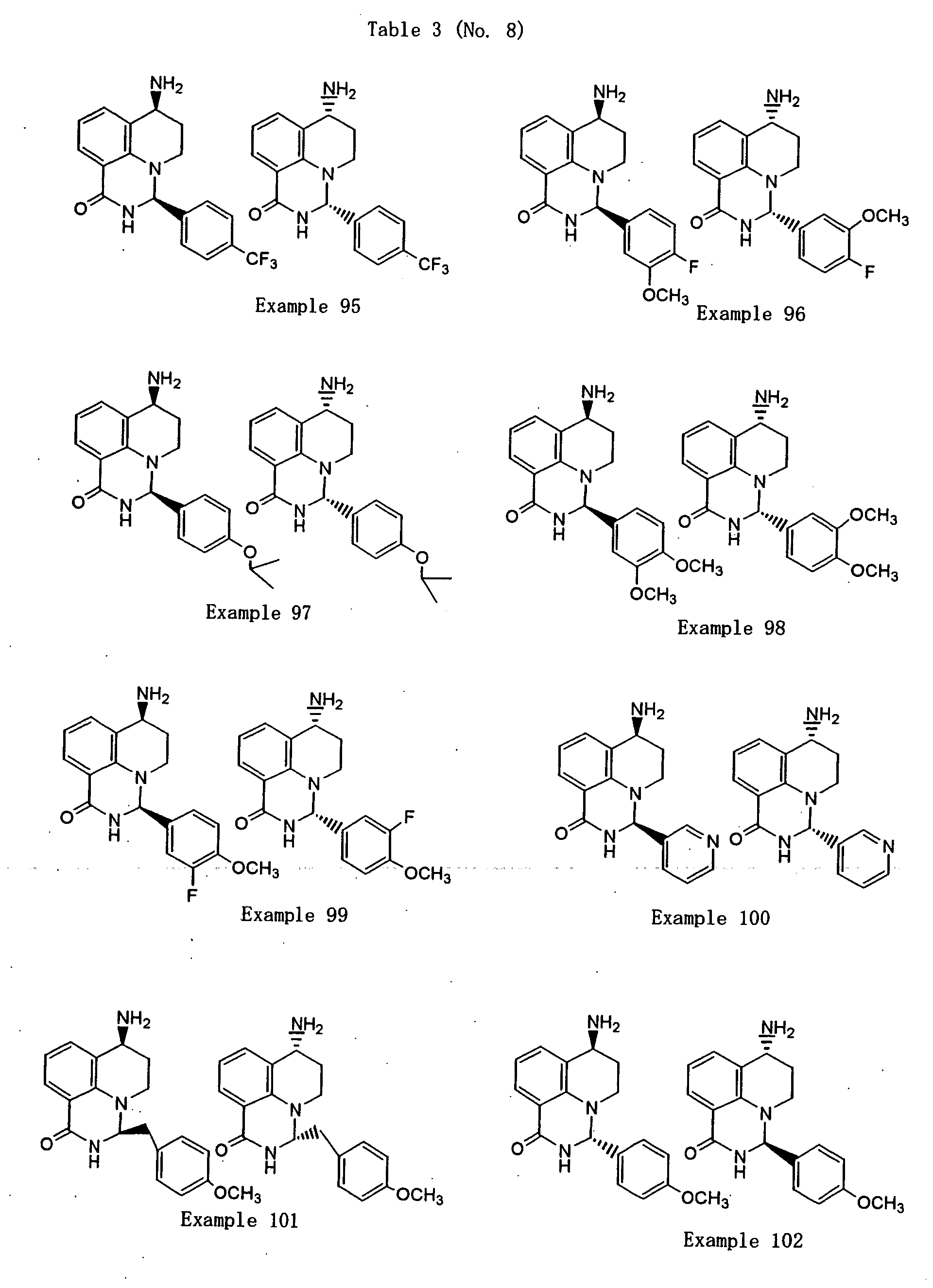

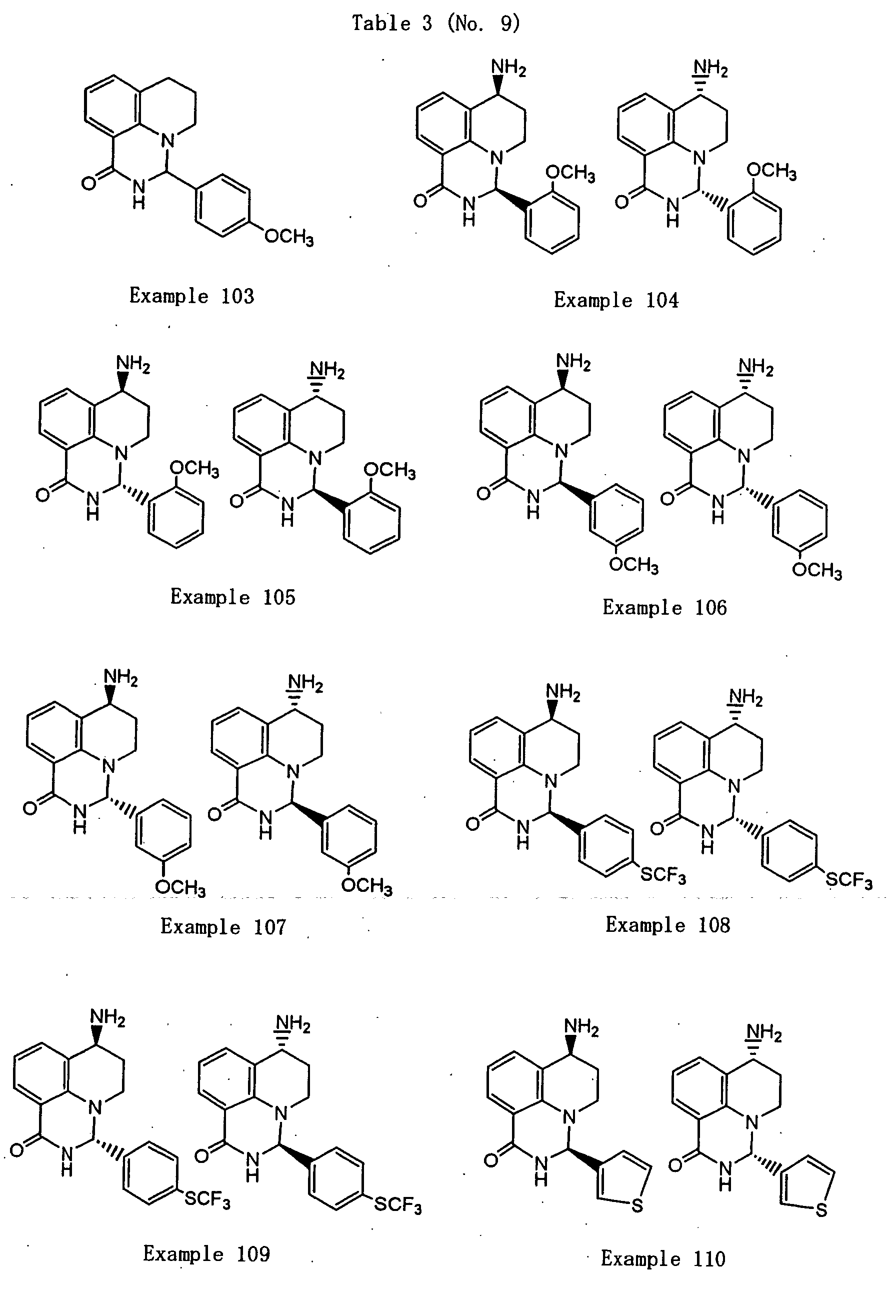

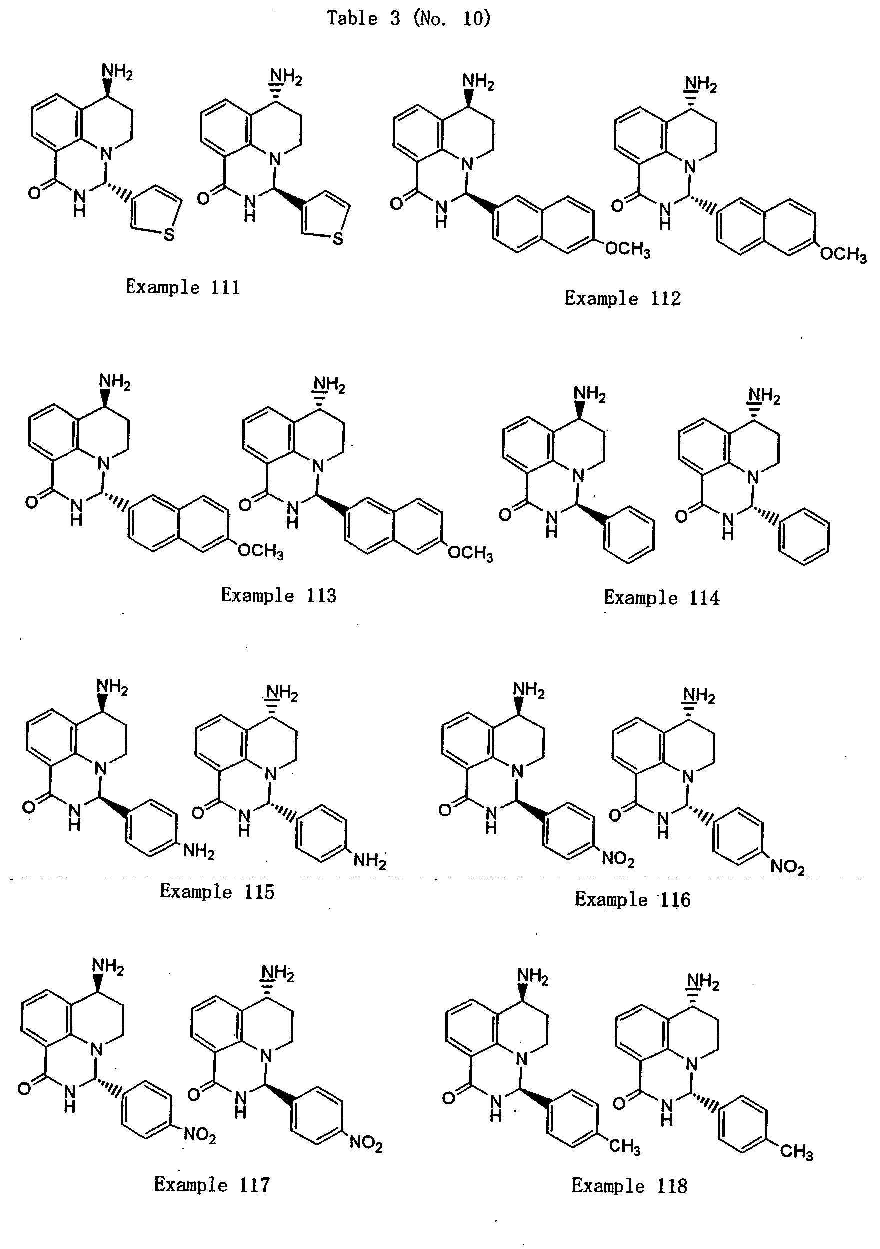

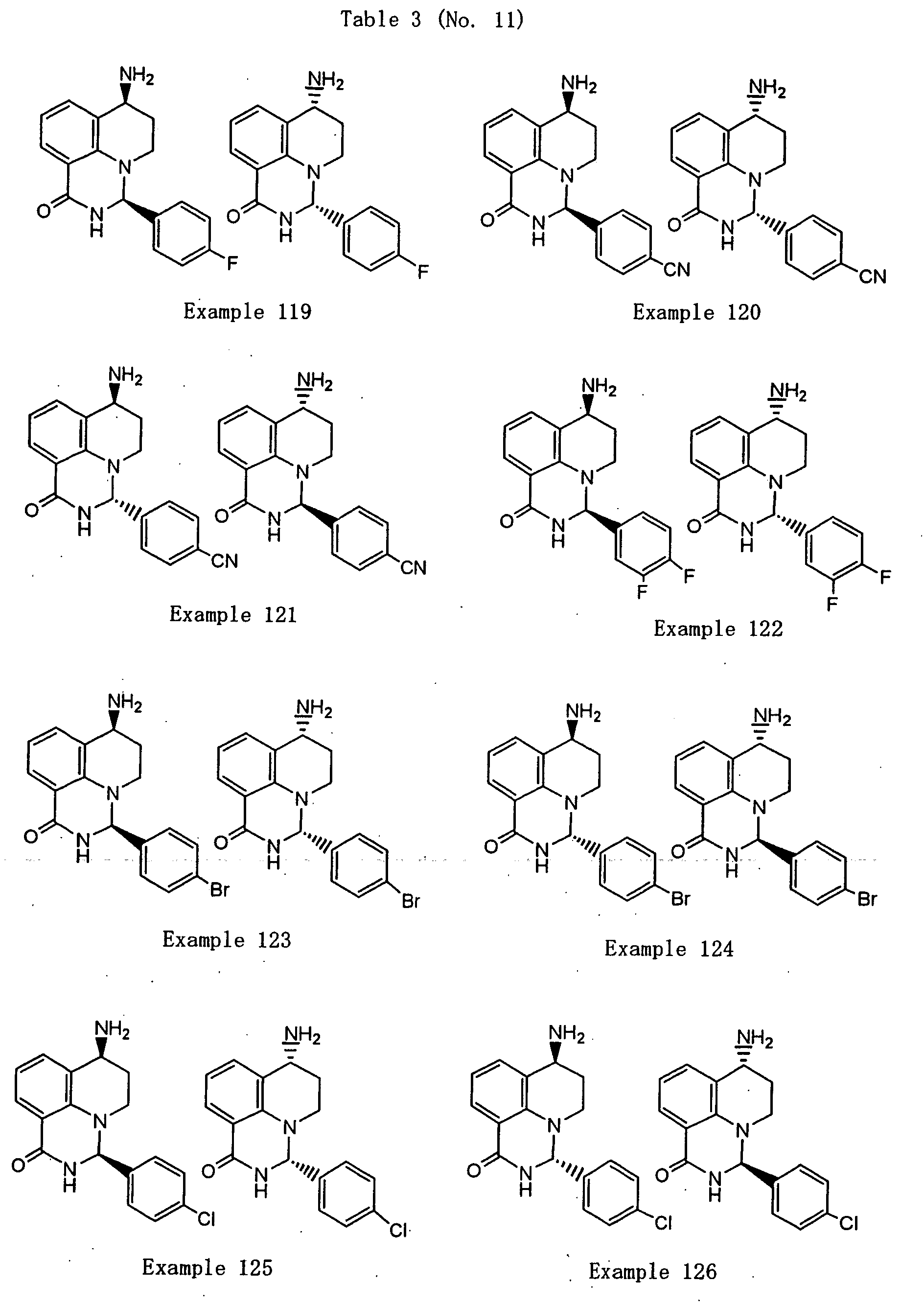

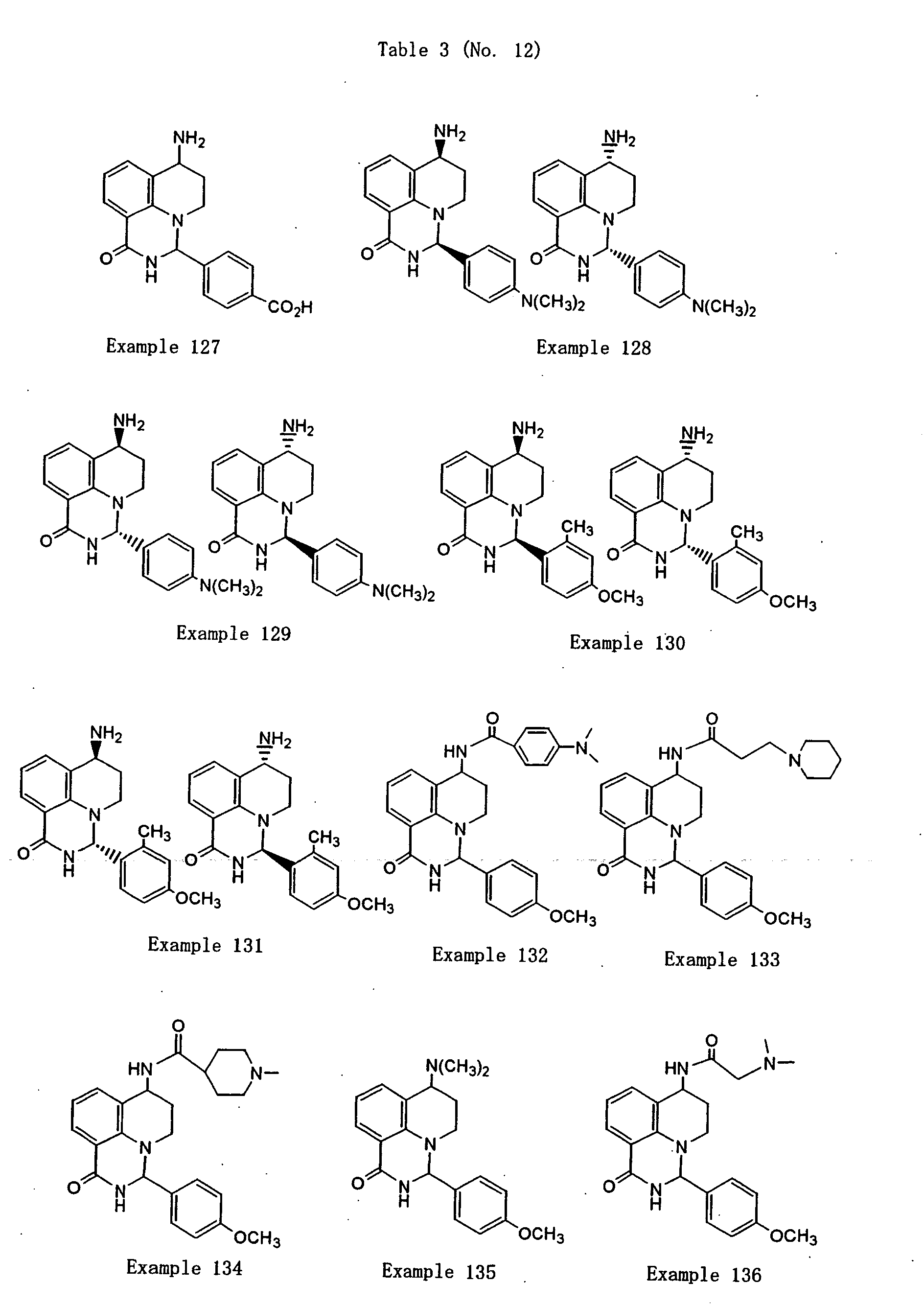

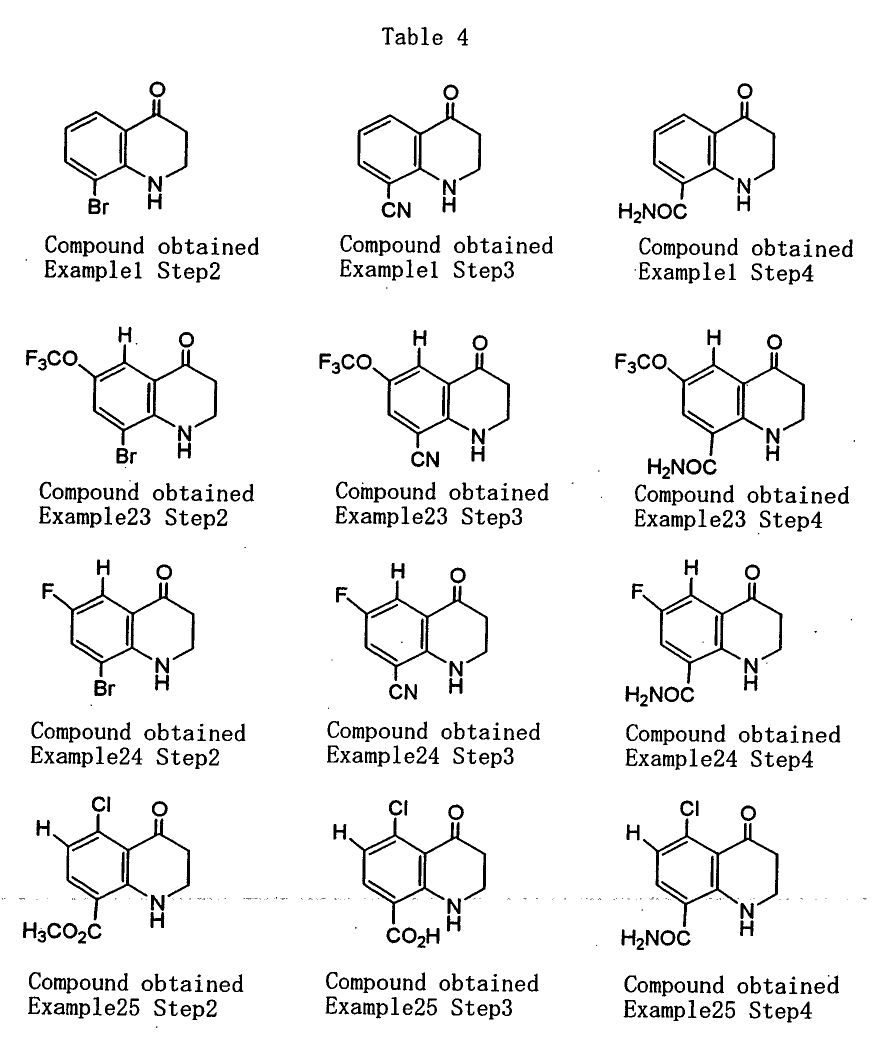

**Table 5**

| Example No. | m.p. °C | IR(cm⁻¹) | NMR (ppm: No mark:300MHz, * :270MHz) |
|---|---|---|---|
| 1 | 137.4-144.2 | 1676, 1456, 1248, 766 | CDCl₃: 8.18(1H, dd, J=2, 8Hz), 8.09(1H, dd, J=2, 8Hz), 7.48(2H, d, J=9Hz), 6.99(1H, t, J=8Hz), 6.98(2H, d, J=9Hz), 5.90(1H, brs), 5.50(1H, s), 3.86(3H, s), 3.1-3.0(2H, m), 2.7-2.6(2H, m) |
| 2 | 95.7-99.6 | 1655, 1512, 1250, 1032, 781 | CDCl₃: 7.89(1H, dd, J=2, 8Hz), 7.46(1H, dd, J=2, 7Hz), 7.33(2H, d, J=9Hz), 6.88(2H, d, J=9Hz), 6.83(1H, t, J=8Hz), 6.00(1H, brs), 5.52(1H, d, J=2Hz), 4.77(1H, dd, J=6, 11Hz), 3.81(3H, s), 3.0-2.8(2H, m), 2.1-1.9(2H, m), 1.81(1H, d, J=6Hz) |
| 3 | 200.8-211.9 | 1664, 1450, 1254, 756 | DMSO-d₆: 11.14(1H, s), 8.48(1H, d, J=2Hz), 7.88(1H, dd, J=2, 8Hz), 7.74(1H, dd, J=2, 8Hz), 7.33(2H, d, J=9Hz), 6.95(2H, d, J=9Hz), 6.83(1H, t, J=8Hz), 5.52(1H, d, J=2Hz), 3.75(3H, s), 3.0-2.9(1H, m), 2.7-2.5(3H, m) |
| 4 | 139.7-145.9 | 1672, 1606, 1485, 1252, 766 | CDCl₃: 7.87(1H, d, J=8Hz), 7.47(1H, d, J=8Hz), 7.38(2H, d, J=8Hz), 6.91 (2H, d, J=8Hz), 6.85(1H, t, J=8Hz), 5.93(1H, brs), 5.48(1H, d, J=1Hz), 3.97(1H, dd, J=5, 8Hz), 3.82(3H, s), 2.9-2.8(2H, m), 2.1-2.0(1H, m), 1.9-1.7(1H, m), 1.6(2H, brs) |
| 5 | 189.7-195.3 | 1660, 1498, 1250, 758 | CD₃OD: 7.86(1H, dd, J=1, 8Hz), 7.47(1H, d, J=8Hz), 7.42(2H, d, J=9Hz), 6.97(2H, d, J=9Hz), 6.90(1H, t, J=8Hz), 5.60(1 H, s), 4.52(1 H, t, J=7Hz), 3.81 (3H, s), 3.1-3.0(2H, m), 2.9-2.8(2H, m), 2.3-2.2(1 H, m) |
| 6 | 110.0-112.7 | 1658,1510, 1250, 1176, 760 | CDCl₃: 7.87(1H, d, J=8Hz), 7.47(1H, d, J=8Hz), 7.38(2H, d, J=9Hz), 6.91 (2H, d, J=9Hz), 6.85(1H, t, J=8Hz), 5.86(1 H, brs), 5.48(1 H, d, J=1 Hz), 3.97(1 H, dd, J=5, 8Hz), 3.82(3H, s), 2.9-2.8(2H, m), 2.1-2.0(1 H, m), 1.9-1.7(1H, m), 1.6(2H, brs) |
| 7 | 110.0-112.7 | 1658, 1510, 1250, 1176, 760 | CDCl₃: 7.87(1H, d, J=8Hz), 7.47(1H, d, J=8Hz), 7.38(2H, d, J=9Hz), 6.91 (2H, d, J=9Hz), 6.85(1H, t, J=8Hz), 5.86(1 H, brs), 5.48(1 H, d, J=1Hz), 3.97(1 H, dd, J=5, 8Hz), 3.82(3H, s), 2.9-2.8(2H, m), 2.1-2.0(1 H, m), 1.9-1.7(1 H, m), 1.6(2H, brs) |
| 8 | 216-218 | 1606, 1495 1456, 1250, 1026, 837, 760 760 | CD₃OD: 7.86(1H, dd, J=1, 8Hz), 7.46(1 H, d, J=6Hz), 7.42(2H, d, J=9Hz), 6.97(2H, d, J=9Hz), 6.89(1H, t, J=8Hz), 5.60(1 H, s), 4.51 (1 H, t, J=6Hz), 3.80(3H, s), 3.1-3.0(1H, m), 2.9-2.8(1H, m), 2.3-2.2(1H, m), 2.1-1.9(1H, m) |
| 9 | 200-202 | 1655, 1604, 1498, 1250, 837, 758 | CD₃OD: 7.86(1 H, dd, J=1, 8Hz), 7.48(1 H. d, J=6Hz), 7.42(2H, d, J=9Hz), 6.99(2H, d, J=9Hz), 6.89(1H, t, J=8Hz), 5.61(1H, s), 4.53(1 H, t, J=6Hz), 3.81 (3H, s), 3.1-3.0(1H, m), 2.9-2.8(1H, m), 2.3-2.2(1 H, m), 2.1-2.0(1H, m) |
| 10 | 275.4-277.1 | 1685, 1531, 1271,769 | CDCl₃: 8.18(1H, dd, J=2, 8Hz), 8.11(1H, dd, J=2, 8Hz), 8.04(1H, d, J=2Hz), 7.80(1 H, dd, J=2, 9Hz), 7.22(1H, d, J=9Hz), 7.03(1H, dd, J=8, 8Hz), 6.02(1H, brs), 5.60(1H, s), 4.03(3H, s), 3.2-3.0(2H, m), 2.8-2.6(2H, m) |
| 11 | 223.0-226.1 | 1674, 1527, 1275, 1012, 769 | CDCl₃*: 7.91 (1H, d, J=2Hz), 7.86(1 H, dd, J=2, 8Hz), 7.68(1H, dd, J=3, 9Hz), 7.5-7.4(1 H,m), 7.11(1H, d, J=9Hz), 6.86(1 H, t, J=8Hz), 6.07(1 H, brs), 5.58(1 H, d, J=2Hz), 4.1-4.0(1H, m), 3.98(3H, s), 3.1-2.9(2H, m), 2.2-2.0(1 H, m), 1.9-1.8(1 H, m) |
| 12 | 240.1-242.8 | 1674, 1529, 1487, 1271, 1014, 771 | CDCl₃*: 8.00(1 H, d, J=2Hz), 7.90(1 H, dd, J=2, 8Hz), 7.76(1H, dd, J=2, 9Hz). 7.41(1H, d, J=6Hz), 7.17(1H, d, J=9Hz), 6.90(1H, t, J=8Hz), 5.75(1H, d, J=2Hz), 5.59(1H, s), 4.1-4.0(1H, m), 4.01(3H, s), 3.1-2.7(2H, m), 2.1-1.9(1 H, m), 1.9-1.7(1 H, m) |
| 13 | 73.2-75.6 | 1664, 1604, 1510, 1250, 1032, 756 | CDCl₃*: 7.85(1H, dd, J=1, 8Hz), 7.4-7.3(3H, m), 7.15(2H, d, J=9Hz), 6.88(2H, d, J=9Hz), 6.77(1 H, t, J=7Hz), 6.69(2H, d, J=9Hz), 5.85(1H, brs), 5.51 (1 H, d, J=2Hz), 3.8-3.6(3H, m), 3.81(3H, s), 3.2=3.0(1H, m), 3.0-2.8(1 H, m), 2.93(6H, s), 2.1-1.9(2H, m) |
| 14 | 279.9 dec. | 1687, 1657, 1628, 1248' 1628, 1248, | DMSO-d₆*: 8.13(1 H, d, J=8Hz), 7.87(1 H, d, J=8Hz), 7.30(2H, d, J=9Hz), 7.17(1H, t, J=8Hz), 6.83(2H, d, J=9Hz), 6.62(1H, brs), 6.40(1H, brs), 5.66(1H, d, J=3Hz), 3.77(3H, s), 3.6-3.4(4H, m) |
| 15 | 172.1-180.4 | 1672, 1481, 1286, 762 | DMSO-d₆: 8.75 (1H, d, J=2Hz), 7.96 (1H, dd, J=2, 8Hz), 7.84 (1H, dd, J=2, 8Hz), 7.39( 5H, s), 6.89 (1 H, t , J=8Hz), 5.77 (1H, d, J=2Hz), 3.4-3.3 (1H, m), 3.0-2.9 (1H, m), 2.7-2.5 (2H, m) |
| 16 | 134.0-146.9 | 2972, 2229, 1678, 1479, 1319, 768 | DMSO-d₆*: 8.84 (1H, d, J=2Hz), 8.0-7.8 (4H, m), 7.60 (2H, d, J=8Hz), 6.90 (1H, t, J=8Hz), 5.92(1H, d, J=2Hz), 3.5-3.3 (1H, m), 3.0-2.9(1H, m), 2.8-2.5 (2H, m) |
| 17 | 198.8-209.3 | 1682, 1523, 1348, 766 | DMSO-d₆*: 8.89 (1H, d, J=3Hz), 8.29 (2H, d, J=9Hz), 7.98(1H, dd, J=2, 8Hz), 7.86(1H, dd, J=2, 8Hz), 7.70(2H, d, J=9Hz), 6.91 (1H, t, J=8Hz), 6.00(1H, d, J=3Hz), 3.5-3.4 (1H, m), 3.1-3.0 (1H, m), 2.8-2.5 (2H, m) |
| 18 | 172.1-175.8 | 1689, 1477, 1321, 764 1321, 764 | DMSO-d₆:8.70 (1H, d, J=2Hz), 7.96(1H, dd, J=2, 8Hz), 7.83(1H, dd, J=2, 8Hz), 7.30(2H, d, J=8Hz), 7.22(2H, d, J= 8Hz), 6.88(1H, t, J=8Hz), 5.72(1H, d, J=2Hz), 3.4-3.3(1H, m), 3.0-2.9(1H, m), 2.8-2.5 (2H, m), 2.3(3H, s) |
| 19 | 216.0-224.8 | 3072, 1678, 1481, 1225, 762 | DMSO-d₆*: 8.74(1H, d, J=2Hz); 7.97(1H, dd, J=2, 8Hz), 7.85(1H, dd, J=2, 8Hz), 7.5-7.4(2H, m), 7.3-7.2 (2H, m), 6.90(1H, t, J=8Hz), 5.80(1H, d, J=2Hz), 3.4-3.3(1 H, m), 3.0-2.9(1 H, m), 2.8-2.6(2H, m) |
| 20 | 184.0-189.6 | 1678, 1479, 1284, 768 | DMSO-d₆: 8.76(1H, d, J=2Hz), 7.97(1H, dd, J=2, 8Hz), 7.86(1H, dd; J=2, 8Hz), 7.6-7.3(3H, m), 6.91 (1H, t, J=8Hz), 5.81 (1H, d, J=2Hz), 3.4-3.3(1H, m), 3.0-2.9(1H, m), 2.7-2.6(2H, m) |
| 21 | 206.2-214.0 | 1678, 1475, 1011, 764 | DMSO-d₆*: 8.75(1H, d, J=2Hz), 7.96(1H, dd, J=2, 8Hz), 7.84(1H, dd, J=2, 8Hz), 7.63(2H, d, J=8Hz), 7.38(2H, d, J=8Hz), 6.90 (1 H, t, J=8Hz), 5.81 (1 H, d, J=2Hz), 3.0-2.9(1 H, m), 2.8-2.5(3H, m) |
| 22 | 154.3-158.9 | 1670, 1479, 1279, 764 | DMSO-d₆: 12.99(1 H, brs), 8.80 (1H, s), 8.0-7.9(3H, m), 7.85(1H, dd, J=2, 8Hz), 7.54 (2H, d, J=8Hz), 6.90(1H, t, J=8Hz), 5.88(1H, d, J=2Hz), 3.1-2.9(1H, m), 2.8-2.5(3H, m) |
| 23 | - | - | DMSO-d₆*: 8.91 (1H, d, J=2Hz), 7.83(1H, d, J=2Hz), 7.69(1 H, d, J=2Hz), 7.36(2H, d, J=9Hz), 6.99(2H, d, J=9Hz), 5.79(1H, d, J=2Hz), 3.76(3H, s), 3.5-3.3(1H, m), 3.0-2.9(1H, m), 2.8-2.5(2H, m) |
| 24 | 164.7-166.0 | 1678, 1454, 1252, 1184, 1028 | DMSO-d₆*: 8.83(1H, d, J=2Hz), 7.73(1H, dd, J=3, 8Hz), 7.55(1H, dd, J=3, 9Hz), 7.34(2H, d, J=9Hz), 6.96(2H, d, J=9Hz), 5.68(1H, d, J=2Hz), 3.75(3H, s), 3.4-3.2(1H, m), 2.9-2.8(1H, m), 2.7-2.6(2H, m) |
| 25 | 209.4-213.1 | 1680, 1591, 1454, 1248, 843 | DMSO-d₆: 8.85(1H, d, J=3Hz), 7.85(1H, d, J=8Hz), 7.29(2H, d, J=9Hz), 6.96(2H, d, J=9Hz), 6.85(1H, d, J=8Hz), 5.77(1H, d, J=3Hz), 3.74(3H, s), 3.6-3.3(1H, m), 3.1-2.9(1H, m), 2.8-2.7(1H, m), 2.6-2.5(1H, m) |
| 26 | 174.3-178.2 | 1682, 1473, 1255, 839, 764 | DMSO-d₆*: 8.65(1H, s), 7.96(1H, dd, J=2, 7Hz), 7-83(1H, dd, J=2, 8Hz), 7.32(2H, d, J=9Hz), 6.95(2H, d, J=9Hz), 6.88(1H, t, J=8Hz), 5.69(1H, s), 3.98(1H, t, J=6Hz), 3.4-3.2(1 H, m), 3.0-2.8(1H, m), 2.7-2.5(2H, m), 2.33(2H, t, J=7Hz), 2.12(6H, s), 1.9-1.7(2H, m) |
| 27 | 212.8-215.7 | 1678, 1456, 1286, 1263, 1053,766 | DMSO-d₆*: 8.70(1H, d, J=2Hz), 7.97(1H, dd, J=2, 8Hz), 7.85(1H, dd, J=2, 8Hz), 7.64(1H, d, J=2Hz), 7.39(1H, dd, J=2, 9Hz), 7.16(1H, d, J=9Hz), 6.90(1H, t, J=8Hz), 5.73(1H, d, J=2Hz), 3.85(3H, s), 3.5-3.2(1H, m), 3.0-2.9(1H, m), 2.8-2.5(2H, m) |
| 28 | 204.7-212.2 | 3200, 1666, 1244, 1134, 762 | DMSO-d₆*: 9.19(1H, s), 8.64(1H, s), 7.95(1H, d, J=7Hz), d, J=2Hz), d, J=8Hz), 7.0-6.8(4H, m), 5.59(1H, d, J=2Hz), 3.75(3H, s), 3.5-3.3(1H, m), 3.0-2.9(1 H, m), 2.8-2.4(2H, m) |
| 29 | - | - | CDCl₃*: 8.2- 8.1 (2H, m), 7.3-7.2(1H, m), 7.1-7.0(2H, m), 5.80(1H. brs), 5.46(1H, s), 3.91(3H, s), 3.90(3H, s), 3.1-3.0(2H, m), 2.7-2.6(2H, m) |
| 30 | - | - | CDCl₃*: 8.18(1H, dd, J=2, 8Hz), 8.09(1H, dd, J=2, 8Hz), 7.4-7.3(2H, m), 6.98(1H, t, J=8Hz), 6.88(1H, d, J=8Hz), 5.96(1H, brs), 5.46(1H, s), 3.88(3H, s), 3.1-3.0(2H, m), 2.8-2.6(2H, m), 2.25(3H, s) |
| 31 | - | - | CDCl₃*: 8.21 (1H, dd, J=2, 8Hz), 8.12(1H, dd, J=2, 8Hz), 7.91 (1H, s), 7.72(2H, d, J=9Hz), 7.53(2H, d. J=9Hz), 7.4-7.2(2H, m), 7.03(1H, t, J=8Hz), 5.97(1H, brs), 5.64(1H, s), 3.2-3.1 (2H, m), 2.7-2.6(2H, m) |
| 32 | - | - | CDCl₃*: 8.18(1H, dd, J=2, 7Hz), 8.09(1H, dd, J=2, 8Hz), 7.49(2H, d, J=9Hz), 7.1-6.9(3H, m), 6.00(1H; brs), 5.52(1H, s), 4.67(2H, s), 4.30(2H, q, J=7Hz), 3.1-3.0(2H, m), 2.7-2.5(2H, m), 1.32(3H, t, J=7Hz) |
| 33 | - | - | DMSO-d₆*: 8.19(1H, dd, J=2, 8Hz), 8.09(1H, dd, J=2, 8Hz), 7.49(2H, d, J=8Hz), 7.0-6.9(3H, m), 5.83(1H, brs), 5.51 (1H, s), 4.2-3.9(4H, m), 3.1-2.9(2H, m), 2.7-2.5(2H, m) |
| 34 | - | - | DMSO-d₆: 8.60(1H,d, J=2Hz),), 7.96(1H, dd, J=2, 8Hz), 7.84(1H, dd, J=2, 8Hz), 7.1-6.8(4H, m), 5.61 (1H, d, J=2Hz), 3.73(3H, s), 3.4-3.3(1H, m), 3.2-2.9(1 H, m), 2.7-2.5(2H, m) |
| 35 | 203.1-208.0 | 1678, 1325, 1113, 1068, 775 | CDCl₃*: 8.20(1H, dd, J=1, 8Hz), 8.12(1H, dd, J=1, 8Hz), 7.77(2H, d, J=8Hz), 7.72(2H, d, J=8Hz), 7.03(1H, t, J=8Hz), 5.96(1H, brs), 5.66(1H, s), 3.2-3.0(2H, m), 2.8-2.6(2H, m) |
| 36 | 171.0-172.9 | 1684,1475, 1288, 1024, 766 | CDCl₃*: 8.20(1H, dd, J=1, 8Hz), 8.12(1H, dd, J=2, 8Hz), 7.2-7.0(4H, m), 5.85(1H, brs), 5.51 (1H, s), 3.93(3H, s), 3.1-3.0(2H, m), 2.7-2.6(2H, m) |
| 37 | 175.8-177.1 | 1676,1481, 1246, 764 | COCl₃*: 8.19(1H, dd, J=2, 8Hz), 8.09(1H, dd, J=2, 8Hz), 7.45(2H, d, J=8Hz), 7.0-6.9(3H, m), 5.86(1H, brs), 5.49(1H, s), 4.7-4.5(1H, m), 3.1-2.9(2H, m), 2.8-2.6(2H, m), 1.37(6H, d, J=6Hz) |
| 38 | - | - | COCl₃*: 8.20(1H, dd, J=2, 8Hz), 8.11 (1 H, dd, J=2, 8Hz), 7.15(1H, d, J=2Hz), 7.04(1H, dd, J2, =8Hz), 7.00(1H, t, J=8Hz), 6.90(1H, d, J=8Hz), 5.99(1H, brs), 5.49(1H, s), 3.93(3H, s), 3.92(3H, s), 3.1-3.0(2H, m), 2.8-2.6(2H, m) |
| 39 | - | - | CDCl₃*: 8.18(1H, dd, J=2, 8Hz), 8.10(1 H, dd, J=2, 8Hz), 7.4-7.2(2H, m), 7.1-6.9(2H, m), 5.87(1H, brs), 5.49(1 H, s), 3.94(3H, s), 3.1-3.0(2H, m), 2.8-2.6(2H, m) |
| 40 | 190.0-191.6 | 1678, 1479, 1321, 764 | CDCl₃: 8.8-8.7(2H, m), 8.19(1 H, d, J=8Hz), 8.11 (1 H, dd, J=2, 8Hz), 7.97(1H, d, J=8Hz), 7.45(1H, dd, J=5, 8Hz), 7.03(1H, t, J=8Hz), 6.12(1H, brs), 5.65(1H, s), 3.2-3.0(2H, m), 2.8-2.6(2H, m) |
| 41 | 81.4-82.0 81.4-82.0 | 1703, 1645, 1703, 1645, 1601, 1510, 1248, 773 | CDCl₃*: 8.56(1H, dd, J=2, 8Hz), 8.31 (1H, dd, J=2, 8Hz), 7.54(1H, t, J=8Hz), 7.25(2H, d, J=9Hz), 6.88(2H, d, J=9Hz), 4.35(2H, t, J=7Hz), 4.25(2H, s), 3.79(3H; s), 2.81 (2H, t, J=7Hz) |
| 42 | 174.5-176.9 | 1672 1479, 1263, 1063, 766 | CDCl₃*: 8.18(1H, dd, J=2, 8Hz), 8.10(1H, dd, J=2, 8Hz), 7.60(1H, d, J=2Hz), 7.42(1H, dd, J=2, 8Hz), 7.1-6.9(2H, m), 5.94(1H, brs), 5.49(1H, s), 3.96(3H, s), 3.14-3.02(2H, m), 2.8-2.6(2H, m) |
| 43 | 214.7-219.5 | 1668, 1508, 1319, 1117, 760 | CDCl₃: 8.12(1H, dd, J=2, 8Hz), 8.05(1 H, dd, J=2, 8Hz), 7.43(1H, dd, J=2, 8Hz), 7.4-7.3(1H, m), 7.0-6.9(2H, m), 6.90(1H, t, J=8Hz), 6.15(1H, d, J=2Hz), 6.11(1H, s), 3.85(3H, s), 3.4-3.2(2H, m), 2.8-2.6(2H, m) |
| 44 | 126.5-142.5 | 1674, 1477 1286, 1265, 1049,760 | CDCl₃: 8.18(1, dd, J=2, 8Hz), 8.09(1H, dd, J=2, 8Hz), 7.38(1H, t, J=8Hz), 7.2-7.1(2H, m), 7.01 (1H, dd, J=1, 8Hz), 6.99(1H, t, J=8Hz), 5.98(1H, brs), 5.52(1H, s), 3.84(3H, s), 3.1-3.0(2H, m), 2.8-2.6(2H, m) |
| 45 | 178.2-180.9 | 1678, 1481, 1321, 1117 | CDCl₃: 8.16(1H, dd, J=2, 8Hz), 8.10(1H, dd, J=2, 8Hz), 7.77(2H, d, J=8Hz), 7.62(2H, d, J=8Hz), 7.00(1H, t, J= 8Hz), 6.23(1H, br.s), 5.62(1H, s), 3.2-3.0(2H, m), 2.8-2.6(2H, m) |
| 46 | 190.9-193.4 | 1691, 1670, 1481, 1321, 779, 453 | CDCl₃ : 8.16(1 H, dd, J=2, 8Hz), 8.07(1 H, dd, J=2, 8Hz), 7.5-7.4(2H, m), 7.24(1H, dd, J=1, 5Hz), 6.97(1H, t, J=8Hz), 6.28(1H, brs), 5.70(1H, s), 3.15(2H, t, J=7Hz), 2.70(2H, t, J=7Hz) |
| 47 | 191.4-195.5 | 1672, 1477, 1271, 1176, 1028, 794 1028,794 | CDCl₃: 8.20(1, dd, J=2, 8Hz), 8.10(1H, dd, J=2, 8Hz), 7.9-7.8(2H, m), 7.77(1H, d, J=9Hz), 7.69(1H, dd,J=2, 9Hz), 7.3-7.2(2H, m), 6.99(1H, t, J=8Hz), 6.01(1H, brs), 5.68(1H, s), 3.95(3H, s), 3.2-3.1(2H, m), 2.8-2.5(2H, m) |
| 48 | 198.7-202.6 | 1678, 1610, 1452, 132.1, 818, 777 | CDCl₃ : 8.19(1H, dd, J=2, 8Hz), 8.08(1H, dd, J=2, 8Hz), 7.38(2H, d, J=9Hz), 6.97(1H, t, J=8Hz), 6.74(2H, d, J=9Hz), 5.79(1H, brs), 5.43(1H, s), 3.2-2.9(2H,m), 3.01(6H, s), 2.8-2.5(2H, m) |
| 49 | 100.2-101.1 | 1676, 1479, 1454 1248 764 | CDCl₃ : 8.19(1H, dd, J=2, 8Hz), 8.10(1 H, dd, J=2, 8Hz), 7.50(1H, d, J=9Hz), 6.99(1H, t, J=8Hz), 6.9-6.8(2H, m), 5.76(1 H, s), 5.72(1 H, brs), 3.84(3H, s), 3.2-2.9(2H, m), 2.8-2.5(2H, m), 2.49(3H, s) |
| 50 | 232.7-234.3 | 1674, 1252 1211, 1171, 970, 833 | DMSO-d₆*: 11.42(1H,s), 8.73(1H,s), 7.73(1H, d, J=2Hz), 7.60(1H, d, J=2Hz), 7.33(2H, d, J=9Hz), 6.97(2H, d, J=8Hz), 5.62(1H, d, J=2Hz), 3.76(3H, s), 3.1-2.9(1 H, m), 2.9-2.5(3H, m) |
| 51 | 200.1-202.9 | 1674, 1252, 1138, 1051 | DMSO-d₆*: 8.69(1H, d, J=1Hz), 7.60(1H, dd, J=3, 10Hz), 7.51(1H, dd, J=3, 8Hz), 7.33(2H, d, J=9Hz), 6.96(2H, d, J=9Hz), 5.53(1H, d, J=1 Hz), 3.89(3H, s), 3.76(3H, s), 3.0-2.7(2H, m), 2.7-2.5(2H, m) |
| 52 | 132.9-133.4 | 1664, 1585, 1250, 839, 785 | DMSO-d₆*: 11.46(1H, s), 8.67(1H, d, J=3Hz), 7.63(1H, d, J=8Hz), 7.24(2H, d, J=9Hz), 6.92(2H, d, J=9Hz), 6.84(1H, d, J=9Hz), 5.62(1H, d, J=3Hz), 3.72(3H, s), 3.2-2.9(2H, m), 2.9-2.7(1H, m), 2.7-2.4(1H, m) |
| 53 | 164.3-167.0 | 1678, 1612, 1479, 1253, 1061, 760 | DMSO-d₆*: 8.51 (1H, d, J=2Hz), 7.87(1H, dd, J=2, 8Hz), 7.76(1H, dd, J=2, 8Hz), 7.30(2H, d, J=9Hz), 6.94(2H, d, J=9Hz), 6.83(1H, t, J=8Hz), 5.52(1H, d, J=2Hz), 3.98(2H, t, J=7Hz), 3.87(3H, s), 3.1-2.9(1H, m), 2.9-2.6(3H, m), 2.33(2H, t, J=7Hz), 2.12(6H, s), 1.9-1.8(2H, m) |
| 54 | 218.7-221.7 | 1672, 1618, 1441, 1263, 1043, 764 | DMSO-d₆*: 8.54(1H, s), 7.88(1H, dd, J=2, 8Hz), 7.76(1H, dd, J=2, 8Hz), 7.59(1H, d, J=2Hz), 7.36(1H, dd, J=2, 9Hz), 7.13(1H, d, J=9Hz), 6.84(1H, t, J=8Hz), 5.56(1H, m), 3.86(3H, s), 3.83(3H, s), 3.1-2.9(1H, m), 2.9-2.5(3H, m) |
| 55 | 185.3-189.8 | 3336, 1660, 1616, 1454, 1240, 1061, 758 | DMSO-d₆: 9.15(1H, brs), 8.47(1H, s), 7.85(1H, d, J=), 7.75(1H, dd, J=7Hz), 7.0-6.7(4H, m), 5.41 (1 H, s), 3.86(3H, s), 3.74(3H, s), 3.0-2.9(1H, m), 2.8-2.6(3H, m) |
| 56 | 133.9-146.8 | 1664, 1489, 1275, 1142, 1047, 760 | DMSO-d₆*: 11.18(1H, s), 8.51 (1H, s), 7.91 (1H, d, J=8Hz), 7.74(1H, d, J=7Hz), 7.19(1H, s), 7.14(1H, s), 6.86(1H, t, 8Hz), 5.56(1H, s), 3.79(3H, s), 3.74(3H, s), 3.1-2.9(1H, m), 2.9-2.5(3H, m) |
| 57 | 236.0-243.3 | 1660, 1448, 1255, 1132, 976, 754 | DMSO-d₆*: 11.13(1H, s), 8.42(1H, brs), 7.87(1H, dd, J=2, 8Hz), 7.74(1H, dd, J=2, 8Hz), 7.3-7.1 (2H, m), 6.95(1 H, d, J=9Hz), 6.83(1 H, t, J=8Hz), 5.46(1H, s), 3.78(3H, s), 3.0-2.5(4H, m), 2.13(3H, s) |
| 58 | 246.3dec. | 1682, 1468, 1055, 766 | DMSO-d₆*: 8.66(1H, d, J=2Hz), 8.38(1H, brs), 7.89(1H, dd, J=2, 8Hz), 7.8-7.7(2H, m), 7.71(2H, d, J=9Hz), 7.53(2H, d, J=9Hz), 7.18(1H, brs), 6.86(1H, t, J=8Hz), 5.70(1H, d, J=2Hz), 3.88(3H, s), 3.1-3.0(1H, m), 2.7-2.5(3H, m) |
| 59 | 198.6-203.3 | 1759, 1664, 1448, 1201, 976, 758 | DMSO-d₆*: 11.14(1H, s), 8.50(1H, brs), 7.87(1H, dd, J=2, 8Hz), 7.73(1H, dd, J=2, 8Hz), 7.32(2H, d, J=8Hz), 6.94(2H, d, J=9Hz), 6.83(1H, t, J=8Hz), 5.53(1H, d, J=2Hz), 4.78(2H, s), 4.16(2H, q, J=7Hz), 3.0-2.9(1H, m), 2.8-2.5(3H, m), 1.21 (3H, t, J=7Hz) |
| 60 | 183.9-186.3 | 3435, 1657, 1450,974, 758 | DMSO-d₆*: 11.14(1H, s), 8.49(1H, brs), 7.87(1H, d, J=8Hz), 7.73(1H, d, J=8Hz), 7.31 (2H, d, J=9Hz), 6.95(2H, d, J=9Hz), 6.82(1H, t, J=8Hz), 5.52(1H, s), 4.87(1H, brs), 4.1-3.8(2H, m), 3.8-3.5(2H, m), 3.1-2.9(1H, m), 2.8-2.5(3H, m) |
| 61 | 174.4-189.3 | 1657, 1450, 1277, 1032, 974, 762 | DMSO-d₆*: 9.19(1H, s), 8.40(1H, s), 7.89(1H, dd, J=2, 8Hz), 7.73(1H, dd, J=2, 8Hz), 6.99(1H, s), 6.9-6.7(3H, m), 5.43(1H, s), 3.73(3H, s), 3.0-2.7(3H, m), 2.7-2.5(1H, m) |
| 62 | 199.4-207.2 | 1684, 1327, 1117, 1051, | DMSO-d₆*: 8.67(1H, d, J=2Hz), 7.89(1H, dd, J=2,8Hz), 7.8-7.7(3H, m), 7.62(2H, d, J=8Hz), 6.86(1H, 845, 752 t, J=8Hz), 5.71(1H, d, d, J=2Hz), 3.88(3H, s), 3.2-3.0(1 H, m), 2.8-2.6(3H, m) |
| 63 | 245.4-246.6 | 1664 1448 1028, 976, 758 | DMSO-d₆*: 11.16(1H, brs), 8.51 (1H, d, J=1Hz), 7.91(1H, dd, J=2, 8Hz), 7.74(1H, dd, J=2, 8Hz), 7.3-7.2(2H, m), 7.0-6.9(1H, m), 6.85(1H, t, J=8Hz), 5.57(1H, d, J=2Hz), 3.82(3H, s), 3.0-2.5(4H, m) |
| 64 | 167.4-168.9 | 1689, 1446, 1234, 1041, 764 | DMSO-d₆*: 8.50(1H, d, J=2Hz), 7.8.7(1H, dd, J=2, 8Hz), 7.76(1H, dd, J=2, 8Hz), 7.29(2H, d, J=9Hz), 6.92(2H, d, J=9Hz), 6.83(1H, t, J=8Hz), 5.52(1H, d, J=2Hz), 4.7-4.5(1H, m), 3.87(3H, s), 3.1-2.9(1H, m), 2.8-2.5(3H, m), 1.25(6H, d, J=6Hz) |
| 65 | - | - | DMSO-d₆*: 8.47(1H, s), 7.89(1H, dd, J=2, 8Hz), 7.77(1H, dd, J=2, 8Hz), 7.0-6.8(4H, m), 5.50(1H, d, J=2Hz), 3.88(3H, s), 3.75(3H, s), 3.72(3H, s), 3.1-2.5(4H, m) |
| 66 | 260.3-261.6 | 1666, 1454, 1273, 1024, 982. 760 | DMSO-d₆*: 11.15(1H, brs), 8.51(1H, s), 7.88(1H, dd, J=2, 8Hz), 7.73(1H, dd, J=1, 8Hz), 7.3-7.1(3H, m), 6.83(1H, t, J=8Hz), 5.53(1H, s), 3.82(3H, s), 3,1-2.9(1H, m), 2.8-2.5(3H, m) |
| 67 | 178.6-185.9 | 1678, 1483, 1039, 764 | DMSO-d₆*: 8.65(1H, brs), 8.6-8.5(2H, m), 7.90(1H, dd, J=2, 8Hz), 7.8-7.7(2H, m), 7.5-7.3(1H, m), 6.87(1H, t, J=8Hz), 5.72(1H, s), 3.88(3H, s), 3.2-3.0(1 H, m), 2.9-2.6(3H, m) |
| 68 | 191.2-195.2 | 1647 1537 1431, 1269, 1045, 777 | DMSO-d₆*: 8.22(1H, dd, J=1, 8Hz), 8.12(1H, dd, J=1, 8Hz), 7.52(1H, t, J=8Hz), 7.25(2H, d, J=9Hz), 6.91(2H, d, J=9Hz), 4.3-4.1 (4H, m), 3.97(3H, s), 3.73(3H, s), 2.93(2H, t, J=7Hz) |
| 69 | 190.0-195.9 | 1668, 1246, 1045, 758 | CDCl₃*: 8.03(1H, d, J=8Hz), 7.93(1H, d, J=8Hz), 7.09(2H, d, J=8Hz), 6.9-6.8(3H, m), 5.71(1H, brs), 4.7-4.6(1 H, m), 3.99(3H, s), 3.80(3H, s), 3.6-3.2(2H, m), 3.2-2.9(2H, m), 2.9-2.6(2H, m) |
| 70 | 220.Odec. | 1655, 1448, 1065, 754 | DMSO-d₆: 11.16(1H, s), 8.53(1H, s), 7.89(1H, dd, J=2, 8Hz), 7.74(1H,dd, J=2, 8Hz), 7.46(1H, d, J=2Hz), 7.34(1H, dd, J=2, 9Hz), 7.18(1H, d, J=9Hz), 6.84(1H, t, 8Hz), 5.56(1H, d, J=2Hz), 3.85(3H, s), 3.0-2.6(4H, m) |
| 71 | 112.9-116.4 | 1672 1523 1672, 1523, 758 | DMSO-d₆: 8.76(1H, d, J=2Hz), 8.27(2H, d, J=9Hz), 7.90(1H, dd, J=2, 8Hz), 7.78(1H, dd, J=2, 8Hz), 7.67(2H, d, J=9Hz), 6.86(1H, t, J=8Hz),5.85(1H, d, J=2Hz), 3.88(3H, s), 3.373.1(1H, m), 2.8-2.6(3H, m) |
| 72 | - | - | DMSO-d₆*: 11.15 (1H, s), 8.58(1 H, s), 7.89 (1H, dd, J=2, 8Hz), 7.74(1 H, dd, J=2, 8Hz), 7.40 (5H, s), 6.83 (1H, t, J=8Hz), 5.60(1 H, d, J=2Hz), 3.2-3.0(1 H, m), 2.8-2.7(3H, m) |
| 73 | 220.0-224.7 | 1664, 1523, 1489, 1452, 1348, 972, 756 | DMSO-d₆: 11.21 (1 H, d, J=1 Hz), 8.73(1 H, d, J=2Hz), 8.3-8.2(2H, m), 7.9-7.6(4H, m), 6.86(1H, dd, J=8, 8Hz), 5.83(1H, d, J=2Hz), 3.2-3.1(1H, m), 2.8-2.7(3H, m) |
| 74 | > 250 | 1660, 1452, 1147, 972, 800, 754 | DMSO-d₆ : 11.2(1H, s), 8.7(1H, s), 7.9-7.7(4H, m), 7.6-7.5 (2H, m), 6.8(1H, t, J=8Hz), 5.76(1H, d, J=2Hz), 3.1 (1H, m), 2.7-2.6 (3H, m) |
| 75 | 218.4-222.3 | 1668, 1520, 1456, 1282, 980, 764 | DMSO-d₆: 11.16(1H, s), 8.58(1H, d, J=2Hz), 7.92 (1H, d, J=2Hz), 7.89(1H, d, J=2Hz), 7.7-7.5(2H, m), 7.3 7.2(1H, m), 6.85(1H, t, J=8Hz), 5.6(1H, d, J=2Hz), 3.0-2.9(1 H, m), 2.8-2.6 (3H, m) |
| 76 | 247.1-252.3 | 1664, 1452, 1325, 1228, 976, 756 | DMSO-d₆: 11.16(1H, s), 8.57(1H, s), 7.89(1H, d, J=8Hz), 7.74(1H, d, J=8Hz), 7.46(2H, dd, J=6, 9Hz), 7.24(2H, t, J=9Hz), 6.84(1H, t, J=8Hz), 5.63(1H, s), 3.1-3.0(1H, m), 2.7-2.5(3H, m) |
| 77 | 215.4-219.0 | 1664, 1485, 1325, 972, 829, 756 | DMSO-d₆*: 11.14(1H,s), 8.51(1H, s), 7.87(1H, dd, J=1, 8Hz), 7.73(1H, dd, J=1. 8Hz), 7.28(2H, d, J=8Hz), 7.20(2H, d, J=8Hz), 6.82(1H, t, J=8Hz), 5.54(1H, d, J=2Hz), 3.1-3.0(1 H, m), 2.8-2.6(3H, m), 2.30(3H, s) |
| 78 | 210.6-217.2 | 1664, 1489, 1304, 1092, 972, 839, 758 | DMSO-d₆*: 11.17(1H, s), 8.59(1H, s), 7.89(1H, d, J=6Hz), 7.73(1H, d, J=8Hz), 7.45(4H, dd, J=8, 17Hz), 6.84(1H, t, J=8Hz), 5.63((1H, d. J=2Hz), 3.0-2.9(1H, m), 2.8-2.6(3H, m) |
| 79 | 230.0-236.9 | 1637, 1487, 1325, 1012, 974, 758 | DMSO-d₆*: 11.18(1H, s), 8.59(1H, s), 7.89(1H, d, J=7Hz), 7.73(1H, d, J=7Hz), 7.62(2H, d, J=7Hz), 7.36(2H, d, J=8Hz), 6.84(1H, t, J=8Hz), 5.62 ((1H, s), 3.1-3.0(1 H, m), 2.7-2.6(3H, m) |
| 80 | 200.7-202.9 | 3334, 1689, 1259, 1220, 1173, 793 | DMSO-d₆*: 8.65(1H, s), 7.41(2H, s), 7.30(2H, d, J=8Hz), 6.93(2H, d, J=9Hz), 5.61 (1H, d, J=3Hz), 3.86(1 H, m), 3.74(3H, s), 3.2-2.9(2H, m), 2.1-1.8(3H, m), 1.8-1.5(1H, m) |
| 81 | 151.2-154.8 | 3332, 1674, 1254, 1178, 837 | DMSO-d₆*: 8.56(1H, d, J=2Hz), 7.4-7.2(4H, m), 6.92(2H, d, J=9Hz), 5.51 (1 H, d, J=3Hz), 3.9-3.8(1 H, m), 3.74(3H, s), 3.1-2.7(2H, m), 2.0-1.7(3H, m), 1.7-1.5(1H, m) |
| 82 | - | 1662, 1593, 1248, 1173, 787 | CDCl₃*: 7.80(1H, d, J=9Hz), 7.23(2H, d, J=7Hz), 6.84(2H, d, J=9Hz), 6.79(1H, d, J=8Hz), 6.16(1H, brs), 5.59(1H, d, J=3Hz), 4.28(1H, t, J=3Hz), 3.78(3H, s), 3.2-2.9(2H, m), 2.0-1.9(2H,m) |
| 83 | - | 1664, 1593, 1248, 847, 785 | CDCl₃*: 7.84(1H, d, J=8Hz), 7.46(2H, d, J=9Hz), 6.97(2H, d, J=9Hz), 6.89(1H, d, J=9Hz), 6.13(1H, brs), 5.54(1H, s), 4.29(1H, t, J=3Hz), 3.85(3H, s), 3.0-2.7(1H, m), 2.8-2.6(1H, m), 2.0-1.7(2H, m) |
| 84 | 159.4-161.1 | 3334, 1682, 1244, 760 | CDCl₃*: 7.88(1H, d, J=7Hz), 7.47(1H, d, J=7Hz), 7.37(2H, d, J=9Hz), 7.0-6.8(3H, m), 5.84(1H, brs), 5.47(1H, s), 4.1-3.9(3H, m), 2.9-2.7(2H, m), 2.45(2H, t, J=8Hz), 2.25(6H, s), 2.2-1.8(3H, m), 1.9-1.6(1H, m) |
| 85 | 145.3-153.4 | 3392, 1657, 1495, 1259, 758 | CDCl₃*: 7.87(1H, dd, J=2, 8Hz), 7.65(1H, d, J=2Hz), 7.48(1H, d, J=7Hz), 7.39(1H, dd, J=2, 8Hz), 7.0-6.7(2H, m), 5.85(1H, brs), 5.46(1H, d, J=2Hz), 4.1-3.8(3H, m), 3.91(3H, s), 3.0-2.8(2H, m), 2.2-2.0(1H, m), 1.9-1.7(1H, m) |
| 86 | 191.7-197.0 | 1672 1489 1263, 1055, 766 | CDCl₃*: 7.90(1H, dd, J=8Hz), 7.74(1H, d, J=2Hz), 7.44(1H, dd, J=2, 8Hz), 7.39(1H, dd, J=1, 8Hz), 6.93(1H, d, J=8Hz), 6.87(1H, t, J=8Hz), 5.78(1H, brs), 5.49(1H, s), 4.01(1H, t), 3.94(3H, s), 3.0-2.8(1H, m), 2.8-2.6(1H, m), 2.1-1.9(1H, m), 1.9-1.7(1H, m) |
| 87 | 130.3-134.0 | 3250, 1657, 1508, 1444, 1275, 760 | DMSO-d₆*: 8.35(1H, d, J=3Hz), 7.53(1H, dd, J=2, 8Hz), 7.33(1H, dd, J=1, 8Hz), 6.9-6.5(4H, m), 5.42(1 H, d, J=3Hz), 3.82(1H, t, J=5Hz), 3.72(3H, s), 3.1-2.9(2H, m), 1.9-1.7(1H, m), 1.7-1.5(1H, m) |
| 88 | - | 1662, 1489, 1273, 1045, 760 | CDCl₃*: 7.87(1H, dd, J=1, 8Hz), 7.49(1H, dd, J=1, 8Hz), 7.18(1H, d, J=2Hz), 7.01(1H, d, J=2Hz), 6.87(1H, t, J=8Hz), 5.83(1H, brs), 5.44(1H, d, J=2Hz), 4.1-4.0(1H, m), 3.87(3H, s), 3.81(3H, s), 3.1-2.8(2H, m), 2.2-2.0(1 H, m), 1.9-1.7(1 H, m) |
| 89 | 122.2-126.4 | 1662, 1489, 1273, 1045, 760 | CDCl₃*: 7.90(1H, d, J= 8Hz), 7.39(1H, d, J=7Hz), 7.26(1 H, d), 7.09(1H, d, J=2Hz), 6.89(1 H, t, J=8Hz), 5.79(1H, brs), 5.46(1H, s), 4.02(1H, m), 3.90(3H, s), 3.87(3H, s), 2.94(1H, dt, J=10.0Hz), 2.8-2.7(1H, m), 2.1-1.9(1H, m), 1.9-1.7(1H, m) |
| 90 | 159.7-163.3 | 1670, 1504, 1255, 1132, 764 | CDCl₃*: 7.88(1H, dd, J=1, 8Hz), 7.48(1H, d, J=7Hz), 7.3-7.2(2H, m), 6.9-6.7(2H, m), 5.80(1H, brs), 5.43(1H, d, J=1Hz), 4.0-3.9(1H, m), 3.85(3H, s), 2.9-2.7(2H, m), 2.22(3H, s), 2.2-2.0(1H, m), 1.9-1.6(1 H, m) |
| 91 | 122.0-125.2 | 1655, 1522, 1306, 1059, 758 | CDCl₃*: 7.9-7.8(2H, m), 7.57(2H, d, J=8Hz), 7.5-7.4(3H, m), 7.3-7.2(1H, m), 7.22(1H, s), 6.86(1H, t, J=8Hz), 6.21 (1H, brs), 5.61 (1H, d, J=2Hz), 4.1-4.0(1H, m), 3.1-2.9(2H, m), 2.1-2.0(1H, m), 1.9-1.8(1H, m) |
| 92 | 85.1-89.3 | 1753, 1662, 1508, 1205, 760 | CDCl₃*: 7.87(1H, dd, J=1, 8Hz), 7.47(1 H, d, J=8Hz), 7.38(2H, d, J=9Hz), 6.92(2H, d, J=8Hz), 6.85(1H, t, J=8Hz), 6.00(1H, brs), 5.49(1H, s), 4.63(2H, s), 4.28(2H, q, J=7Hz), 4.1-4.0(1H, m), 3.0-2.8(2H, m), 2.2-2.0(1 H, m), 1.9-1.7(1 H, m), 1.31(3H, t, J=7Hz) |
| 93 | 96.8-97.6 | 3466,1657, 1510, 1248, 760 | CDCl₃*: 7.87(1H, d, J=7Hz), 7.47(1H, d, J=7Hz), 7.37(2H, d, J=9Hz), 6.92(2H, d, J=8Hz), 6.85(1H, t, J=8Hz), 5.93(1H, brs), 5.48(1H, s), 4.09(2H, t, J=4Hz), 4.0-3.9(3H, m), 3.0-2.8(2H, m), 2.1-2.0(1 H, m), 1.9-1.7(1H, m) |
| 94 | 138.9-142.1 | 1655, 1603, 1508, 1275, 760 760 | CDCl₃*: 7.87(1H, d, J=7Hz), 7.51(1H, d, J=7Hz), 7.04(1H, s), 7.0-6.8(3H, m), 5.85(1H, brs), 5.44(1H, s) 4.1-3.9(1H, m), 3.49(3H, s), 3.0-2.8(2H, m), 2.2-2.0(1H, m), 1.9-1.7(1H, m) |
| 95 | 141.0-144.2 | 1666, 1325, 1126, 1066, 762 | CDCl₃*: 7.86(1H, d, J=8Hz), 7.66(2H, d, J=8Hz), 7.57(2H, d, J=8Hz), 7.45(1H, d, J=8Hz), 6.85(1H, t, J=8Hz), 6.09(1H, brs), 5.62(1H, s), 4.1-3.9(1H, m), J=8Hz), 6.09(1H, brs), 5.62(1H, s), 4.1-3.9(1H, m), 3.1-2.9(2H, m), 2.2-2.0(1H, m), 1.9-1.7(1H, m) |
| 96 | 183.1-184.4 | 1680,1514, 1273, 1034, 771 | CDCl₃*: 7.86(1 H, dd, J=1, 8Hz), 7.48(1 H, d, J=7Hz), 7.2-7.0(2H, m), 7.0-6.9(1H, m), 6.86(1H, t, J=8Hz), 6.12(1H, brs), 5.49(1H, d, 1Hz), 4.1-3.9(1H, m), 3.85(3H, s), 3.0-2.8(2H, m), 2.2-2.0(1H, m), 1.9-1.7(1H, m) |
| 97 | 154.5-156.3 | 1672, 1508, 1252, 1103, 768 | CDCl₃*: 7.87(1H, d, J=8Hz), 7.48(1H, d, J=7Hz), 7.35(2H, d, J=9Hz), 6.9-6.8(3H, m), 5.84(1H, brs), 5.46(1H, s), 4.6-4.5(1H, m), 4.0-3.9(1H, m), 3.0-2.8(2H, m), 2.2-2.0(1H, m), 1.9-1.7(1H, m), 1.34(6H, d, J=6Hz) |
| 98 | 133.1-138.2 dec. | 1657, 1514, 1261, 1024, 762 | CDCl₃*: 7.87(1H, dd, J=2, 8Hz), 7.50(1H, d, J=7Hz), 7.05(1H, d, J=2Hz), 6.96(1H, dd, J=2, 8Hz), 6.9-6.8(2H, m), 5.89(1H, brs), 5.46(1H, s), 4.1-3.9(1H, m), 3.90(3H, s), 3.86(3H, s), 3.07(2H, brs), 2.9-2.8(2H, m), 2.2-2.0(1 H, m), 1.7-1.5(1 H, m) |
| 99 | 128.5-137.4 | 1676, 1444, 1273, 1026, 762 | CDCl₃*: 7.86(1H, dd, J=2, 8Hz), 7.46(1H, dd, J=8Hz), 7.22(1H, dd, J=2, 12Hz), 7.15(1H, d, J=8Hz), 6.95(1H, dd, J=8, 8Hz), 6.85(1H, t, J=8Hz), 6.02(1H, brs), 5.47(1H, s), 4.1-4.0(1H, m), 3.90(3H, s), 3.0-2.9(2H, m), 2.2-2.0(1H, m), 1.9-1.7(1H, m) |
| 100 | 127.0-131.2 | 1655, 1502, 1317, 760 | CDCl₃*: 8.75-8.56(2H, m), 7.9-7.7(2H, m), 7.44(1 H, d, J=7Hz), 7.5-7.3(1 H. m), 6.85(1H, t, J=8Hz), 6.25(1H, brs), 5.62(1H, s), 4.1-4.0(1H, m), 3.1-2.9(2H, m), 2.2-2.0(1 H, m), 1.9-1.7(1 H, m) |
| 101 | 74.9-77.8 | 1682, 1510, 1022, 760 1022, 760 | CDCl₃*: 7.83(1H, d, J=8Hz), 7.31(1H, d, J=7Hz), 7.10(2H, d, J=9Hz), 6.86(2H, d, J=9Hz), 6.75(1H, t, J=8Hz), 6.10(1H, brs), 4.7-4.6(1H, m), 4.1-4.0(1H, m), 3.79(3H, s), 3.6-3.5(1H, m), 3.3-3.2(1H, m), 3.1-2.8(2H, m), 2.1-1.9(2H, m) |
| 102 | 184.8-188.3 | 1662, 1489, 1255, 839, 769 | CDCl₃: 7.90(1H, dd, J=1, 8Hz), 7.46(2H, d, J=9Hz), 7.38(1H, dd, J=1, 8Hz), 6.95(2H, d, J=9Hz), 6.86(1H, t, J=8Hz), 5.78(1H, s), 5.50(1H, s), 3.99(1H, t, J=4Hz), 3.85(3H, s), 2.9-2.7(2H, m), 2.0-1.7(2H, m) |
| 103 | 143.3-154.6 | 143.3-154.6 1664, 1489, 1254, 758 | CDCl₃: 7.81 (1H, dd, J=2, 8Hz), 7.41 (2H, d, J=9Hz), 7.15(1H, dd, J=2, 8Hz), 6.92(2H, d, J=9Hz), 6.78(1H, t, J=8Hz), 5.79(1H, brs), 5.46(1H, d, J=1Hz), 3.83(3H, s), 2.9-2.7(4H, m), 2.0-1.9(2H, m) |
| 104 | 189.0-190.8 | 1662,1491, 1242, 1030, 768 | CDCl₃*: 7.78(1H, dd, J=2, 8Hz), 7.4-7.2(3H, m), 6.91(1H, d, J=8Hz), 6.84(1H, t, J=8Hz), 6.73(1H, t, J=8Hz), 6.30(1H, brs), 6.0.1(1H, d, J=3Hz), 4.2-4.1(1H, m), 3.87(3H, s), 3.5-3.4(1H, m), 3.2-3.1 (1H, m), 2.2-2.0(1 H, m), 1.9-1.8(1 H, m) |
| 105 | 190.2-193.1 | 3330. 1601, 1510, 1238, 957, 754 | CDCl₃*: 7.80(1H, dd, J=2, 8Hz), 7.5-7.4(1H, m),7.4-7.3(2H, m), 7.0-6.9(2H, m), 6.77(1H, t, J=8Hz), 6.16(1H, brs), 6.05(1H, d, J=3Hz), 4.1-4.0(1H, m), 3.86(3H, s), 3.3-3.2(1H, m), 3.2-3.1(1H, m), 2.1-2.0(1H, m), 1.9-1.8(1H, m) |
| 115 | 198.0-200.8 | 3350, 1660, 1491, 1323, 758 | DMSO-d₆: 8.27(1H, d, J=3Hz), 7.55(1H ,dd, J=2, 8Hz), 7.4-7.3(1H, m), 6.99(2H, d, J=8Hz), 6.65(1H, t, J=8Hz), 6.49(2H, d, J=8Hz), 5.35(1H, d, J=3Hz), 5.16(2H, brs), 3.9-3.8(1H, m), 3.0-2.9(2H, m), 1.8-1,6(2H, m) |
| 116 | 117.7-121.7 | 1662, 1348, 758 | DMSO-d₆*: 8.70(1H. d, J=4Hz), 8.21(2H, d, J=9Hz), 7.62(2H, d, J=9Hz), 7.55(1 H, dd, J=2, 8Hz), 7.34(1H, dd, J=2, 8Hz), 6.68(1H, t, J=8Hz), 5.82(1H, d, J=4Hz), 3.91(1H, t, J=4Hz), 3.2-3.1(2H, m), 1.9-1.7(2H, m) |
| 117 | 198.3-200.9 | 1664, 1348, 750 | DMSO-d₆*: 8.62(1H, d, J=3Hz), 8.25(2H, d, J=9Hz), 7.61 (2H, d, J=9Hz), 7.5-7.4(1H, m), 7.48(1H, d, J=7Hz), 6.71 (1H, t, J=8Hz), 5.78(1H, d, J=3Hz), 3.8-3.7(1H, m), 3.4-3.2(1H, m), 2.9-2.8(1H, m), 2.0-1.6 (2H, m) |
| 118 | 162.9-168.8 | 1664, 1315, 1149, 762 | DMSO-d₆: 8.45(1H, d, J=3Hz), 7.54(1H, dd, J=1, 8Hz), 7.33(1H, dd, J=1, 8Hz), 7.23(2H, d, J=8Hz), 7.14(2H, d, J=8Hz), 6.65(1 H, t, J=8Hz), 5.55(1H, d, J=3Hz), 3.86(1H, t, J=4Hz), 3.1-3.0(2H, m), 2.28(3H, s), 1.9-1.6(4H, m) |
| 119 | 176.8-180.2 | 1664, 1485, 1225, 845 | DMSO-d₆*: 8.52(1H, d, J=3Hz), 7.55(1H, dd, J=2, 8Hz), 7.4-7.3(3H, m), 7.18(2H, t, J=9Hz), 6.66(1 H, t, J=8Hz), 5.63(1H, d, J=3Hz), 3.88(1H, t, J=4Hz), 3.1-3.0(2H, m), 1.9-1.6(4H, m) |
| 120 | 119.6-122.1 | 2229, 1660, 1319, 758 | DMSO-d₆: 8.7-8.6(1H, m), 7.84(2H, d, J=8Hz), 7.6-7.5(3H, m), 7.4-7.3(1H, m), 6.67(1H, t, J=8Hz), 5.8-5.7(1H, m), 4.0-3.9(1H,m), 3.3-3.0(2H, m), 2.2-1.7(4H, m) |
| 121 | 118.5-121.8 | 2227, 1660, 1454, 1319, 760 | DMSO-d₆: 8.6-8.5(1H, m), 7.86(2H, d, J=8Hz), 7.6-7.4(4H, m), 6.70(1H. t, J=8Hz), 5.72(1H. d, J=3Hz), 3.8-3.7(1H, m), 3.4-3.2(1H, m), 2.9-2.8(1H, m), 2.0-1.7(4H, m) |
| 122 | 155.7-158.6 | 1666, 1452, 1279, 1115, 766 | DMSO-d₆: 8.6-8.5(1 H, m), 7.6-7.2(5H, m), 6.67(1H, t, J=7Hz), 5.67(1H, d, J=3Hz), 4.0-3.9(1H, m), 3.2-3.1 (2H, m), 1.9-1.7(4H, m) |
| 123 | 185.5-191.2 | 1664, 1313, 1011, 796, 758 | DMSO-d₆*: 8.54(1H, d, J=3Hz), 7.6-7.5(3H, m), 7.4-7.2(3H, m), 6.66(1H, t, J=7Hz), 5.62(1H, d, J=3Hz), 3.88(1H, t, J=4Hz), 3.1-3.0(2H, m), 1.9-1.7(4H, m) |
| 115 | 198.0-200.8 | 3350,1660, 1491, 1323, 758 | DMSO-d₆: 8.27(1H, d, J=3Hz), 7.55(1H ,dd, J=2, 8Hz), 7.4-7.3(1H, m), 6.99(2H, d, J=8Hz), 6.65(1 H, t, J=8Hz), 6.49(2H, d, J=8Hz), 5.35(1H, d, J=3Hz), 5.16(2H, brs), 3.9-3.8(1H, m), 3.0-2.9(2H, m), 1.8-1.6(2H, m) |
| 116 | 117.7-121.7 | 1662,1348, 758 | DMSO-d₆*: 8.70(1 H, d, J=4Hz), 8.21 (2H, d, J=9Hz), 7.62(2H, d, J=9Hz), 7.55(1H, dd, J=2, 8Hz), 7.34(1 H, dd, J=2, 8Hz), 6.68(1 H, t, J=8Hz), 5.82(1 H, d, J=4Hz), 3.91 (1H, t, J=4Hz), 3.2-3.1 (2H, m), 1.9-1.7(2H, m) |
| 117 | 198.3-200.9 | 1664, 1348, 750 750 | DMSO-d₆*: 8.62(1 H, d, J=3Hz), 8.25(2H, d, J=9Hz), 7-61 (2H, d, J=9Hz), 7.5-7.4(1H, m), 7.48(1H, d, J=7Hz), 6.71 (1H, t, J=8Hz), 5.78(1H, d, J=3Hz); 3.8-3.7(1H, m), 3.4-3.2(1H, m), 2.9-2.8(1H, m), 2.0-1.6 (2H, m) |
| 118 | 162.9-168.8 | 1664, 1315, 1149, 762 | DMSO-d₆: 8.45(1H, d, J=3Hz), 7.54(1H, dd, J=1, 8Hz), 7.33(1H, dd, J=1, 8Hz), 7.23(2H, d, J=8Hz), 7.14(2H, d, J=8Hz), 6.65(1H, t, J=8Hz), 5.55(1H, d, J=3Hz), 3.86(1H, t, J=4Hz), 3.1-3.0(2H, m), 2.28(3H, s), 1.9-1.6(4H, m) |
| 119 | 176.8-180.2 | 1664, 1485, 1225, 845 | DMSO-d₆*: 8.52(1H, d, J=3Hz), 7.55(1H, dd, J=2, 8Hz), 7.4-7.3(3H, m), 7.18(2H, t, J=9Hz), 6.66(1H, t, J=8Hz), 5.63(1H, d, J=3Hz), 3.88(1H, t, J=4Hz), 3.1-3.0(2H, m), 1.9-1.6(4H, m) |
| 120 | 119.6-122.1 | 2229,1660, 1319, 758 | DMSO-d₆: 8.7-8.6(1H, m), 7.84(2H, d, J=8Hz), 7.6-7.5(3H, m), 7.4-7.3(1H, m), 6.67(1H, t, J=8Hz), 5.8-5.7(1H, m), 4.0-3.9(1H,m), 3.3-3.0(2H, m), 2.2-1.7(4H, m), |
| 121 | 118.5-121.8 | 2227, 1660, 1454, 1319, 760 | DMSO-d₆: 8.6-8.5(1H, m), 7.86(2H, d, J=8Hz), 7.6-7.4(4H, m), 6.70(1H, t, J=8Hz), 5.72(1H, d, J=3Hz), 3.8-3.7(1H, m), 3.4-3.2(1H, m), 2.9-2.8(1H, m), 2.0-1.7(4H, m) |
| 122 | 155.7-158.6 | 1666,1452, 1279, 1115, 766 | DMSO-d₆: 8.6-8.5(1H, m), 7.6-7.2(5H, m), 6.67(1H, t, J=7Hz), 5.67(1H, d, J=3Hz), 4.0-3.9(1H, m), 3.2-3.1(2H, m), 1.9-1.7(4H, m) |
| 123 | 185.5-191.2 | 1664, 1313, 1011, 796, 758 | DMSO-d₆*: 8.54(1H, d, J=3Hz), 7.6-7.5(3H, m), 7.4-7.2(3H, m), 6.66(1H, t, J=7Hz), 5.62(1H, d, J=3Hz), 3.88(1H, t, J=4Hz), 3.1-3.0(2H, m), 1.9-1.7(4H, m) |
| 124 | 186.2-190.8 | 1668, 1489, 1011, 762 | DMSO-d₆*: 8.47(1H, d, J=3Hz), 7.6-7.5(3H, m), 7.45(1H, d, J=7Hz), 7.30(2H, d, J=8Hz), 6.70(1H, t, J=7Hz), 5.59(1H, d, J=3Hz), 3.8-3.7(1H, m), 3.3-3.1 (1H, m), 2.9-2.7(1H, m), 1.9-1.8(3H, m), 1.8-1.6(1H, m) |
| 125 | 192.4-195.5 | 1666, 1487, 1090, 796, 764 | DMSO-d₆: 8.6-8.5(1H, m), 7.54(1H, dd, J=1, 8Hz), 7.5-7.3(5H, m), 6.66(1H, t, J=6Hz), 5.64(1H, d, J=3Hz), 3.9-3.8(1H, m), 3.2-3.0(2H, m), 1.9-1.6(4H, m) |
| 126 | 180.3-184.6 | 1662,1491,1090, 841,756 | DMSO-d₆: 8.48(1H, brs), 7.6-7.3(6H, m), 6.70(1H, t, J=8Hz), 5.61 (1H, d, J=2Hz), 3.8-3.7(1H, m), 3.3-3.2(1H, m), 2.9-2.7(1H, m), 2.0-1.6(4H, m) |
| 127 | 232.3-238.7 | 1655, 1385, 802,764 802, 784 | CF₃CO₂D: 8.32(2H, d, J=8Hz), 8.08(1H, dd, J=1, 8Hz), 7.8-7.7(3H, m), 7.08(1H, t, J=8Hz), 6.09(1H, s), 4.9-4.8(1H, m), 3.4-3.0(2H, m), 2.6-2.3(2H, m) |
| 128 | 208.0-209.5 | 1668, 1525, 1444, 1360, 829, 771 | CDCl₃ : 7.87(1H, dd, J=1, 8Hz), 7.5-7.4(1H, m), 7.30(2H, d, J=9Hz), 6.84(1H, t, J=8Hz), 6.69(2H, d, J=9Hz), 5.84(1H, brs), 5.41 (1H, d, J=1Hz), 3.96(1H, dd, J=5, 8Hz), 2.98(6H, s), 3.0-2.7(2H, m), 2.1-2.0(1H, m), 1.9-1.7(1H, m) |
| 129 | 237.8-239.7 | 1662, 1523, 1454, 1354, 825, 769 825, 769 | CDCl₃*: 7.90(1H, dd, J=2, 8Hz), 7.4-7.3(3H, m), 6.85(1H, t, J=8Hz), 6.73(2H, d, J=9Hz), 5.76(1H, brs), 5.44(1H, s), 3.98(1H, t, J=4Hz), 3.00(6H, s), 2.9-2.7(2H, m), 2.0-1.9(1H, m), 1.8-1.7(1H, m) |
| 130 | 172.7-173.5 | 1660, 1458, 1257,1051, 760 | CDCl₃*: 7.9-7.8(1 H, m), 7.6-7.5(1H, m), 7.40(1 H, d, J=8Hz), 6.87(1H, d, J=8Hz), 6.8-6.7(2H, m), 5.74(1H, s), 5.67(1H, brs), 4.00(1H, dd, J=5, 10Hz), 3.82(3H, s), 3.0-2.7(2H, m), 2.45(3H, s), 2.2-2.0(1H. m), 1.9-1.7(1H, m) |
| 131 | 213.6-214.8 | 1670, 1464, 1257, 1049, 773 | CDCl₃*: 7.90(1 H, dd, J=1, 8Hz), 7.44(1 H, d, J=8Hz), 7.36(1H, dd, J=1, 8Hz), 6.86(1H, t, J=8Hz), 6.8-6.7(2H, m), 5.77(1H, s), 5.67(1H, brs), 4.1-4.0(1H, m), 3.83(3H, s), 2.9-2.7(2H, m), 2.47(3H, s), 2.0-1.9(1H, m), 1.8-1.7(1H, m) |
| 132 | 201.4-202.9 | 1666, 1608, 1514, 1252, 1030, 758 | CDCl₃*: 7.91 (1H, dd, J=1, 8Hz), 7:60(2H, d, J=9Hz), 7.5-7.4(3H, m), 6.95(2H, d, J=9Hz), 6.85(1H, t, J=8Hz), 6.65(2H, d, J=9Hz), 5.96(1H, d, J=8Hz), 5.79(1H, brs), 5.49(1H, s), 5.4-5.3(1H, m), 3.85(3H, s), 3.02(6H, s), 3.0-2.8(2H, m), 2.3-2.2(1H, m), 2.1-1.9(1H, m) |
| 133 | 186.0-187.2 | 1664, 1252, 1030, 758 | CDCl₃*: 9.3-9.2(1H, m), 7.90(1H, dd, J=1, 8Hz), 7.41(2H, d, J=9Hz), 7.4-7.3(1H, m), 6.92(2H, d, J=9Hz), 6.84(1 H, t, J=8Hz), 5.82(1 H, brs), 5.49(1 H, d, J=1Hz), 5.1-5.0(1H, m), 3.83(3H, s), 2.9-2.7(2H, m), 2.6-2.1(9H, m), 1.9-1.8(1H, m), 1.4-1.2(6H, m) |
| 134 | 252.3-253.1 | 1666, 1450, 1254, 1030, 756 | CDCl₃*: 7.88(1 H, d, J=8Hz), 7.40(2H, d, J=9Hz), 7.3-7.2(1H, m), 6.94(2H, d, J=9Hz), 6.84(1H, t, J=8Hz), 5.79(1 H, brs), 5.55(1 H, d, J=9Hz), 5.45(1 H, s), 5.2-5.1(1H, m), 3.84(3H, s), 3.0-2.7(4H, m), 2.26(3H, s), 2.2-2.0(2H, m), 2.0-1.7(7H, m) |
| 135 | 188.2-192.4 | 1658, 1450, 1254, 764 | CDCl₃*: 7.86(1H, dd, J=1, 8Hz), 7.7-7.6(1H, m), 7.44(2H, d, J=9Hz), 6.93(2H, d, J=9Hz), 6.86(1H, t, J=8Hz), 5.74(1H, brs), 5.41(1H, s), 3.84(3H, s), 3.8-3.7(1H, m), 3.0-2.9(1H, m), 2.8-2.7(1H, m), 2.24(6H, s), 2.0-1.8(2H, m) |
| 136 | 161.4-166.6 | 1664, 1604 1664, 1604, 1518, 1254, 779 | DMSO-d₆*: 8.43(1H, d, J=2Hz), 7.7-7.6(1H, m), 7.61(1H, brs), 7.30(2H, d, J=9Hz), 7.21(1H, d, J=7Hz), 6.94(2H, d, J=9Hz), 6.73(1H, t, J=7Hz), 5.54(1H, d, J=2Hz), 5.0-4.8(1H, m), 3.75(3H, s), 5.54(1H, d, J=2Hz), 5.0-4.8(1H, m), 3.75(3H, s), 3.17-3.02(1H, m), 2.9-2.7(3H, m), 2.12(6H, s), 2.0-1.8(2H, m) |

**Table 6**

| Example No. | m.p. **°**C | IR (cm⁻¹) | NMR (ppm : No mark:300MHz, *:270MHz) |
|---|---|---|---|
| 1-1 | 59.3-60.4 | - | CDCl₃ : 7.82(1H, dd, J=2, 8Hz), 7.57(1H, dt, J=2, 8HZ), 6.62(1H, dt, J=1, 8Hz), 5.24(1H, brs), 3.66(2H, dt, J=2, 7Hz), 2.72(2H, t, J=7Hz) |
| 1-2 | 147.4-150.9 | - | CDCl₃: 8.04(1H, dd, J=2, 8Hz), 7.59(1H, dd, J=2, 8Hz), 6.77(1H, t, J=8Hz), 3.72(2H, dt, J=2, 7Hz), 2.77(2H, t, J=7Hz) |
| 1-3 | 188.4-193.2 | - | DMSO-d₆: 8.60(1H, brs), 8.00(1H, brs), 7.83(1H, dd, J=2, 8Hz), 7.77(1H, dd, J=2, 8Hz), 7.40(1H, brs), 6.67(1H, t, 8Hz), 3.54(2H, t, J=7Hz), 2.56(2H, t, J=7Hz) |
| 22 -1 | - | 3348,1666, 1518, 1263, 1157 | DMSO-d₆*: 7.71(1H, dd, J=1, 2Hz), 7.49(1H, dd, J=1, 3Hz), 5.04(brs, 1H), 3.68(2H, dt, J=2, 7Hz), 2.74(2H, t, J=7Hz) |
| 22-2 | 111.7-117.5 | 3435, 2224, 1678, 1535, 1263, 1213, 1167 | CDCl₃: 7.91(1H, d, J=3Hz), 7.46(1H, d, J=3Hz), 5.33(1H, brs), 3.74(2H, dt, J=2, 7Hz), 2.79(2H, t, J=7Hz) |
| 22-3 | - | - | DMSO-d₆*: 8.72(1H, brs), 8.14(1H, brs), 7.88(1H, d, J=2Hz), 7.64(1H, d, J=2Hz), 7.59(1H, brs), 3.6-3.5(2H, m), 2.61 (1 H, t, J=7Hz) |
| 23-1 | 103.1-108.7 | 3415, 1670, 1502, 1350, 1250, 1149, 793 | CDCl₃*: 7.54(1H, dd, J=3, 9Hz), 7.39(1H, dd, J=3, 7Hz), 4.85(1H, brs), 3.7-3.6(2H, m), 2.72(2H, t, J=7Hz) |
| 23-2 | 162.2-183.5 | 3336, 2220, 1666, 1523, 1278, 1159, 895, 631 | CDCl₃*: 7.77(1H, dd, J=3, 8Hz), 7.34(1H, dd, J=3, 7Hz), 5.13(1H, brs), 3.8-3.7(2H, m), 2.77(2H, t, J=7Hz) |
| 23-3 | 199.4-202.4 | 3415,3330, 3188, 1660, 1595, 1514, 1250, 1149 | DMSO-d₆*: 8.06(1H, brs),7.75(1H, dd, J=3, 9Hz), 7.54(1H, brs), 7.49(1H, dd, J=3, 9Hz), 3.51(2H, t, J=7Hz), 2.56(2H, t, J=7Hz) |
| 24-1 | - | - | CDCl₃: 8.64(1 H, brs), 7.90(1 H, d, J=9Hz), 6.63(1 H, d, J=9Hz), 3.88(3H, s), 3.7-3.6(2H, m), 2.74(2H, t, J=7Hz) |
| 24-2 | - | - | DMSO-d₆*: 8.76(1H, brs), 7.87(1H, d, J=8Hz), 6.60(1H, d, J=8Hz), 3.60(2H, t, J=7Hz), 2.61(2H, t, J=7Hz) |
| 24-3 | - | - | DMSO-d₆: 9.11(1H, s), 8.03(1H, brs), 7.68(1H, d, J=8Hz), 7.46(1H, brs), 6.57(1H, d, J=8Hz), 3.6-3.5(2H, m), 2.57(2H, t, J=7Hz) |

Sequence ID No.1

Lys Leu Thr Val Asn Pro Gly Thr Lys Ser Lys Leu Pro Lys Pro Val
1               5                   10                  15
Gln Asp Leu Ile Lys Met Ile Phe Asp Val Glu Ser Met Lys Lys Ala
                20                  25                  30
Met Val Glu Tyr Glu Ile Asp Leu Gln Lys Met Pro Leu Gly Lys Leu
            35                  40                  45
Ser Lys Arg Gln Ile Gln Ala Ala Tyr Ser Ile Leu Ser Glu Val Gln
    50                  55                  60
Gln Ala Val Ser Gln Gly Ser Ser Asp Ser Gln Ile Leu Asp Leu Ser
65                  70                  75                  80
Asn Arg Phe Tyr Thr Leu Ile Pro His Asp Phe Gly Met Lys Lys Pro
                85                  90                  95
Pro Leu Leu Asn Asn Ala Asp Ser Val Gln Ala Lys Val Glu Met Leu
            100                 105                 110
Asp Asn Leu Leu Asp Ile Glu Val Ala Tyr Ser Leu Leu Arg Gly Gly
        115                 120                 125
Ser Asp Asp Ser Ser Lys Asp Pro Ile Asp Val Asn Tyr Glu Lys Leu
    130                 135                 140
Lys Thr Asp Ile Lys Val Val Asp Arg Asp Ser Glu Glu Ala Glu Ile
145                 150                 155                 160
Ile Arg Lys Tyr Val Lys Asn Thr His Ala Thr Thr His Asn Ala Tyr
                165                 170                 175
Asp Leu Glu Val Ile Asp Ile Phe Lys Ile Glu Arg Glu Gly Glu Cys
            180                 185                 190
Gln Arg Tyr Lys Pro Phe Lys Gln Leu His Asn Arg Arg Leu Leu Trp
        195                 200                 205

His Gly Ser Arg Thr Thr Asn Phe Ala Gly Ile Leu Ser Gln Gly Leu
    210                 215                 220
Arg Ile Ala Pro Pro Glu Ala Pro Val Thr Gly Tyr Met Phe Gly Lys
225                 230                 235                 240
Gly Ile Tyr Phe Ala Asp Met Val Ser Lys Ser Ala Asn Tyr Cys His
                245                 250                 255
Thr Ser Gln Gly Asp Pro Ile Gly Leu Ile Leu Leu Gly Glu Val Ala
            260                 265                 270
Leu Gly Asn Met Tyr Glu Leu Lys His Ala Ser His Ile Ser Lys Leu
        275                 280                 285
Pro Lys Gly Lys His Ser Val Lys Gly Leu Gly Lys Thr Thr Pro Asp
    290                 295                 300
Pro Ser Ala Asn Ile Ser Leu Asp Gly Val Asp Val Pro Leu Gly Thr
305                 310                 315                 320
Gly Ile Ser Ser Gly Val Asn Asp Thr Ser Leu Leu Tyr Asn Glu Tyr
                325                 330                 335
Ile Val Tyr Asp Ile Ala Gln Val Asn Leu Lys Tyr Leu Leu Lys Leu
            340                 345                 350
Lys Phe Asn Phe Lys Thr Ser Leu Trp
        355                 360

## Claims

1. A compound represented by Formula (I) or a pharmaceutically acceptable salt thereof: 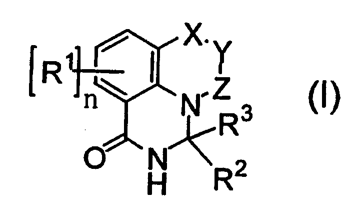 (wherein R¹ represents a hydrogen atom, a halogen atom, a hydroxyl group, an amino group, a straight or branched chain C1 to 4 alkyl group unsubstituted or substituted by 1 to 5 halogen atoms, or a C1 to 4 alkoxy group unsubstituted or substituted by 1 to 5 halogen atoms;
n represents an integer of 0 to 2; and
R² and R³ each independently represent a hydrogen atom, a straight or branched chain C1 to 6 alkyl group, a straight or branched chain C2 to 4 alkenyl group or a group represented by -L-Ar (L represents a bond or a C1 to 6 alkylene, and Ar represents a saturated or unsaturated 5- to 6-membered ring or saturated or unsaturated 8- to 12-membered condensed-bicyclic hydrocarbon group which may contain 1 to 3 heteroatoms arbitrarily selected from the group consisting of nitrogen atom, sulfur atom and oxygen atom), and any of these groups may have 1 to 3 substituents arbitrarily selected from group A:
[group A: halogen atoms, a nitro group, a cyano group, a hydroxy group, oxo groups, amino groups unsubstituted or substituted by one or two C1 to 4 alkyl groups, straight or branched chain C1 to 6 alkyl groups unsubstituted or substituted by 1 to 5 substituents arbitrarily selected from group Aa, straight or branched chain C1 to 5 alkoxy groups unsubstituted or substituted by 1 to 5 substituents arbitrarily selected from group Aa, straight or branched chain C1 to 5 alkylthio groups unsubstituted or substituted by 1 to 5 substituents arbitrarily selected from group Aa, straight or branched chain C2 to 4 alkenyl groups unsubstituted or substituted by 1 to 5 substituents arbitrarily selected from group Aa, and saturated or unsaturated 5- or 6-membered rings containing nitrogen unsubstituted or substituted by 1 to 5 substituents arbitrarily selected from group Aa,
group Aa: a halogen atom, a nitro group, a hydroxy group, C1 to 3 alkoxy groups, cyano groups, amino groups unsubstituted or substituted by one or two C1 to 4 alkyl groups, carboxyl groups and C1 to 3 alkoxycarbonyl groups], and
X represents -(CHR⁴)-, -(C=NOR⁵)- or -(C=O)-,
R⁴ represents -NR⁶R⁷ (R⁶ and R⁷ each idependently represent a hydrogen atom, a C1 to 6 alkyl group, or a C1 to 6 alkylcarbonyl group; the hydrogen atom of the alkyl group and the alkylcarbonyl group are unsubstituted or substituted by a phenyl group, a piperidinyl group or an amino group unsubstituted or substituted by one or two or C1 to 4 alkyl groups; and hydrogen atoms of the phenyl group and the piperidinyl group are unsubstituted or substituted by an amino group unsubstituted or substituted by one or two C1 to 4 alkyl groups), a hydroxy group, a C1 to 6 alkoxy group, or a hydrogen atom; and
R⁵ represents a hydrogen atom or a C1 to 6 alkyl group;
Y represents an imino group (-NH-) or a methylene group;
Z represents a methylene group, an ethylene group, or a vinylene group (-CH=CH-); the hydrogen atom of Y and Z are unsubstituted or substituted by a halogen atom, a nitro group, a straight or branched chain C1 to 4 alkyl group, a straight or branched chain C1 to 4 alkoxy group, a cyano group, a phenyl group unsubstituted or substituted by one or two straight or branched chain C1 to 4 alkyl or alkoxy groups, or an amino group unsubstituted or substituted by one or two straight or branched C1 to 4 alkyl groups may be substituted).

2. The compound or the pharmaceutically acceptable salt thereof according to Claim 1, wherein Y and Z each represent a methylene group and X represents CHR⁴ (R⁴ has the same meaning as defined in Claim 1).

3. The compound or the pharmaceutically acceptable salt thereof according to Claim 1 or 2, wherein R² represents a hydrogen atom, and R³ represents a saturated or unsaturated 5-to 6-membered ring or saturated or unsaturated 8- to 12-membered condensed-bicyclic hydrocarbon group unsubstituted or substituted by 1 to 3 substituents arbitrarily selected from group A, and not containing or containing 1 to 3 heteroatoms.

4. A pharmaceutical composition containing at least one active ingredient selected from the group consisting of the compound and its pharmaceutically acceptable salt as set forth in any one of Claims 1 to 3.

5. A poly(ADP-ribose)polymerase inhibitor containing at least one type selected from the group consisting of the compound and its pharmaceutically acceptable salt as set forth in any one of Claims 1 to 3.

6. A crystal of poly(ADP-ribose)polymerase catalytic fragment having lattice constants of a = 67Å±5Å, b = 92Å±5Å, and c = 64Å±5Å, and having an orthorhombic space group of P2₁2₁2.

7. A co-crystal of a human poly(ADP-ribose)polymerase catalytic fragment with the compound and its pharmaceutically acceptable salt as set forth in any one of Claims 1 to 3.

8. The co-crystal with the human poly(ADP-ribose)polymerase catalytic fragment according to Claim 7, wherein the compound and its pharmaceutically acceptable salt as set forth in any one of Claims 1 to 3 is (+)-(3R,7R)-7-amino-3-(4-methoxyphenyl)-6,7-dihydro-3H,5H-pyrido[3,2,1-ij]quinazoline-1(2H)-one.

9. The co-crystal with the human poly(ADP-ribose)polymerase catalytic fragment according to Claim 7, wherein the compound and its pharmaceutically acceptable salt as set forth in any one of Claims 1 to 3 is 7-amino-3-(4-methoxy-3-nitrophenyl)-6,7-dihydro-3H,5H-pyrido[3,2,1-ij]quinazoline-1(2H)-one.

10. The co-crystal with the human poly(ADP-ribose)polymerase catalytic fragment according to Claim 7, wherein the compound and its pharmaceutically acceptable salt as set forth in any one of Claims 1 to 3 is 7-(4-N,N-dimethylaminophenyl)methylamino-3-(4-methoxyphenyl)-6,7-dihydro-3H,5H-pyrido[3,2,1-ij]quinazoline-1(2H)-one.
